# EUROPÄISCHE PATENTSCHRIFT

(11) **EP 4 135 600 B1**
(45) Veröffentlichungstag und Bekanntmachung des Hinweises auf die Patenterteilung: **13.08.2025**
(21) Anmeldenummer: 21719116.2
(22) Anmeldetag: 14.04.2021
(51) Int. Cl.: A61B 17/15, A61B 17/00, A61B 17/17

(54) **AUSRICHTUNGSVORRICHTUNG FÜR EINE TIBIALE RESEKTIONSFÜHRUNG**
ALIGNMENT DEVICE FOR A TIBIAL RESECTION GUIDE
DISPOSITIF D'ALIGNEMENT SERVANT À EFFECTUER UNE RÉSECTION DU TIBIA

(30) Priorität: 15.04.2020 DE 102020110346
(43) Veröffentlichungstag der Anmeldung: 22.02.2023
(73) Patentinhaber: Aesculap AG, 78532 Tuttlingen (DE)
(72) Erfinder: FIRMBACH, Franz-Peter, 78576 Emmingen-Liptingen (DE); BÖTTIGER, Roland, 78604 Rietheim-Weilheim (DE); RICHTER, Berna, Dr., 78570 Mühlheim (DE)
(74) Vertreter: Winter, Brandl - Partnerschaft mbB
(86) Internationale Anmeldenummer: PCT/EP2021/059647
(87) Internationale Veröffentlichungsnummer: WO 2021/209496

(56) Entgegenhaltungen:
- EP-A2- 0 839 501
- WO-A1-00/71035
- WO-A1-2005/110249
- WO-A1-2009/037479
- WO-A1-2020/013584
- WO-A2-2019/046518
- DE-T2- 60 028 278
- US-A- 5 197 944
- US-A1- 2012 101 504
- US-B1- 6 221 035

## Beschreibung

### Technisches Gebiet

Die vorliegende Offenbarung betrifft eine Ausrichtungsvorrichtung bzw. Ausrichthilfe für eine tibiale Resektionsführung, die eine Klemmvorrichtung umfasst, die zumindest zwei gegeneinander wirkende Klammerelemente zum Klemmen des distalen Endes einer Tibia eines Patienten aufweist; die eine Antastvorrichtung zum Antasten des proximalen Endes der Tibia umfasst, die eine Werkzeugführungsvorrichtung zum Führen eines Werkzeuges während der Resektion der Tibia umfasst; und eine Teleskopvorrichtung, die mit der Antastvorrichtung trennbar und mit der Klemmvorrichtung verstellbar verbunden ist und die dazu ausgebildet ist, die Vorrichtungen gegenüber der Tibia auszurichten. Daneben betrifft die Erfindung jeweils eine Ausrichtungsvorrichtung für eine tibiale Resektionsführung gemäß dem jeweiligen Oberbegriff der jeweils nebengeordneten Ansprüche.

### Hintergrund der Erfindung

Für den Erfolg einer Operation zur Implantation einer Gelenksprothese hat die präzise Resektion von Knochen eines Patienten, insbesondere der Tibia, eine große Bedeutung. Die Ebene der Resektion muss hierbei exakt lokalisiert werden, um einerseits ein Maß einer Knochenentnahme möglichst geringzuhalten, wobei andererseits gleichzeitig gewährleistet werden muss, dass auch das gesamte defekte Knochengewebe entfernt wird. Der Abgleich der Ebene in Bezug zu einer anatomischen Achse, insbesondere einer Tibia-Achse, muss bei einer Operation kontinuierlich kontrolliert werden, um die Ausrichtung der Gelenksoberflächen des Gelenks über den gesamten Bereich der Gelenksbewegung zu gewährleisten.

Die exakte Festlegung einer tibialen Resektionsebene in einem Kniegelenk wird üblicherweise unter Verwendung einer Ausrichtungsvorrichtung bzw. einer Einstellführung (für einen Sägeblock) mit einem säulenförmigen Einstellstab bzw. einer Teleskopvorrichtung eingestellt, der entfernt zur Tibia nahe am Fußknöchel befestigt wird. Die Teleskopvorrichtung erstreckt sich dabei entlang der Tibia (im Wesentlichen) parallel zu der zugehörigen anatomischen Tibia-Achse. Die Resektionsebene kann dann in Bezug auf die Tibia-Achse definiert werden. Ein an der Ausrichtungsvorrichtung befestigte Werkzeugführungsvorrichtung definiert schließlich die Ebene der Resektion. Üblicherweise weist die Werkzeugführungsvorrichtung dafür einen Durchgangsschlitz auf, durch welchen eine sich hin- und herschwenkende, plane Schneide eines chirurgischen Instruments (Säge) hindurchgeführt wird.

Um die Ausrichtung der Tibia Resektionsebene einzustellen, wird an der Tibia eine Ausrichtungsvorrichtung angebracht, welche an der Teleskopvorrichtung an seinem einen zu einem Fuß des Patienten hinweisenden Ende angebunden ist und an dessen anderem Ende (Endabschnitt) die Werkzeugführungsvorrichtung angebunden ist. Hierbei wird grundsätzlich zwischen zwei Ausricht- und Befestigungsprinzipien unterschieden, nämlich zwischen der "anterioren Fixierung" und der "proximalen Fixierung". Bei der "anterioren Fixierung" erfolgt die Befestigung der Ausrichtvorrichtung ausschließlich mittels der vorstehend beschriebenen Fuß-/Knöchelfessel, wohingegen bei der "proximalen Fixierung" zusätzliche Schlagpins am proximalen Endabschnitt der Ausrichtvorrichtung vorgesehen sind, welche an der sogenannten Eminentia-Intercondylaris Tibiae eingeschlagen werden.

### Stand der Technik

Die US 6,221,035 B1 offenbart eine Fuß-/Knochenfessel in Form einer Fixierungsklammer einer Ausrichtungshilfe der anterioren Fixierungsbauart, welche bei einer tibialen Resektion verwendet wird. Die Fixierungsklammer ist dabei am distalen Endabschnitt eines teleskopierbaren Ausrichtstabs angeordnet, an dessen distalem Endabschnitt ein Tibia-Schnittblock montiert ist.

Die Klammer selbst weist zwei federvorgespannte Klammerarme auf, die gegenüber einem Rahmen um jeweils eine Drehachse gedreht werden können. Diese Klammerarme werden in eine geöffnete Position gebracht und nach Anlage an das Schienbein hiernach mittels einer manuellen Auslösevorrichtung freigegeben. Sie umschließen dann aufgrund der Federvorspannung ein Fußgelenk bzw. die Tibia und klemmen diese ein. Die Klammerarme sind dabei in die Schließ-/Umklammerungsrichtung vorgespannt. Die Federvorspannung bewirkt eine kraftschlüssige Fixierung der Ausrichthilfe am bzw. unmittelbar oberhalb des Fußknöchels, was jedoch zum Nachteil hat, dass beim Patienten an den betreffenden Körperstellen aufgrund der Klemmkraft Hämatome entstehen können.

Die Klammerarme wirken federbelastend auf das Fußgelenk bzw. auf den Knöchel. Die Fixierungsklammer ist jedoch nicht an eine Anatomie des Patienten angepasst, sodass die Klammerarme je nach Fuß-/Beinform und Fußgelenksdicke eine unterschiedlich starke Anpresskraft/Klemmkraft ausüben. Hierdurch ergibt sich auch eine je nach Ausprägung des Fußgelenks abhängige Haltekraft der Fixierungsklammer.

Des Weiteren ist beispielsweise aus US 7 344 542 B2 eine Tibia-Ausrichtungsführung der proximalen Fixierungsbauart bekannt, bei welcher ein Querbalken starr mit einer teleskopierbaren Hauptstange an deren proximalen Endabschnitt verbunden ist. Am freien Endabschnitt des Querbalkens sind Befestigungsstifte gelagert, die zur zusätzlichen Fixierung der Ausrichthilfe in die Tibia eingeschlagen werden, wobei zudem ein Hebel am Querbalken gelagert ist, welcher bei einer Betätigung gegen die Tibia drückt, um die an dem Querbalken gehaltenen Stifte aus der Tibia zu ziehen. Das heißt, der gesamte Querbalken mit den integrierten Pins wird gegen den Knochen ausgehebelt, wobei Verbindung des Tibia-Schnittblocks bzw. der Sägelehre zur senkrechten Hauptstange in nachteiliger Weise gleichzeitig mit gelöst werden muss.

Schließlich ist aus US 2010/0087831 A1 grundsätzlich ein chirurgischer Nagelzieher bekannt, welcher unter einen Nagelkopf greifen und diesen mittels eines Hebels und eines Getriebes aus dem Knochen ziehen kann.

Tibia-Ausrichthilfen/ Ausrichtungsvorrichtungen der vorstehend beschriebenen Gattung weisen grundsätzliche Probleme insbesondere hinsichtlich ihrer Handhabung auf:
- So ist es beispielsweise erforderlich, für die beiden genannten Befestigungsprinzipien zueinander unterschiedlich konstruierte Ausrichthilfen vorzuhalten. Dies ist teuer und verkompliziert den gesamten Handhabungsprozess einschließlich Lagerung, Sterilisation, etc.
- Ferner wird die Ausrichthilfe während eines Operationsvorgangs vom Bein eines Patienten häufig abgenommen. Hierfür sind bei herkömmlichen Ausrichthilfen je nach Befestigungsprinzip immer mehrere Handgriffe/Aktionen notwendig, beispielsweise um die distalen und/oder proximalen Befestigungen zu lösen.
- An dieser Stelle sei nochmals daran erinnert, dass Körperdimensionen patientenspezifisch sind und erheblich voneinander abweichen. Die herkömmlichen Ausrichthilfen bekannter Bauart können hingegen jeweils nur einen begrenzten Abweichungsbereich durch entsprechende Einstellmöglichkeiten abdecken. D.h. mit den bekannten Systemen ist es nicht möglich, das komplette, weltweite Längenspektrum an Tibiae jeweils zu bedienen, sondern es müssen mehrere Systeme/Ausrichthilfen mit ggf. sich überlappenden Längenspektren vorgehalten werden, was die vorstehend bereits genannten Probleme hinsichtlich Kosten und komplizierte Handhabungsprozesse nach sich zieht. Auch muss für die Einstellung des Varus/Valgus-Alignement immer die als erstes grob eingestellte Länge des teleskopierbaren Stabs/Hauptstange aktiv (händisch) gegen eine Dejustage gesichert werden. D.h. für eine spätere Feinkorrektur der Längeneinstellung ist immer eine Manipulation (z.B. zeitweiliges manuelles Teillösen) eines Sicherungsmechanismus erforderlich, der normalerweise die grob eingestellte Länge fixieren soll.
- Ein weiteres Problem des Standes der Technik ist, dass die Fixierung am Knöchel des Patienten meist kraftschlüssig erfolgt bzw. die Klammerarme kraftschlüssig vorgespannt werden, wodurch sich durch die dadurch entstehende Eigenelastizität der Klammerarme eine nur ungenügende Positionierbarkeit ergibt. Eine Anpassung an eine Anatomie des Patienten unterbleibt. Dies führt im schlechtesten Fall zu einer lösbaren Fixierung, die jedoch aufgrund der hohen Anforderungen an eine Maßhaltigkeit bzw. Genauigkeit an die eingangs beschriebene Ausrichtung der Ebene der Resektion nicht ausreichend ist. Auch können durch die unterschiedlichen Klemmkräfte der elastisch vorgespannten Klemmarme u.a. Hämatome an den Körperstellen des Patienten entstehen.
- Durch die nach dem Stand der Technik bekannten Fixierungsklammern werden nicht alle anatomischen Größen abgedeckt, da die Klemmkraft von der jeweiligen Anatomie des Patienten abhängig sind. Daher müssten für eine optimale Anpassung verschiedene Varianten von Fixierungsklammern der Ausrichtungsvorrichtungen hergestellt und bereitgehalten werden, was jedoch in der Praxis aufgrund von hohen Kosten und schlechter Handhabbarkeit nicht praktiziert wird. Bei Fixierungsklammern von Ausrichtungsvorrichtungen, welche federbelastenden gegen das Fußgelenk bzw. den Knöchel anliegen, besteht das Problem, dass je nach Fußform und Fußgelenksdicke eine unterschiedlich starke Anpresskraft durch die Feder ausgeübt wird.
- Weiterer Stand der Technik ist aus der WO 2009 / 037479 A1, der WO 2019 / 046518 A2, der DE 600 28 278 T2, der US 5 197 944 A, der WO 2005 / 110249 A1, der WO 00 / 71035 A1, WO 2020 / 013584 A1, EP 0 839 501 A2**,** sowie aus der US 2012 / 101504 A1 bekannt.

### Zusammenfassung der Erfindung

Es ist daher eine grundsätzliche Aufgabe der vorliegenden Erfindung, die vorstehend genannten Nachteile aus dem Stand der Technik zumindest teilweise zu vermeiden oder wenigstens zu mindern und insbesondere eine (Tibia-) Ausrichtungsvorrichtung zur Verfügung zu stellen, die eine effiziente Handhabung, insbesondere eine einfache, sichere und schnelle Fixierung sowie ein einfaches und schnelles Lösen der Fixierung von einer Körperextremität / eines Körpergliedes / des Unterschenkels eines Patienten ermöglichen, wobei bevorzugt die Ausrichtungsvorrichtung für unterschiedliche Anatomien von Körperextremitäten, angepasst bzw. anpassbar ist und vorzugsweise für jede Anatomie eingesetzt werden kann und durch ihre Funktionsweisen und Konfigurationen Hämatome möglichst vermeidet. Auch soll die Ausrichtungsvorrichtung vorzugsweise eine möglichst einfache Konstruktion aufweisen, die ggf. gut zu montieren, zu reinigen und zu sterilisieren ist. Insgesamt sollen auch die mit der Handhabung einer gattungsgemäßen Ausrichtvorrichtung bzw. Ausrichthilfe verbundenen Kosten möglichst niedrig gehalten werden können. Schließlich sollte die Ausrichtungsvorrichtung bevorzugt möglichst Anwendungs-flexibel sein.

Die Aufgabe wird hinsichtlich der Ausrichtungsvorrichtung/Ausrichthilfe durch die Merkmale des Anspruchs 1 gelöst.

Die Ausrichtungsvorrichtung ("extramedulläres Tibia Ausrichtsystem") gemäß der vorliegenden Erfindung ist/bildet ein extramedulläres Ausrichtsystem, insbesondere um den proximalen Tibiaschnitt in einer Total-oder Uni-Kondylären-Kniearthroplastie (TKA/UKA) regelgerecht durchzuführen. Sie kann im Wesentlichen dazu dienen, den aus dem Stand der Technik bekannten Sägeblock für den proximalen Tibiaschnitt gemäß den klinischen Erfordernissen und der Vorstellung des Chirurgen an den entsprechenden Landmarken der Tibia auszurichten und eine ausreichende Stabilität bei der Befestigung des Sägeblocks am anterioren, epiphysären Bereich des Tibiaknochens zu erreichen. Außerdem soll sie, wenn vom Chirurgen gewünscht, ein ausreichend hohe Stabilität auch während des Sägens des Knochens mit Hilfe des Sägeblocks gewährleisten, indem die Ausrichtungsvorrichtung beispielsweise an der Tibia wahlweise fixiert bleibt.

Das vorliegende System bzw. die erfindungsgemäße Ausrichtungsvorrichtung erfüllt beide von den Anwendern geforderte, unterschiedliche Ausricht- und Befestigungs-Prinzipien in einer Einheit. Es sind dies im Folgenden die als "Anteriore Fixierung" und "Proximal Fixierung" genannte Versionen. Die Benennung erfolgte gemäß ihrer primären Fixationsmöglichkeit, wie dies vorstehend bereits definiert wurde. Die Version "Proximale Fixierung" wird erfindungsgemäß bevorzugt dadurch erreicht, dass auf die Version "Anteriore Fixierung" eine zusätzliche "Proximale-Fixationseinheit" (adapterartig) aufgesetzt wird und somit die proximale Befestigung an der "Eminentia-Intercondylaris Tibiae" zusätzlich möglich ist. Bei der "Anterioren Fixierung" erfolgt die Primär-Fixation (standardgemäß) im proximalen Bereich mittels eines Pins, der durch ein Langloch im Sägeblock in der "Tuberositas Tibiae" platziert wird.

Es werden mit der erfindungsgemäßen Ausrichtungsvorrichtung folgende Parameter eingestellt bzw. ausgerichtet:
∘ Varus/Valgus -Schnittwinkel.
∘ Der Flexion/Extension-Schnittwinkel (posteriorer/anteriorer oder auch dorsaler/ventraler Schnittwinkel (Slope)).
∘ Die proximal Schnitthöhe ( ≙ der Dicke des gewünschten Knochenschnittes).

Die erfindungsgemäße Ausrichtungsvorrichtung besteht im Wesentlichen aus den folgenden Bestandteilen:
- Distale Halteklammer insbesondere Fußklammer

Die distale Halteklammer (nachfolgend nur noch beispielhaft als Fußklammer bezeichnet) befindet sich am distalen Ende der erfindungsgemäßen Ausrichtungsvorrichtung. Sie erfüllt folgende Funktionen:
∘ Halten und Stabilisieren der Ausrichtungsvorrichtung im Bereich des Fußgelenks bzgl. seitlichen und rotativen Bewegungen relativ zur Tibia (bzw. relativ zur anatomischen Tibiaachse und somit im Regelfall auch relativ zur mechanischen Tibiaachse)
∘ Ausrichten der Ausrichtungsvorrichtung in Referenz zu den distalen Landmarken
   1. Distales, anteriores Fußgelenkszentrum
   2. Distale tibiale Vorderkante
∘ Einstellbarkeit folgender Parameter:
   1. Varus/Valgus Schnittwinkels
   2. Anteriore/posteriorer Schnittwinkel (Slope)
∘ Einfaches und traumatisches Entfernen der Ausrichtungsvorrichtung vom Bein des Patienten
∘ Vergrößern/Verkleinern des Längsspektrums der Ausrichtungsvorrichtung

- Schaft

Der (teleskopierbare) Schaft ist bevorzugt aufgebaut aus einem Gleitstab und einem Handgriff. Diese beiden Elemente sind teleskopartig miteinander verbunden. Der Schaft verbindet den distalen Instrumententeil (Fußklammer) mit dem proximalen Instrumententeil (Sägeblock, ggf. Adapter für Sägeblock und/oder Befestigungsarm für die Version mit proximaler Befestigung).

Außerdem erfüllt der Schaft zusätzlich folgende Funktionen:
∘ Er unterstützt die Ausrichtung der Ausrichtungsvorrichtung hinsichtlich aller vorstehend genannten drei Parameter. Dies erfolgt, indem der Schaft optisch und/oder haptisch durch gleichzeitiges Tasten der Tibiavorderkante (mit Zeig-, Mittelfinger und Daumen) und der anterioren Schaftkante (mit dem Handgrund zwischen Zeigefinger und Daumen) zur Tibiavorderkante referenziert wird.
∘ Er beinhaltet bevorzugt eine Feststelleinheit (z.B. Hand-Schraube mit gefedertem Klemmelement), die zur Sicherung der eingestellten Ausrichthöhe für die Ausrichtungsvorrichtung dient. Dabei erfolgt die Höheneinstellung der Ausrichtungsvorrichtung in der Version "Anteriore Fixierung" ausschließlich nur über diese Funktion. Für die Version "Proximale Fixierung" existiert bevorzugt zusätzlich noch ein adaptiv anbringbarer, vertikal verschiebbarer Schlitten, an dem dann der Sägeblock angebracht bzw. anbringbar ist.
∘ Eine weitere Funktion der Feststelleinheit ist, bei der Ausrichtung der Ausrichtungsvorrichtung, die zunächst abgeschätzte und noch nicht endgültig festgelegte Höheneinstellung zu sichern. Dies erfolgt mittels Selbsthemmung, beispielsweise realisiert mit einem gefederten Stift in der Feststelleinheit, der gegen den distalen Schaftanteil der Ausrichtungsvorrichtung drückt. Dies ermöglicht dem Anwender, Korrekturen bezüglich Varus/Valgus-, Rotation und Höhenausrichtung gleichzeitig durchzuführen. Die einstellbare Selbsthaftung verhindert das Zusammenrutschen der Ausrichtungsvorrichtung aufgrund der Schwerkraft und erleichtert somit den Handlings- und Zeitaufwand und wirkt sich somit positiv auf OP-Zeit und somit auf OP-Team und Patienten aus.
   Die Feststelleinheit kann in unterschiedlicher Ausführung realisiert werden.
   1. Durch eine Lösung mit Klemmfunktion und selbsthaftende Gleitfunktion oder
   2. Mit Klemmfunktion, selbsthaftende Gleitfunktion und ganz geöffnet mit frei fallender (Free-Falling-) Funktion

- Adapter zur Befestigung des Sägeblocks am Schaft

Ein bevorzugt vorgesehener Sägeblockadapter befindet sich am proximalen Ende der Ausrichtungsvorrichtung (insbesondere am Handgriff) und hat folgende Funktionen.
∘ Aufnahme der Tibia-Sägeböcke für das rechte oder linke Bein.
∘ Freigleitende Anordnung am Schaft, insbesondere im Zusammenspiel mit der Version "Proximale Fixierung" für die endgültige Schnitthöheneinstellung. Der Einsatz des Freigleitens ist auch bei der Version "Anteriore Fixation" denkbar, insbesondere, wenn der Adapter zunächst in einer Mittenposition fixiert ist. Dadurch wird eine einfachere Höhenkorrektur nach Arretieren der Feststelleinheit möglich.

- Proximale Fixationseinheit

**Eine wahlweise** angeordnete "Proximale Fixationseinheit" besteht im Wesentlichen aus einem horizontalen Auslegearm und einem vertikalen Tragpfeiler. Die "Proximale Fixationseinheit", bzw. der vertikale Tragpfeiler kann am proximalen Ende des Schafts in der Version "Anteriore Fixierung" wahlweise angebracht werden. Sie hat folgende Funktionen:
∘ Einfaches Fixieren und Lösen der "Proximalen Fixationseinheit" im proximalen Schaftteil der Version "Anteriore Fixierung"
∘ Horizontale Verschiebbarkeit des Auslegearms im Tragpfeiler ggf. mit Selbsthaftung, wenn keine bis geringe Schiebekräfte von außen wirken. Durch die Verschiebbarkeit des Auslegearms wird garantiert, dass die Ausrichtungsvorrichtung kompatible zu den weltweit gegeben Tibia-Anatomien ist und somit der Anwenderr jederzeit den von ihm vorgesehen Slope einstellen kann.
∘ Sicherung gegen Rotation und lateralem Versatz durch Fixation an der Eminentia- Intercondylaris Tibiae mittels vorzugsweise zwei Schlagpins.
∘ Lösen der Pins mit Schlaghebel
∘ Führen des Sägeblockadapters während der Schnitthöheneinstellung

- Einstellbarer Schnitthöhentaster

Der Schnitthöhentaster kann wahlweise an dem proximalen Tibia-Sägeblock angebracht werden und besteht im wesentlich aus den folgenden Elementen:
∘ Adapterschnittstelle zum Tibia-Sägeblock mit Rast-und Löse-Mechanismus
∘ Selbsthaltender, gleitender Tastarm mit Tastspitze
∘ Einheit zur Höhenverstellung

Der einstellbare Schnitthöhentaster hat folgende Funktionen:
∘ Einfache Montage und Demontage des Schnitthöhentasters an den/die Tibia-Sägeblock(s).
∘ Abtasten einer knöchernen Landmarke mit der Tastspitze. Die vom Anwender gewählte Landmarke ist die Referenz, gegen die die Schnitthöhe eingestellt wird.
∘ Horizontal Verschiebbarkeit des Höhentasters zur Adaption an die verschiedenen Anatomien der epiphysären Tibia und zum Erreichen des medialen und lateral tibialen Kondylus von derselben Adapterstelle aus.
∘ Einstellung der Schnitthöhe

- Proximaler Tibia-Sägeblock

Es gibt bevorzugt verschiedene Varianten von Tibiassägeblöcken. Die Varianten unterscheiden sich durch folgende Merkmale:
∘ Einsetzbar für rechte und linke Tibia
∘ Adaptiert an die zwei Versionen " Anteriore Fixierung" und Proximal Fixierung"

Die Sägeblöcke haben folgende Funktion bzw. Funktionselemente:
∘ Führung des Sägeblattes für den Knochenschnitt
∘ Löcher für die Befestigung des Sägeblocks am Knochen und zu Korrektur der Schnitthöhe um bis zu ±4 mm
∘ Adapterschnittstelle zu Montage an die Ausrichtungsvorrichtung/Schaft über den (Sägeblock-)Adapter
∘ Adapterschnittstelle für die Montage des Schnitthöhentasters

Die vorstehenden, separat schütz- und damit beanspruchbaren Erfindungsgegenstände werden nachfolgend detaillierter beschrieben:
**Die** vorliegende Erfindung besteht darin, eine Ausrichtvorrichtung insbesondere für eine tibiale Resektionsführung bereitzustellen, mit einem teleskopartig ausziehbaren Schaft (Teleskopvorrichtung), der an seinem proximalen Endbereich mit einem Sägeblock bestückbar/bestückt ist und an seinem distalen Endbereich eine Art (Fuß-)Fesselvorrichtung, insbesondere eine Klemmvorrichtung aufweist, die zumindest zwei gegeneinander wirkende sowie schwenkbare Klammerelemente/Klemmarme zum Klemmen des distalen Endes beispielsweise einer Tibia eines Patienten umfasst, wobei die Klammerelemente der Klemmvorrichtung jeweils bogenförmig ausgebildet sind und so zueinander ausgerichtet sind, dass diese in Richtung der Längsachse der Ausrichtvorrichtung/des Schafts betrachtet zwischen sich einen ovalförmigen Bereich ausbilden, der zur klemmenden Aufnahme des distalen Bereiches der Tibia ausgebildet ist. Die Klammerelemente sind erfindungsgemäß jeweils federelastisch ausgebildet bzw. aus einem federelastischen Material gefertigt, so dass die Klemmvorrichtung der Ausrichtvorrichtung unter Ausnutzung der Federeigenschaften der Klemmelemente vorzugsweise einhändig von der Tibia abziehbar ist.

In anderen Worten ausgedrückt hat die am distalen Endabschnitt der Ausrichtungsvorrichtung bzw. des teleskopierbaren/ausziehbaren Schafts angeordnete Klemmvorrichtung einen vorzugsweise Y-förmigen Tibia-Anlageblock u.a. bestehend aus zwei im Wesentlichen V-förmig auseinanderstrebenden (einen Schlitten bildenden), starren Anlagearmen, an deren jeweils freien Endbereichen die federelastisch biegbaren/nachgebenden Klemmarme schwenkbar angelenkt sind, die jeweils wiederum in Schließ-Schwenkrichtung/Umklammerungsrichtung bogenförmig vorgeformt sind. Werden die so geformten Klemmarme in Umklammerungsrichtung zueinander verschwenkt (Fußklammer geschlossen), ergibt sich in Draufsicht die vorstehend genannte ovale Umklammerungsform, wodurch eine hiervon umklammerte Tibia im Köchelbereich eines Patienten nahezu längs des gesamten Klammerumfangs, insbesondere von hinten, seitlich und von vorn gleichzeitig kraftbeaufschlagt wird.

Durch die federelastische Ausbildung der Klammerelemente sind diese darüber hinaus gewebeschonend, d.h. atraumatisch, mit einer hohen Translations- und Rotationsstabilität am distalen Ende der Tibia befestigt. Wenn hierbei auf das untere Ende der Tibia Bezug genommen wird, ist damit der Bereich des unteren Tibiadrittels einschließlich des Fußgelenkes bis direkt zum Kontakt des Fußrückens gemeint.

Die Ausrichtungsvorrichtung bzw. die Fußfesselvorrichtung (Fußklammer) umfasst weiterhin bevorzugt einen Ratschen-Mechanismus, über welchen die Klammerelemente jeweils am Tibia-Anlageblock der Fußfesselvorrichtung gelagert sind und durch den die Klammerelemente unabhängig voneinander mit unterschiedlich hohen Vorspannkräften vorspannbar sind.

Aufgrund der Federelastizität haben die Klammerelemente in vorteilhafter Weise eine Elastizität bei gleichzeitig hoher Haltekraft und eine hohe Rotations- und Translationsstabilität gegenüber ungewolltem Verstellen der gesamten Ausrichtungsvorrichtung. Diese hohe Stabilität wird dadurch erreicht, dass die Klammerelemente über den Ratschen-Mechanismus geschlossen werden und zusätzlich gehalten werden.

Des Weiteren Verschränken sich die beiden Klemmelemente an deren jeweiligen freien Enden, wenn die Fußklammer geschlossen ist. Durch dieses Verschränken ist es in vorteilhafter Weise möglich, gleichzeitig eine seitliche und eine von seitlich posterior kommende Haltekraft zu applizieren. Dadurch wird in vorteilhafter Weise eine hohe flächige Schließ- und Haltekraft mehrdimensional an der Tibia appliziert.

In besonders vorteilhafter Weise ist diese Haltekraft für alle anatomischen Umfangsgrößen der Tibia konstant, da die federnden Klammerelemente erst beansprucht werden, wenn sie in Kontakt mit der Tibia treten. Die Klammerelemente erfüllen somit in vorteilhafter Weise eine Doppelfunktion, da die Druckkraft zum Halten der Tibia erst aufgebracht wird, wenn die Tibia die Klammerelemente berührt.

Gemäß einer weiteren bevorzugten Ausführungsform sind die Klammerelemente zusätzlich jeweils gabelförmig ausgebildet und die Zinken der jeweiligen Gabel sind so zueinander versetzt angeordnet, dass diese im geschlossenen Zustand (geschlossene Fußklammer) überlappend ineinandergreifen, so dass die Tibia fest gehalten ist. Die Klammerelemente bewirken somit ein gleichzeitiges Greifen und Halten der Tibia von hinten, seitlich und von vorne. Durch die Ausbildung der Klammerelemente in Gabelform können sich diese demnach im posterioren Bereich der Tibia verschränken und diese somit gegen den Anlageblock drücken. In besonders vorteilhafter Weise sind die Klammerelemente so ausgebildet, dass sich diese im Bereich des Fußgelenkes verschränken.

Vorzugsweise sind die jeweiligen Spitzen der Zinken entgegen der jeweiligen Bogenform der Klammerelemente geformt, so dass der Abziehvorgang von der Tibia verletzungsfrei erfolgt. Dadurch, dass die Klammerelemente in ihrem Endbereich gebogen sind, können diese sehr gewebeschonend von der Tibia abgezogen werden (ohne zuvor den Ratschen-Mechanismus öffnen zu müssen), wobei ein Einschneiden der Enden in das Gewebe ist in vorteilhafter Weise verhindert bzw. gemindert wird.

Ein zweiter, ggf. separat beanspruchbarer Kern der vorliegenden Erfindung besteht darin, dass die Teleskopvorrichtung, d.h. der teleskopierbare Schaft in seinem proximalen Bereich vorzugsweise zusätzlich zu der vorstehend beschriebenen Fußklammer eine Entkopplungsvorrichtung d.h. einen Sägeblock-Adapter (kann auch als Entkopplungsvorrichtung bezeichnet werden) aufweist, der dazu ausgebildet ist, bei (manueller) Aktivierung/Auslösung den Sägeblock von der Teleskopvorrichtung bzw. dem Schaft zu trennen/entkoppeln. Auf diese Weise ist es in einfacher Weise möglich, die Ausrichtungsvorrichtung/Ausrichthilfe, d.h. den teleskopierbaren Schaft mit quasi einem Handgriff vom Schneid-/Sägeblock abzuziehen. Ist in einem bevorzugten Fall die Fußklammer gemäß dem vorstehend beschriebenen ersten Kerngedanken der vorliegenden Erfindung ausgebildet, können infolge des sich hierbei einstellenden Synergieeffekts die Klammerelemente aufgrund ihrer Eigenelastizität unter federelastischen Aufspreizen (und ohne die Fußklammer am Ratschen-Mechanismus zu öffnen) einfach von der distalen Tibia (ebenfalls einhändig) abgezogen werden, sodass die Ausrichtungsvorrichtung insgesamt mit einer einzigen Bewegung abgenommen werden kann.

Mit anderen Worten ausgedrückt, kann in vorteilhafter Weise durch die konstruktive Auslegung der Fußklammer und dem proximalen Sägeblockadapter/Adaperschnittstelle der telekopierbare Schaft (bzw. die Ausrichtungshilfe) gewebeschonend, d.h. atraumatisch, in einer einfachen (einzigen) Bedienaktion von der Tibia des Patienten abgezogen werden.

Entscheidend hierfür ist, dass die Halteelemente der Fußklammer ausreichend elastisch ausgebildet sind, sodass man diese ohne einen Löse-Mechanismus zu bedienen, einfach von der distalen Tibia abziehen kann und dass diese dabei gleichzeitig eine ausreichend hohe Rotations- und Translationsstabilität gegenüber ungewolltem Verstellen der Ausrichtungsvorrichtung aufweisen. Zusätzlich ist es für diesen Fall vorteilhaft, dass die Entkopplungsvorrichtung, also die Adapterschnittstelle, für den Tibia-Sägeblock derart gestaltet ist, dass die Handhabe zum Lösen (vorzugsweise ein Hebel oder Release-Button) der Ausrichtungsvorrichtung von dem Sägeblock mit einem einfachen Daumendruck betätigbar ist, sodass der Schaft von der zugehörigen ganzen Hand umgriffen werden kann. Mit der Ausrichtungsvorrichtung, welche die beschriebenen Vorrichtungen und Elemente aufweist, ist es somit möglich, nach Befestigung des Sägeblocks an dem Knochen und nach der Bedienung des Release-Buttons per Daumendruck die Ausrichtvorrichtung mit einer Hand von der Tibia des Patienten abzuziehen, ohne dass eine weitere Bedienaktion notwendig ist.

Gemäß einer bevorzugten Ausführungsform zum zweiten Kerngedanken der vorliegenden Erfindung weist die Teleskopvorrichtung bzw. der telekopierbare Schaft in seinem proximalen Bereich einen Handgriff auf, der so ausgestaltet ist, dass dieser von einer Hand umgreifbar ist und dass die Entkopplungsvorrichtung/Sägeblockadapter zur Aktivierung oberhalb des Handgriffes ein Druckelement aufweist, welches vorzugsweise in einem Winkel A zwischen 90° und 150°, weiter vorzugsweise in einem Winkel zwischen 95° und 120° und insbesondere in einem Winkel A von 100° zu der Längsachse der Teleskopvorrichtung angeordnet ist, so dass das Druckelement von dem Daumen der einen Hand aktivierbar ist.

Durch die Anordnung des Druckelementes in dem bevorzugten Winkel A von 100° Grad wird eine besonders gut zu erreichende Position des Druckelementes geschaffen. Dadurch ist das Druckelement in vorteilhafter Weise mit einem einzigen Daumendruck auslösbar und die Ausrichtungsvorrichtung ist durch eine einzige Hand des Bedieners von der Tibia entfernbar. Dadurch sind in vorteilhafter Weise zusätzliche Handbewegungen bzw. Verfahrensschritte verhindert, wodurch die Ausrichtungsvorrichtung in einem einzigen Bewegungsvorgang abnehmbar ist.

Konkreter ausgedrückt ist der Sägeblockadapter auf Seiten des Schafts als männliche Adapterschnittstelle etwa nach Art eines Steckers beispielsweise mit zwei zapfenförmigen Vorsprüngen ausgebildet, an welcher eine Klammer oder ein Haltebügel vorzugsweise wippenartig gelagert ist. Der Haltebügel bildet an seinem einen Endabschnitt mindestens eine Eingriffs-Hinterschneidung (z.B. Rasthaken), wohingegen an seinem anderen Endabschnitt das Druckelement für ein manuelles Verschwenken des Haltebügels vorzugsweise in Freigaberichtung angeordnet ist.

Demzufolge ist am Sägeblock eine weibliche Adapterschnittstelle etwa nach Art einer Steckdose beispielsweise mit zwei (Sack-)Bohrungen angeordnet/ausgebildet, in welche der Stecker insbesondere drehfest eingreifen kann, wobei dieser Eingriff mittels des Haltebügels beispielsweise durch Hintergreifen von Haltekanten auf Seiten des Sägeblocks gesichert wird. Es sei an dieser Stelle ausdrücklich darauf hingewiesen, dass die männliche Adapterschnittstelle natürlich auch auf Seiten des Sägeblocks und die weibliche Adapterschnittstelle am Schaft vorgesehen sein können.

Vorzugsweise ist der Sägeblockadapter als ein separates (eigenständiges) Bauteil ausgeformt mit einer Andockstelle, an welcher der Adapter mit dem Handgriff vorzugsweise an dessen proximalem Ende fest verbindbar ist. Weiter vorzugsweise hat der Sägeblockadapter ein vertikales Durchgangsloch, dergestalt, dass der eine Teil des teleskopierbaren Schafts (Gleitstabelement, an welchem distal die Fußklammer angeordnet ist) sowohl den Handgriff als auch den Adapter gleitend (vollständig) durchdringen kann.

Des Weiteren besteht die vorliegende Erfindung gemäß einem dritten, ggf. separat beanspruchbaren Erfindungskern darin, eine Ausrichtungsvorrichtung für eine tibiale Resektionsführung bereitzustellen, welche die folgende Komponenten umfasst:
- eine Klemmvorrichtung (Fußklemme) zum Klemmen des distalen Endes einer Tibia eines Patienten vorzugsweise gemäß dem ersten Kerngedanken der Erfindung,
- eine Werkzeugführungsvorrichtung/Sägeblock zum Führen eines Werkzeuges/Säge während der Resektion der Tibia,
- eine Teleskopvorrichtung/teleskopierbarer Schaft vorzugsweise gemäß dem zweiten Kerngedanken der Erfindung, die mit der Werkzeugführungsvorrichtung/Sägeblock und mit der Klemmvorrichtung verbunden ist und die dazu ausgebildet ist, die Vorrichtungen gegenüber der Tibia auszurichten, wobei die Teleskopvorrichtung ein Handgriffelement bzw. ein Handgriff aufweist, welcher dazu ausgebildet ist, ein verschiebbar darin gelagertes Gleitstabelement (mit distal daran angeordneter Klemmvorrichtung) aufzunehmen, wobei die Teleskopvorrichtung ein (manuell einstellbares) Sicherungselement aufweist, welches zwischen dem Handgriff und dem Gleitstabelement angeordnet ist und welches in eine erste Stellung (manuell) einstellbar ist, in der eine erste Druckkraft zwischen dem Handgriff und dem Gleitstabelement bewirkt ist und welches zusätzlich in eine zweite Stellung einstellbar ist, in der eine zweite Druckkraft zwischen den Elementen unterschiedlich (größer als) zur ersten Druckkraft bewirkt ist.

Es wird somit ein Sicherungselement bereitgestellt, welches mit der Teleskopvorrichtung verbunden ist und welches dazu ausgebildet ist, zwischen dem Handgriff und dem Gleitstabelement angeordnet zu werden und welches eine erste und eine zweite Druckkraft zwischen dem Handgriff und dem Gleitstabelement erzeugen kann. Durch die Bereitstellung von zwei unterschiedlichen Druckkräften zwischen den Elementen ist die Teleskopvorrichtung in Abhängigkeit der Anforderungen des behandelnden Arztes leicht anpassbar und bedienbar.

Eine bevorzugte Ausführungsform ist dadurch bereitgestellt, dass in der ersten Stellung die dabei erzeugte (erste) Druckkraft in einer vertikalen Anordnung der Ausrichtvorrichtung, d.h. in Längsrichtung des telekopierbaren Schafts eine Selbsthemmung zwischen dem Handgriffelement und dem Gleitstabelement bewirkt und dass in der zweiten Stellung die dabei erzeugte (zweite) Druckkraft eine Fixierung der Elemente zueinander bewirkt

**In** der ersten Stellung ist somit in vorteilhafter Weise ein lediglich selbsthemmender Gleitstab bewirkt, wobei die Selbsthemmung zumindest ausreicht, eine Relativverschiebung von Handgriff und Gleitstabelement aufgrund der Schwerkraft zu verhindern und in der zweiten Stellung ist zusätzlich eine Fixierung des Gleitstabes im Handstück mittels des Sicherungselementes bewirkt. Die Fixierung beispielsweise mittels Reibklemmung wird in vorteilhafter Weise dadurch erreicht, dass das Sicherungselement, also eine Feststell-/ Befestigungsschraube, bis auf Anschlag eingedreht wird. Die selbsthemmende Freigabe des Gleitstabes für dessen axiale Verschiebbarkeit im Handgriff wird dadurch erreicht, dass in dem Sicherungselement, also der Feststell-/Befestigungsschraube ein gefederter Stift eingebaut ist, der bei teilgeöffneter bis vollständig geöffneter Schraube einen Reibschluss mittels (federkraftabhängiger) Klemmkraft zwischen dem Handgriff und dem Gleitstab erzeugt. Diese Klemmkraft ist so ausgelegt, dass die erzeugte Haftreibung der Schwerkraft entgegenwirkt, wobei die wirkende Masse durch die am proximalen und/oder distalen Ende der Ausrichtungsvorrichtung befestigten Elemente maßgeblich beeinflusst wird. In vorteilhafter Weise wird durch die Klemmkraft bewirkt, dass der behandelnde Arzt oder der Anwender zu jedem Zeitpunkt die Elemente für die Höheneinstellung loslassen kann und die eingestellte Höhe erhalten bleibt. Die Ausrichtungsvorrichtung fällt somit in vorteilhafter Weise nicht in sich zusammen, wie dieses aufgrund der Schwerkraft zu erwarten wäre. Dadurch kann der Anwender jederzeit die Höhe nachkorrigieren, während er sich auf die Einstellung der anderen Parameter, bspw. Varus/ Valgus und/oder Slope, konzentrieren kann. Insgesamt ist dadurch der Arbeitsablauf während der Behandlung in vorteilhafter Weise erleichtert. Erst wenn die (Grob-) Höhenjustage abgeschlossen ist wird die Feststell-/Befestigungsschraube bis auf Anschlag eingedreht, wodurch die Federwirkung aufgehoben wird und eine Klemmkraft in Abhängigkeit der Einschraubkraft zwischen dem Handgriff und dem Gleitstab(element) wirkt, die viel höher ist als die zuvor wirkende Federkraft und letztlich die beiden Elemente gegeneinander fest verspannt.

**In** einer weiter bevorzugten Ausführungsform ist das Sicherungselement zusätzlich so einstellbar, dass in einer dritten Stellung eine dritte Druckkraft zwischen den Elementen wirkt, die so ausgebildet ist, dass in einer vertikalen Anordnung der Ausrichtvorrichtung eine selbsttätiges Ineinandergleiten der Elemente (Handgriff, Gleitstab) aufgrund der angreifenden Schwerkraft bewirkt ist. **In** dieser dritten Stellung ist somit ein frei fallender Gleitstab bereitgestellt.

Dieses wird beispielsweise dadurch erreicht, dass der Klemmstift des Sicherungselementes keine oder nur geringe/vernachlässigbare Haftreibung mehr erzeugt, welches wiederum dadurch bewirkt ist, dass bei vollständiger Öffnung der Befestigungsschraube, die Kraft der Feder, die auf den Klemmstift wirkt so gering ist, dass die beschriebene Haftreibung nicht mehr relevant ist und nur noch eine Gleitreibung zwischen dem Klemmstift und dem Gleitstab vorhanden ist.

Ein weiterer, ggf. separat zu beanspruchender vierter Kerngedanke der vorliegenden Erfindung besteht darin, eine Ausrichtungsvorrichtung für eine tibiale Resektionsführung bereitzustellen, die folgende Komponenten umfasst:
- eine Klemmvorrichtung (Fußfessel) vorzugsweise gemäß dem ersten Kerngedanken der Erfindung zum Klemmen des distalen Endes einer Tibia eines Patienten;
- eine Werkzeugführungsvorrichtung/Tibia-Sägeblock zum Führen eines Werkzeuges während der Resektion der Tibia,
- eine Teleskopvorrichtung/teleskopierbarer Schaft vorzugsweise gemäß dem zweiten und/oder dritten Kerngedanken der Erfindung, die mit der Werkzeugführungsvorrichtung und mit der Klemmvorrichtung verbunden/verbindbar ist und die dazu ausgebildet ist, die Vorrichtungen gegenüber der Tibia auszurichten, wobei die Werkzeugführungsvorrichtung eine Aufnahmeausnehmung/Andock-/Adapterschnittstelle aufweist, die dazu ausgebildet ist, ein Höhentastelement bzw. Schnitthöhentaster (verstellbares Antastelement) zur Ertastung/Feineinstellung der Resektionshöhe wiederlösbar aufzunehmen.

Gemäß der vorstehenden Ausführung wird der Schnitthöhentaster an dem proximalen Tibia-Sägeblock wahlweise angebracht und besteht im wesentlich aus den folgenden Elementen:
- Adapterschnittstelle zum Tibia-Sägeblock vorzugsweise mit Rast-und Löse-Mechanismus
- Ggf. selbsthaltender, jedoch gleitender Tastarm mit Tastspitze
- Einheit zur (manuellen) Höhenverstellung

Der (einstellbare) Schnitthöhentaster hat folgende Funktionen:
- Einfache Montage und Demontage des Stylus an die einzelnen ggf. unterschiedlich zueinander gestalteten Tibia-Sägeblocks (z.B. für linkes und rechtes Bein)
   ∘ Am distalen Ende des Stylus ist ggf. ein gefederter Rast-Mechanismus angebracht, der den Stylus arretiert, nachdem der Stylus in dafür vorgesehene Löcher/Aufnahmeausnehmungen/Andockstellen der **Tibiasägeblöcke** eingebracht/eingesteckt wurde. Um die Steckachse bleibt der Stylus relativ zum jeweiligen Sägeblock rotierbar.
   ∘ Die wahlweise axiale Arretierung erfolgt beispielsweise über eine "gefedert Nase", die beim Einbringen aufgrund der abgeschrägten distalen Anlagenfläche lateral zurückgeschoben wird und bei vollständigen eintauchen in den Tibiasägeblock in eine Nut im Tibiasägeblock einrastet.
   ∘ Aus der Arretierung gelöst, kann die "gefederte Nase mittels eines Hebels werden, der die Nase bei Bedienung gegen die Feder zurückzieht und somit die Rastung aufhebt. In diesem Zustand, kann der Stylus vom Sägeblock einfach abgezogen werden.
- Abtasten einer knöchernen Landmarke mit der Tastspitze. Die vom Anwender gewählte Landmarke ist die Referenz, gegen die die Schnitthöhe eingestellt wird.
   ∘ Die Landmarke wird mit der Tastspitze detektiert, die am posterioren Ende des Tastarms angebracht ist. Durch die Feinheit der Tastspitze können sehr kleine knöcherne Strukturen mittels Augenkontrolle optisch sehr gut und genau detektiert werden.
- Horizontale Verschiebbarkeit des Höhentasters zur Adaption an die verschiedenen Anatomien der epiphysären Tibia und zum Erreichen des medialen und lateral tibialen Kondylus von der selben Adapterstelle aus.
   ∘ Mit Hilfe des vorzugsweise rotierbaren Stylus und des entlang seiner Hauptachse vorzugsweise verschiebbaren Tastarms kann jede knöcherne Landmarke an der proximalen Oberfläche der Tibiakondylen erreicht werden. Dabei ist die Dimension des Tastarms so ausgelegt, dass die weltweit existierende Anatomie (von Asiaten, Kaukasiern, etc.) berücksichtigt sind.
   ∘ Damit die gewünschte, ausgefahren Länge des Tastarmes beibehalten wird, kann der Tastarm selbsthemmend z.B. mittels Reibschluss gegen axiales Verschieben gesichert sein.
- Einstellen der Schnitthöhe
   ∘ Durch das Einrasten des Stylus im Tibia-Sägeblock, und einem definierten Anschlag des Stylus am Sägeblock, wird der Abstand der Tastspitze des Stylus bezogen auf die Unterkante des Sägeschlitzes im Sägeblock determiniert.
   ∘ Angezeigt wird die eingestellte Schnitthöhe durch Zahlen beispielsweise am Umfang eines Schraubenkopfes des verstellbaren Höhentasters. Die Zahl, die die eingestellte Höhe angibt, wird vorzugsweise mit einem Zeigeelement am anterioren Ende der Halteeinheit des Tastarms indiziert.
   ∘ Das Einstellen der Tibiaschnittdicke von 0 bis 16 mm (oder 0 bis 14 mm) wird mit bevorzugt nur **einer** Umdrehung des Schraubenkopfes erreicht. Die Höhenverstellung erfolgt in diesem Fall mittels einer Wendel in einem Führungselement des Schraubenkopfes. Ein Anschlagelement am oberen Ende des Führungselements kann ggf. vorgesehen sein, um zu verhindern, dass der Schraubenkopf vollständig vom Stylus herunter gedreht werden kann.
- Finales Einstellen der Schnitthöhe und Ausrichten der Ausrichtungsvorrichtung für den proximalen Tibiaschnitt:
   ∘ Nach Einstellen der gewünschten Schnitthöhe, wird der Taster gegen die vom Anwender ausgewählte Landmarke gefahren und die Ausrichtungsvorrichtung ausgerichtet. Das Anfahren der Landmarke erfolgt durch Verschieben des Handgriffs mit Aufbauelemente/Sägeblock-Adapter auf dem Gleitstab. Ist die Ausrichtungsvorrichtung in Höhe, Varus/Valgus und Slope wie vom Anwender gewünscht ausgerichtet, wird der Sägeblock endgültig mit einem Fixationsspin durch die dafür im Sägeblock vorgesehen Fixierlöcher am Knochen fest verankert. Danach muss zumindest der Stylus entfernt werden, um den tibialen Sägeschnitt durchführen zu können.

Das Höhentastelement, welches hier auch als Schnitthöhentaster bezeichnet wird, kann, wie vorstehend ausgeführt wurde, am proximalen Tibia-Sägeblock angebracht werden. Durch die Verwendung des Höhentastelements wird in vorteilhafter Weise der Abstand der Tastspitze des Höhentastelementes bezogen auf die Unterkante eines Sägeschlitzes der Werkzeugführungsvorrichtung/Sägeblock definiert. Durch die Tastspitze des Höhentastelements können in vorteilhafter Weise sehr feine knöchernde Strukturen mittels Augenkontrolle sehr genau detektiert werden. Insgesamt wird durch das Höhentastelement eine besonders genaue Ausrichtung der gesamten Ausrichtungsvorrichtung erreicht.

**In** einer bevorzugten Ausführungsform ist die Aufnahmeausnehmung eine (Sack- oder Durchgangs-) Bohrung, die sich ausgehend von der Oberseite der Werkzeugführungsvorrichtung/Sägeblocks entlang der Längsachse der Ausrichtungsvorrichtung/Schafts erstreckt, wobei das Höhentastelement ein einen Rastmechanismus aufweisendes Einsteckelement/Zapfen aufweist, welches in die Aufnahmeausnehmung wiederverrastbar einsteckbar ist.

Am distalen Ende des Höhentastelementes (das auch als Stylus bezeichnet wird), d.h. am distalen (freien) Ende/Endabschnitt des Einsteckelements/Zapfens, ist der gefederte Rastmechanismus angebracht, der den Stylus in der Aufnahmeausnehmung des Sägeblocks arretiert. Der Stylus bleibt in vorteilhafter Weise um die Steckachse rotierbar, so dass eine leichte Ausrichtung des Tastelementes ermöglicht ist.

**In** einer weiter bevorzugten Ausführungsform ist der Rastmechanismus vorzugsweise in dem Einsteckelement selbst angeordnet ist und umfasst eine Rastnase, die im eingesteckten Zustand so hinter die Aufnahmeausnehmung greift, dass das Höhentastelement darin axial gehaltert ist.

Die axiale Arretierung erfolgt in vorteilhafter Weise über eine "gefederte Nase", die beim Einbringen des Stylus aufgrund ihrer abgeschrägten distalen Anlage-/Abgleitfläche selbsttätig lateral zurückgeschoben wird und bei vollständigem Eintauchen in die Werkzeugführungsvorrichtung in eine Hinterschneidung in der Werkzeugführungsvorrichtung einrastet. Die "gefederte Nase" kann zusätzlich mittels eines Hebels oder einer Betätigungstaste manuell aus der Arretierungsposition entgegen deren Federvorspannung lateral zurückgezogen werden, um somit den Rasteingriff aufzuheben. **In** vorteilhafter Weise kann in diesem Zustand der Stylus von der Werkzeugführungsvorrichtung, also von dem Sägeblock, einfach abgezogen werden.

Des Weiteren besteht ein ggf. separat beanspruchbarer fünfter Erfindungsgedanke der vorliegenden Erfindung darin, eine Ausrichtungsvorrichtung für eine tibiale Resektionsführung bereitzustellen, welche bevorzugt die folgenden Komponenten umfasst:
- eine Klemmvorrichtung (Fußfessel) vorzugsweise nach dem ersten Erfindungsgedanken der vorliegenden Erfindung zum Klemmen des distalen Endes einer Tibia eines Patienten;
- eine Werkzeugführungsvorrichtung/Sägeblock zum Führen eines Werkzeuges während der Resektion der Tibia,
   - eine Teleskopvorrichtung bzw. teleskopierbarer Schaft vorzugsweise gemäß zumindest einem aus dem zweiten bis vierten Kerngedanken der Erfindung, die an ihrem proximalen Endabschnitt mit der Werkzeugführungsvorrichtung und an ihrem distalen Endabschnitt mit der Klemmvorrichtung verbunden/verbindbar ist und die dazu ausgebildet ist, die Vorrichtungen gegenüber der Tibia auszurichten, wobei die Teleskopvorrichtung in ihrem proximalen Bereich zur wahlweisen Aufnahme einer proximalen Fixationseinheit bzw. Einschlagvorrichtung eine Einschlagvorrichtungsaufnahme aufweist, die durch den zumindest teilweise als Hohlkörper ausgebildeten Schaft der Teleskopvorrichtung, vorzugsweise Gleitschaft-/Gleitstabelement gebildet ist.

Wie bereits vorstehend erwähnt, wird die erfindungsgemäße Ausrichtungsvorrichtung durch eine einfache instrumentelle Ergänzung von einer Lösungsvariante in eine zweite umfunktioniert. Bei den beiden Varianten handelt es sich um die "Anteriore Fixierung" und die "Proximal Fixierung".

Gelöst wird das Umfunktionieren dadurch, dass auf die Version "Anteriore Fixierung" eine zusätzliche "Proximale-Fixationseinheit" nämlich die Einschlagvorrichtung aufgesetzt wird und somit die proximale Befestigung (Version "Proximale Fixierung") an der "Eminentia-Intercondylaris Tibiae" möglich wird.

**Die** "Proximale Fixationseinheit" besteht im Wesentlichen aus dem horizontalen Auslegearm mit Schlagpin-Einheit und dem vertikalen Tragpfeiler mit Hebel-Mechanismus zum Klemmen der Einheit an der Ausrichtungsvorrichtung bzw. dem Schaft, insbesondere dem Gleitschaftelement. Die "Proximal Fixationseinheit" wird am proximalen Ende der Ausrichtungsvorrichtung in der Version "Anteriore Fixierung" angebracht. Sie hat folgende Funktionen:
- Einfaches Fixieren und Lösen der "Proximalen Fixationseinheit im proximalen Teil der Version "Anteriore Fixierung"
   ∘ Das Fixieren erfolgt bevorzugt durch einen Hebel-Spreiz-Mechanismus.
   ∘ Durch Betätigen eines Hebels (Daumenpressung nach unten), wird eine Stange innerhalb des Tragpfeilers nach oben bewegt.
   ∘ Die Stange wird hierzu in einer Bohrung in dem distalen Schaftteil (Tragpfeiler) der "Proximale Fixationseinheit" geführt.
   ∘ Am distalen Ende der Stange ist ein, proximal z.B. 45° abgeschrägtes Vierkantelement fest angebracht.
   ∘ Das genannte Vierkantelement stößt an das distale, z.B. ebenfalls 45° abgeschrägte Ende des Schaftes der "Proximalen Fixationseinheit" an, das bevorzugt die identische Vierkantform wie das Vierkantelement an genannter Stange besitzt.
   ∘ Beide Vierkantelemente bilden die Einheit, die in das Vierkant-Schaftrohr des proximalen Endes der Version "Anteriore Fixierung" (im Bereich des Handgriffs) eingeschoben wird.
   ∘ Die bevorzugt 45° Schrägen der beiden Vierkantelemente sind gegengleich zueinander angeordnet.
   ∘ Wird nun das weiter distal angeordnete Vierkantelement durch den Hebel-Mechanismus und die Stange nach proximal gezogen, kommt es aufgrund der gegenläufig angeordneten 45°-Schrägen zu einem seitlichen Versatz der beiden genannten Vierkantelemente.
   ∘ Erfolgt dies, wenn beide Element sich im Vierkantrohr befinden, kommt es zwangsläufig zu einer Verklemmung und somit zur Fixation der "Proximalen Fixationseinheit" im proximalen Ende (im Vierkantrohr) des Schafts der Ausrichtungsvorrichtung in der Version "Anteriore Fixierung"
   ∘ Durch Öffnen des genannten Hebel-Mechanismus wird die Verklemmung gelöst und die "Proximal Fixationseinheit" kann im Ganzen von der Ausrichtungsvorrichtung mit nur einem Handgriff abgenommen werden. Der Hebel zum Lösen wird dabei beim Greifen der "proximalen Fixationseinheit mit Fingern (bevorzugt der Mittelfinger und/oder Ringfinger) nach oben bewegt.
- Horizontale Verschiebbarkeit des Auslegearms im Tragpfeiler mit Selbsthaftung, wenn keine bis geringe Schiebekräfte von außen wirken.
   ∘ Durch die Verschiebbarkeit des Auslegearms, wird garantiert, dass die Ausrichtungsvorrichtung kompatibel zu den weltweit gegebenen Tibia-Anatomien ist und somit der Anwenderr jederzeit den von ihm vorgesehen Slope einstellen kann.
- Sicherung gegen Rotation und lateralen Versatz durch Fixation an der Eminentia-Intercondylaris Tibiae mittels bevorzugt zweier Schlagpins.
- Lösen der Pins mit ggf. zusätzlichem Schlaghebel
- Führen des Sägeblocks bzw. Sägeblockadapters in Schaftlängsrichtung während der Schnitthöheneinstellung
   ∘ Für die Höheneinstellung des Sägeblocks muss der Tibia Sägeblockadapter, angebracht am proximalen Ende der Ausrichtungsvorrichtungs-Version "Anteriore Fixierung" für axiales Gleiten längs des teleskopierbaren Schafts sowie unabhängig vom Handgriff freigegeben werden.
   ∘ Das Freigeben wird dadurch erreicht, dass ein entsprechender Verriegelungs-Mechanismus betätigt wird, um die Kopplung zwischen dem Sägeblockadapter und dem Handgriff zu lösen. Der Verriegelungs-Mechanismus (Schiebeknopf) ist beispielsweise seitlich am proximalen Ende der Ausrichtungsvorrichtung angebracht.
   ∘ Der Schiebeknopf nimmt in seiner Funktion 2 Stellungen ein:
      1. Position: Arretieren des Tibiasägeblockadapters ≙ "Anteriore Fixation"
      2. Position: Freigabe des Tibiasägeblockadapters ≙ "Proximal Fixation"

In vorteilhafter Weise wird die Ausrichtungsvorrichtung folglich durch eine einfache instrumentale Ergänzung von einer Lösungsvariante in eine zweite umfunktioniert, wobei es sich hierbei um die "Anteriore Fixierung" und die "Proximale Fixationseinheit" gemäß vorstehender Definition handelt.

Weiter vorteilhaft ist die Einschlagvorrichtung an ihrem distalen Ende so in den Hohlkörper des Schaftes d.h. in das zumindest teilweise hohle, sowie proximal offene Gleitschaftelement der Teleskopvorrichtung einführbar, dass über den genannten Klemmmechanismus eine Verklemmung der Einschlagvorrichtung in dem Hohlkörper/Gleitschaftelement bewirkbar ist. Es wird also bevorzugt auf formschlüssige Verbindungen wie Bolzen, Schrauben etc. verzichtet und ausschließlich auf Reibschlussverbindungen gesetzt, die schnell und einfach herstellbar sind.

Gemäß einem ggf. separat beanspruchbaren sechsten Kerngedanken besteht die vorliegende Erfindung vorzugsweise darin, eine Ausrichtungsvorrichtung für eine tibiale Resektionsführung bereitzustellen, die folgende Komponenten umfasst:
- eine Klemmvorrichtung vorzugsweise gemäß dem ersten Kerngedanken der Erfindung, die zumindest zwei gegeneinander wirkende Klammerelemente zum Klemmen des distalen Endes Tibia eines Patienten aufweist, die an einem Kragarm befestigt sind, der dazu ausgebildet ist, in eine Fußklammeraufnahmevorrichtung eingeführt zu werden,
- eine Werkzeugführungsvorrichtung zum Führen eines Werkzeuges während der Resektion der Tibia,
- eine Teleskopvorrichtung vorzugsweise gemäß zumindest einem aus dem zweiten bis fünften Kerngedanken der Erfindung, die mit der Werkzeugführungsvorrichtung und mit der Klemmvorrichtung verbunden ist und die dazu ausgebildet ist, die Vorrichtung gegenüber der Tibia auszurichten, wobei die Teleskopvorrichtung ein Handgriffelement aufweist, welches dazu ausgebildet ist, ein verschiebbar darin gelagertes Gleitschaft-/Gleitstabelement aufzunehmen, wobei die Ausrichtungsvorrichtung ein erstes Gleitschaft-/stabelement mit einer ersten Länge und ein zweites Gleitschaft-/stabelement mit einer zweiten Länge umfasst, wobei die jeweiligen Gleitstabelemente dazu vorgesehen sind, bedarfsweise in das Handgriffelement eingesetzt zu werden, wobei die jeweiligen Gleitstäbe an ihrem distalen Ende vorzugsweise rechtwinklig mit der Fußklammeraufnahmevorrichtung verbunden sind und dass das Verhältnis der ersten Länge des ersten Gleitstabelementes zur zweiten Länge des zweiten Gleitstabelementes vorzugsweise zwischen 1 und 1,5, weiter vorzugsweise zwischen 1,1 und 1,3 und insbesondere 1,27 ist, wobei die Länge jeweils vom proximalen Ende des Gleitstabes bis zu dem Mittelpunkt der Fußklammeraufnahmevorrichtung gemessen wird, so dass durch den ersten und den zweiten Gleitstab jeweils unterschiedliche Längen der Tibia resektionierbar sind.

**In** anderen Worten ausgedrückt betrifft der sechste Kerngedanke der vorliegenden Erfindung die generelle Schaffung von Möglichkeiten zur möglichst kostengünstigen Veränderung des Längenspektrums einer Ausrichtungsvorrichtung vorzugsweise gemäß zumindest einem aus den ersten bis fünften Kerngedanken der Erfindung im Rahmen einer einzigen Ausrichtungsvorrichtung. Hierfür stehen im Wesentlichen zwei Maßnahmen zu Verfügung, die gemeinsam oder unabhängig voneinander ergriffen werden können:
Zunächst besteht die erste Möglichkeit, eine Ausrichtungsvorrichtung der vorliegenden Gattung bereitzustellen mit einem telekopierbaren (ausziehbaren) Schaft bzw. einerTeleskopvorrichtung, an deren proximalem Endabschnitt ein Sägeblock montiert oder montierbar ist und an dessen distalem Endabschnitt eine Klemmvorrichtung bzw. Fußklammer angeordnet oder anbringbar ist. Der ausziehbare Schaft bzw. die Teleskopvorrichtung hat erfindungsgemäß ein Gleitschaft-/stabelement (oder einfach Gleitstab), welches in einem Handgriff axialverschiebbar aufgenommen und vorzugsweise mittels einer Feststelleinrichtung (Feststellschraube) relativ zum Handgriff in einer gewählten/wählbaren Ausziehposition fixierbar ist. Diese Ausrichtvorrichtung umfasst erfindungsgemäß einen Satz an Gleitstäben/Gleitschaftelementen unterschiedlicher Schaft-/Stablänge (mindestens zwei Gleitstäbe mit zueinander unterschiedlichen Stablängen) die wahlweise untereinander austauschbar und in ausgewählter Weise in den Handgriff teleskopartig einführbar sind.

**In** vorteilhafter Weise sind somit unterschiedliche Längen der Tibia, also unterschiedliche Beinlängen, resektionierbar. Dieses wird durch das Auswechseln des Gleitstabes erreicht, der im Rahmen des zur Verfügung stehenden Gleitstabsatzes in unterschiedlichen Längen vorhandenen ist. Es lassen sich somit in vorteilhafter Weise durch die Ausrichtungsvorrichtung sämtliche Längen der Tibia resektionieren.

Des Weiteren besteht die zweite Möglichkeit (diese kann separat oder in Kombination mit der vorstehenden ersten Möglichkeit zur Anwendung kommen), dass die Klammerelemente der Fußklammeraufnahmevorrichtung in Längsrichtung versetzt an dem Kragarm befestigt ist, wobei der Kragarm durch eine Drehung um 180° Grad in einer ersten Stellung und in einer zweiten Stellung in die Fußklammeraufnahmevorrichtung einführbar ist, derart, dass in der ersten Stellung die Klammerelemente zum distalen Ende der Ausrichtungsvorrichtung und in der zweiten Stellung zum proximalen Ende der Ausrichtungsvorrichtung ausgerichtet sind, so dass ein Höhenversatz der Klammerelemente in Längsrichtung bewirkt ist, wenn der Kragarm von der ersten in die zweite Stellung um 180°Grad gedreht in die Fußklammeraufnahmevorrichtung eingeführt wird.

In anderen Worten ausgedrückt wird bevorzugt eine Ausrichtungsvorrichtung der vorliegenden Gattung bereitgestellt mit einem telekopierbaren (ausziehbaren) Schaft bzw. einer Teleskopvorrichtung, an deren proximalem Endabschnitt ein Sägeblock montiert oder montierbar ist und an deren distalem Endabschnitt eine Klemmvorrichtung bzw. Fußfesselvorrichtung angeordnet oder anbringbar ist. Der ausziehbare Schaft/Teleskopvorrichtung hat erfindungsgemäß ein Gleitschaft-/stabelement (oder einfach Gleitstab), welches in einem Handgriff axialgleitfähig aufgenommen und vorzugsweise mittels einer Feststelleinrichtung (Feststellschraube) relativ zum Handgriff in einer gewählten/wählbaren Ausziehposition fixierbar ist. Am distalen Endabschnitt der Teleskopvorrichtung, insbesondere des Gleitstabelements ist hierfür eine Aufnahme/Halterung zur Montage/Anbringung der Klemmvorrichtung /Fußfesselvorrichtung vorgesehen.

Dir Klemm-//Fußfesselvorrichtung hat einen Fußfesselabschnitt vorzugsweise einen Y- oder V-förmigen Tibia-Anlageblock oder Montageblock (wie dieser bevorzugt bereits zum ersten Kerngedanken der Erfindung beschrieben wurde), an welchem (die) Klammerelemente vorzugsweise gemäß dem ersten Kerngedanken der Erfindung gelagert sind, wobei die Fußfesselvorrichtung des Weiteren einen Kopplungsabschnitt vorzugsweise ein Einsteckstab hat, welcher mit der Gleitstab-seitigen Aufnahme in Eingriff bringbar ist.

Erfindungsgemäß ist der Fußfesselabschnitt, insbesondere die Klammerelemente und/oder der Tibia-Anlageblock bezüglich des Kopplungsabschnitts, insbesondere des Einsteckstabs asymmetrisch angeordnet, d.h. in Längsrichtung der Teleskopvorrichtung versetzt angeordnet, derart, dass sich der Fußfesselabschnitt, insbesondere die Klammerelemente bei einem Einsetzen/in Eingriff bringen des Kopplungsabschnitts in/mit der Schaft-seitigen Aufnahme je nach Drehausrichtung des Kopplungsabschnitts entweder oberhalb (proximal) oder unterhalb (distal) des Kopplungsabschnitts (in Schaftrichtung gesehen) anordnen. Auf diese Weise kann durch eine entsprechende Drehausrichtung des Kopplungsabschnitts bei dessen Montage die Distanz zwischen dem proximalen Sägeblock und dem Fußfesselabschnitt, insbesondere den Klammerelementen wahlweise (in Abhängigkeit der Asymmetrie/Längsversatzes) größer oder kleiner gemacht werden.

In einer besonders bevorzugten Ausführungsform wird der Höhenversatz der Fußklammeraufnahme- bzw. Einsetzvorrichtung von dem Mittelpunkt der Fußklammeraufnahmevorrichtung zu dem jeweiligen Randpunkt der Klammerelemente gemessen. Dieser beträgt in der ersten Stellung zum distalen Ende vorzugweise zwischen 10 mm und 20 mm und insbesondere 15 mm. Durch die Wendbarkeit der Fußklammeraufnahmevorrichtung um 180° Grad wird eine zusätzliche Längenverstellbarkeit in beide Richtungen, vorzugsweise um 15mm, erreicht. Insgesamt ist somit durch die Kombination der unterschiedlichen Längen der Gleitstäbe mit der wendbaren Fußklammeraufnahme- bzw. Einsetzvorrichtung eine Ausrichtungsvorrichtung bereitgestellt, die sehr flexibel in der Einstellung der Länge ist und die somit für die weltweiten Größen der Tibia eingesetzt werden kann.

Alle weiteren vorteilhaften Ausgestaltungen sind Gegenstand der Unteransprüche.

### Kurzbeschreibung der Figuren

Die vorliegende Erfindung wird nachfolgend anhand bevorzugter Ausführungsformen unter Bezugnahme auf die begleitenden Figuren näher erläutert.
- Fig. 1: zeigt eine perspektivische Ansicht einer erfindungsgemäßen Ausrichtungsvorrichtung gemäß einer ersten bevorzugten Ausführungsform,
- Fig. 2: zeigt eine perspektivische Explosionsdarstellung einer zweiten bevorzugten Ausführungsform aus Fig. 1,
- Fig. 3: zeigt eine Seitenansicht der Ausrichtungsvorrichtung aus Fign 1 und 2,
- Fig. 4: zeigt eine perspektivische Ansicht einer erfindungsgemäßen Ausrichtungsvorrichtung gemäß einer weiteren bevorzugten Ausführungsform,
- Fig. 5: zeigt eine perspektivische Ansicht einer Werkzeugführungsvorrichtung,
- Fig. 6: zeigt eine perspektivische Seitenansicht der Werkzeugführungsvorrichtung aus Fig. 5,
- Fig. 7: zeigt eine perspektivische Frontansicht der Werkzeugführungsvorrichtung aus Fig. 5,
- Fig. 8: zeigt eine perspektivische Ansicht einer zweiten Ausführungsform der Werkzeugführungsvorrichtung,
- Fig. 9: zeigt eine perspektivische Seitenansicht der zweiten Ausführungsform der Werkzeugführungsvorrichtung aus Fig. 8,
- Fig. 10: zeigt eine perspektivische Frontansicht der zweiten Ausführungsform der Werkzeugführungsvorrichtung aus Fig. 8,
- Fig. 11: zeigt eine perspektivische Ansicht der erfindungsgemäßen Klemmvorrichtung bei geschlossenen Klammerelementen,
- Fig. 12: zeigt eine Aufsicht der erfindungsgemäßen Klemmvorrichtung bei geschlossenen Klammerelementen,
- Fig. 13a: zeigt eine perspektivische Ansicht der erfindungsgemäßen Klemmvorrichtung bei geöffneten Klammerelementen,
- Fig. 13b: zeigt eine weitere perspektivische Ansicht der erfindungsgemäßen Klemmvorrichtung bei geöffneten Klammerelementen,
- Fig. 14: zeigt einen Ratschenmechanismus der erfindungsgemäßen Klemmvorrichtung der Fig. 13,
- Fig. 15: zeigt die erfindungsgemäße Klemmvorrichtung der Fig. 13 mit eingesetzter Tibia bei geöffneten Klammerelementen,
- Fig. 16: zeigt die erfindungsgemäße Klemmvorrichtung der Fig. 13 mit eingesetzter Tibia bei geschlossenen Klammerelementen,
- Fig. 17: bis Fig. 19 zeigen die Ratschenmechanismen der Klemmvorrichtung in unterschiedlichen Einstellungen,
- Fig. 20: bis Fig. 23 zeigen die erfindungsgemäße Entkopplungsvorrichtung in Kombination mit der Werkzeugführungsvorrichtung in unterschiedlichen Positionen,
- Fig. 24: zeigt die Abzugsrichtung der erfindungsgemäßen Ausrichtvorrichtung,
- Fig. 25: zeigt eine perspektivische Ansicht der erfindungsgemäßen Entkopplungsvorrichtung,
- Fig. 26: zeigt eine weitere perspektivische Ansicht der erfindungsgemäßen Ausrichtvorrichtung
- Fig. 27: zeigt eine erste perspektivische Ansicht des erfindungsgemäßen Sicherungselementes,
- Fig. 28: zeigt eine weitere perspektivische Ansicht des erfindungsgemäßen Sicherungselementes,
- Fig. 29: bis Fig. 35 zeigen unterschiedliche Detailansichten des erfindungsgemäßen Sicherungselementes,
- Fig. 36: zeigt eine perspektivische Ansicht der erfindungsgemäßen Antastvorrichtung,
- Fig. 37: zeigt eine weitere perspektivische Ansicht der erfindungsgemäßen Antastvorrichtung,
- Fig. 38: und Fig. 39 zeigen weitere perspektivische Ansicht der erfindungsgemäßen Antastvorrichtung,
- Fig. 40: bis Fig. 41 zeigen Detailansichten der erfindungsgemäßen Antastvorrichtung,
- Fig. 42: bis Fig. 44 zeigen weitere perspektivische Ansicht der erfindungsgemäßen Antastvorrichtung,
- Fig. 45: zeigt eine erste perspektivische Ansicht der erfindungsgemäßen Einschlagvorrichtung,
- Fig. 46: zeigt eine weitere perspektivische Ansicht der erfindungsgemäßen Einschlagvorrichtung,
- Fig. 47: bis Fig. 51 zeigen Detailansichten der erfindungsgemäßen Einschlagvorrichtung,
- Fig. 52 bis Fig. 55: zeigen perspektivische Ansichten der erfindungsgemäßen Ausrichtungsvorrichtung,
- Fig. 56: zeigt die Gleitstäbe in unterschiedlichen Längen,
- Fig. 57 und Fig.: 58 zeigen die erfindungsgemäße Klemmvorrichtung und die
- Fig. 59 bis Fig. 62: zeigen weitere perspektivische Ansichten der erfindungsgemäßen Ausrichtungsvorrichtung.

Die Figuren sind lediglich schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

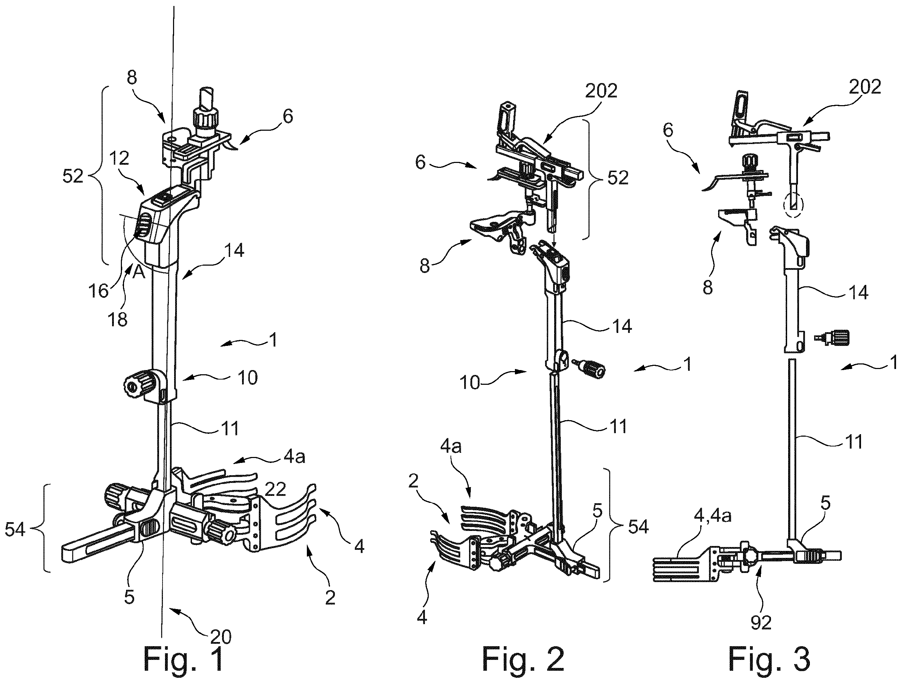

Die Fig. 1 zeigt eine Ausrichtungsvorrichtung bevorzugt in Form einer extramedullären Tibia Ausrichtungsvorrichtung 1 gemäß der vorliegenden Erfindung in einer "anterioren Fixations-Version", die einen proximalen Bereich 52 und einen distalen Bereich 54 aufweist. Der proximale Bereich 52 ist als der zum Körper des Patienten hingewandte Bereich definiert und der distale Bereich 54 ist als der vom Körper des Patienten abgewandte Bereich definiert. Der distale Bereich 54 ist also der Bereich, an welchem sich bspw. der Fuß/Fußknöchel des Patienten befindet.

Die Ausrichtungsvorrichtung 1 umfasst/hat demzufolge u.a.:
- eine Teleskopvorrichtung 10, die den Mittelteil der Ausrichtungsvorrichtung 1 bildet und einen proximalen Handgriff 14, ein im Handgriff 14 axialverschiebbar gelagertes (teleskopierbares/ausziehbares) distales Gleitschaftelement 11 sowie eine distale Aufnahme/Anschlussstelle 5 für eine Fußfessel/Klemmvorrichtung 2 aufweist,
- eine distale Fußfessel/Klemmvorrichtung 2, welche in die distale Aufnahme 5 einsetzbar (eingesetzt) bzw. mit dieser in Eingriff bringbar ist,
- einen proximalen (Montage-) Sägeblockadapter/Entkopplungsvorrichtung 12, der lösbar mit dem Handgriff 14 vorzugsweise an dessen proximaler Stirnseite gekoppelt/koppelbar ist,
- einen Sägeblock/Werkzeugführungsvorrichtung 8, der mit dem Sägeblockadapter 12 lösbar gekoppelt ist sowie bevorzugt
- eine Antastvorrichtung (verstellbarer Höhentaster/Abtastvorrichtung) 6, die lösbar mit dem Sägeblock 8 an dessen distaler Seite gekoppelt ist.

Im distalen Bereich der Ausrichtungsvorrichtung 1 ist die Klemmvorrichtung 2 angeordnet, welche Klammerelemente 4 und 4a umfasst/aufweist, die bogenförmig ausgeformt sind und die somit zwischen sich einen ovalförmigen Bereich 22 ausbilden, der dafür vorgesehen ist, die Tibia zu klemmen bzw. zu haltern. Die Teleskopvorrichtung 10 erstreckt sich entlang einer Längsachse 20. Im proximalen Bereich der Teleskopvorrichtung 10 ist der Handgriff 14 angeordnet, an dessen distalem Ende/Endabschnitt der Sägeblockadapter/Entkopplungsvorrichtung 12 daran (direkt oder indirekt) angeschlossen/angeordnet ist und somit wahlweise eine Einheit mit dem Handgriff 14 bildet. An dem Sägeblockadapter 12 ist ein Betätigungs-/Druckelement 16 vorgesehen, welches bevorzugt in einem Winkel A zu dem Handgriff 14 angeordnet/ausgerichtet ist. Dieses weist somit in Richtung hin zum proximalen Bereich 52 (nach oben), so dass ein behandelnder Arzt mit den Fingern einer Hand den Handgriff 14 umschließen und gleichzeitig mit seinem Daumen das Druckelement 16 bedienen kann. Im proximalen Bereich 52 ist der Sägeblock (auch als Werkzeugführungsvorrichtung zu bezeichnen) 8 angeordnet, an dem die Antastvorrichtung 6 lösbar befestigt ist. Das Druckelement 16 wirkt auf die Entkopplungsvorrichtung (Sägeblockadapter) 12, die dazu vorgesehen ist, die Teleskopvorrichtung 10 von der Werkzeugführungsvorrichtung (Sägeblock) 8 zu trennen. Sobald die Werkzeugführungsvorrichtung 8 mit der Tibia fest verbunden ist, kann diese somit durch Auslösung der Entkopplungsvorrichtung 12 über das Druckelement 16 von der Teleskopvorrichtung 10 entkoppelt werden.

Die Fig. 2 zeigt die Ausrichtungsvorrichtung 1 in einer "proximalen Fixations-Version" in Explosionsdarstellung.

Die Ausrichtungsvorrichtung 1 weist in Übereinstimmung mit der Fig. 1 in ihrem Mittelteil die Teleskopvorrichtung 10 auf, die in ihrem proximalen Bereich 52 über den Sägeblockadapter 12 wiederum mit der Werkzeugführungsvorrichtung/Sägeblock 8 verbunden/verbindbar ist, an welcher die Antastvorrichtung 6 befestigt/befestigbar ist. Des Weiteren ist zusätzlich eine Einschlagvorrichtung 202 an der Teleskopvorrichtung 10 (adaptiv/wahlweise) befestigt/befestigbar. Im distalen Bereich 54 ist die Klemmvorrichtung 2 an der Teleskopvorrichtung 10 befestigt/befestigbar, welche die Klammerelemente 4, 4a aufweist. Insofern entspricht die Ausrichtungsvorrichtung 1 gemäß der Fig. 2 konzeptionell jener gemäß der Fig. 1 mit dem Unterschied, dass zusätzlich die Einschlagvorrichtung 202 an der Teleskopvorrichtung 10 an deren proximalem Endabschnitt montiert ist.

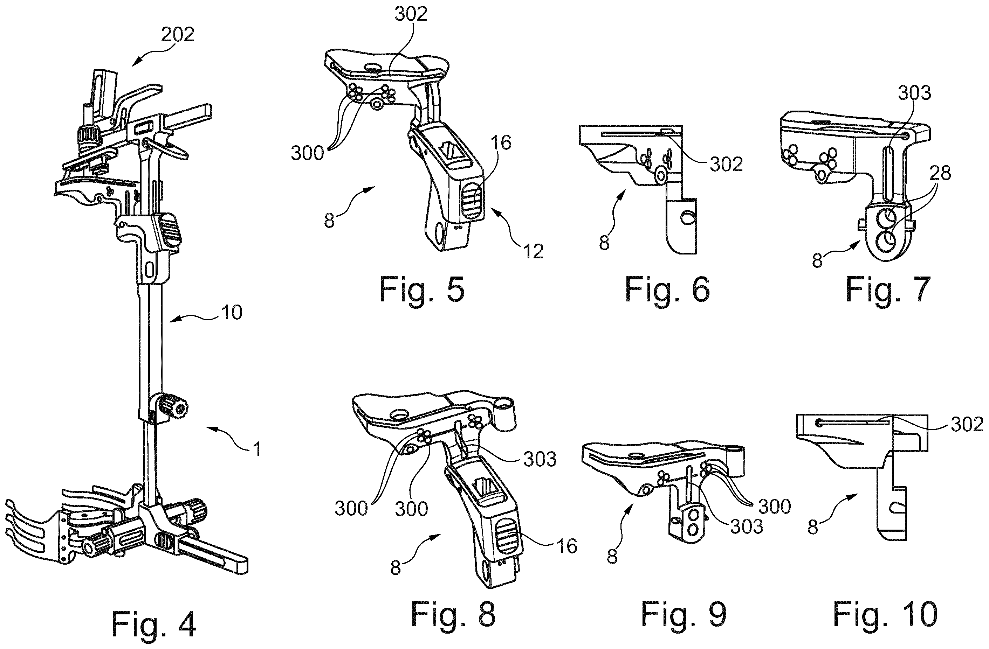

Die Fig. 3 zeigt die Ausrichtungsvorrichtung 1 gemäß der Fig. 2 in einer Seitenansicht sowie ebenfalls in Explosionsdarstellung und die Fig. 4 zeigt die Ausrichtungsvorrichtung 1 im vollständig montierten Zustand. Insbesondere in der Darstellung gemäß Fig. 3 sind die einzelnen Schnittstellen an der erfindungsgemäßen Ausrichtungsvorrichtung 1 zumindest teilweise erkennbar. Demzufolge kann der Sägeblock 8 mit dem Sägeblockadapter/Entkopplungsvorrichtung 12, die Abtastvorrichtung 6 mit dem Sägeblock 8 sowie die Einschlagvorrichtung 202 mit der Teleskopvorrichtung 10 und insbesondere dem distalen Gleitschaftelement 11 an dessen proximalem Ende/Endabschnitt wahlweise in Eingriff gebracht werden, der zu diesem Zweck den Handgriff 14 axial in Richtung proximal (vollständig) durchdringt. In der Fig. 4 ist gezeigt, wie die Einschlagvorrichtung 202 in den proximalen Endabschnitt des Gleitschaftelements 11 eingesetzt ist.

Die Fig. 5 bis Fig. 10 zeigen verschiedene Ansichten der Werkzeugführungsvorrichtung/Sägeblock 8 sowie dem Sägeblockadapter/Entkopplungsvorrichtung 12. In der Fig. 5 ist die Werkzeugführungsvorrichtung 8 zusammen mit der Entkopplungsvorrichtung 12 dargestellt, die das Druckelement/Drucktaste 16 umfasst. Die Werkzeugführungsvorrichtung/Sägeblock 8 weist vorzugsweise seitliche Einschlaglöcher 300 auf, die dafür genutzt werden können, den Sägeblock 8 an der Tibia per Schrauben oder Nägel zu fixieren. Die Werkzeugführungsvorrichtung/Sägeblock 8 ist dazu vorgesehen, ein Werkzeug/Säge zur Resektion der Tibia führend aufzunehmen, wofür ein Werkzeugführungs-/Sägeschlitz 302 im Sägeblock 8 ausgebildet ist. Die Fig. 6 zeigt die Werkzeugführungsvorrichtung 8 mit dem Werkzeugführungsschlitz 302, der bei fixiertem Zustand an der Tibia in horizontaler Richtung ausgerichtet ist, in einem vom Sägeblockadapter 12 getrennten Zustand. Die Fig. 7 zeigt die Werkzeugführungsvorrichtung 8 mit einem weiblichen Kopplungsabschnitt vorliegend mit zwei höhenbeabstandeten Aufnahmebohrungen 28 und einem zusätzlichen Einschlagschlitz 303, der dazu vorgesehen ist, Befestigungselemente bzw. Nägel zum Einschlagen in die Tibia aufzunehmen.

Die Werkzeugführungsvorrichtung, also der Sägeblock 8, hat folgende Funktionen bzw. folgende Funktionselemente:
- Führen eines Sägeblattes für den Knochenabschnitt innerhalb des Führungsschlitzes 302,
- Bereitstellung von Einschlag-/Befestigungslöchern 300 und/oder des Einschlagschlitzes 303 für die Befestigung der Werkzeugführungsvorrichtung 8, also des Sägeblocks, am Knochen und ggf. zur Korrektur der Schnitthöhe um bis zu +/- 4 mm,
- Bereitstellung einer Adapterschnittstelle (weiblicher Kopplungsabschnitt mit Aufnahmebohrungen 28) zur Montage an der Ausrichtungsvorrichtung/Teleskopvorrichtung 10 und
- Bereitstellung einer Adapterschnittstelle für die Montage der Abtastvorrichtung/ Schnitthöhentasters 6.

Darüber hinaus zeigen beispielsweise die Fig. 5 und 8 unterschiedliche Versionen für einen Sägeblock 8 gemäß der Erfindung, nämlich eine Version (Fig. 5) für eine "anteriore Fixations-Variante" und eine Version (Fig. 8) für eine "proximale Fixations-Variante", bei welcher die Adapterschnittstelle (Einsteckbohrung) für den Schnitthöhentaster 6 bezgl. der Version gemäß Fig. 5 versetzt ist bzw. zwei Adapterschnittstellen für beide Varianten vorgesehen sind.

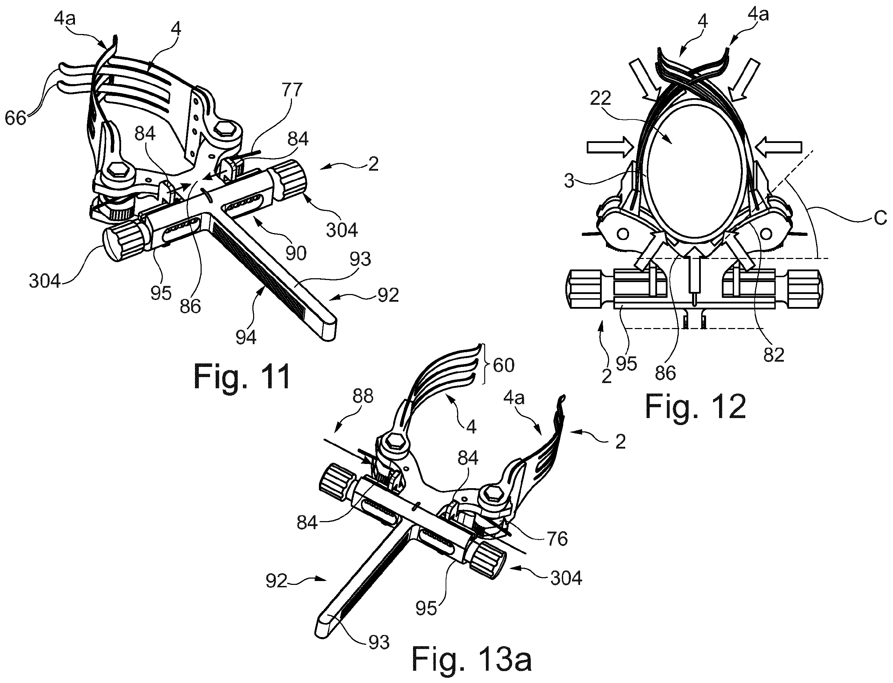

Die Fig. 11 bis Fig. 13b zeigen perspektivische Ansichten der Klemmvorrichtung 2. Die Fig. 13a zeigt die Klemmvorrichtung 2, welche die zwei Klammerelemente/Klemmarme 4 und 4a aufweist. Die Klammerelemente 4 und 4a sind jeweils bogenförmig geformt und laufen an ihren jeweiligen freien Enden sanft aus (nach außen gebogen), sodass die Klammerelemente 4, 4a verletzungsfrei von der Tibia bzw. dem Fußknöchel (federelastisch) abgezogen werden können, ohne dass die freien Klemmarm-Enden an der Patientenhaut kratzen können.

Im Konkreten hat die Klemmvorrichtung 2 einen Montageblock in Form eines T-Stücks 92 mit einem vorzugsweise im Querschnitt vierkantförmigen (rechteckigen) Einsteck-Stab 93 und einem hohlen Querträger 95, in welchem ein Spindelmechanismus/Spindel 90 gelagert ist, die mittels stirnseitig am Querträger 95 angeordneter Drehknöpfe 304 um deren Längsachse gedreht werden kann. Am Querträger 95 ist ein Schlitten (Tibia-Anlageblock) 86 gelagert, in welchen die Spindel 90 eingreift, sodass der Schlitten 86 bei einem manuellen Drehen der Spindel 90 mittel der Drehköpfe 304 längs des Querträgers 95 hin- und hergefahren werden kann. Der Schlitten 86 weist ferner einen zentralen/mittigen Anlagebereich 78 sowie zwei V-förmig zueinander ausgerichtete Anlagearme 82 auf, an deren freien Endabschnitten jeweils ein Ratschenmechanismus 76 angeordnet/eingebaut ist, an welchen jeweils ein Klammerelement 4, 4a eingreift, derart, dass die Klammerelemente 4, 4a schwenkbar an den Anlagearmen 82 gelagert und manuell aufeinander zu (in Schließrichtung) verschwenkbar sind, wobei der jeweils zugehörige Ratschenmechanismus 76 ein Rückschwenken (in Öffnungsrichtung) zunächst verhindert.

Des Weiteren hat jeder Ratschenmechanismus 76 eine Vorspannfeder 77 (diese sind insbesondere in der Fig. 11 als Schenkelfedern dargestellt), welche die zugehörigen Klammerelemente 4, 4a in Öffnungsrichtung vorspannen. Schließlich hat jeder Ratschenmechanismus 76 einen Ratschenhebel 84, über welchen der zugehörige Ratschenmechanismus 76 entriegelbar/freigebbar ist.

Die Klammerelemente 4 und 4a sind aus einem vorgebogenen (Blatt-) Federstahl gefertigt und bestehen aus mehreren, nebeneinander angeordneten Zinken oder Fingern 62 die nach Art einer Gabel 60 voneinander beabstandet sind, sodass die Zinken/Finger 62 der einander zugewandten Klammerelemente 4, 4a bei deren Verschwenken in Schließrichtung in sich überlappender Weise ineinandergreifen können und so eine sichere Fixierung an der Tibia 3 gewährleistet ist.

Fig. 11 zeigt die Klemmvorrichtung 2 mit den geschlossenen Klammerelementen 4 und 4a, die so ineinandergreifen, dass daran eine Tibia 3 ausreichend klemmbar bzw. halterbar ist. Zum Vorspannen der Klammerelemente 4 und 4a werden die Ratschenhebel 84 zunächst in einer voneinander weg gerichteten Bewegung gedrückt, wodurch Rastklinken (diese sind in Fig. 11 schematisch als einstückig mit den Ratschenhebeln 84 ausgebildete Klinken/Zähne dargestellt) in eine Außenverzahnung an den jeweiligen Klammerelementen 4, 4a einrasten. Wenn die Klammerelemente 4, 4a gelöst werden sollen, werden die Ratschenhebel 84 leicht nach innen (aufeinander zu) gedrückt (siehe Fig. 11), wodurch die Rastklinken außer Eingriff mit den zugehörigen Klammerelementen 4, 4a kommen und damit die Ratschenwirkung aufgehoben wird. Über den Spindelmechanismus 90, an welchem die Drehköpfe 304 befestigt sind, können die Klammerelemente 4, 4a über den gemeinsamen Schlitten 86 in beide Richtungen längs des Querträgers 95 bewegt werden. Der Spindelmechanismus 90 ist, wie vorstehend bereits ausgeführt, in dem Querträger 95 integriert. Auf diese Weise können die Klemmelemente 4, 4a im bereits die Tibia umgreifenden Zustand optimal in Querrichtung der Tibia ausgerichtet werden, ohne dass der Patient oder der Arzt einem Verletzungsrisiko z.B. infolge hervorragender Teile der Spindel etc. ausgesetzt wird.

Das T-Stück 92 bildet in Zusammenwirkung mit dem Schlitten 86 und den daran angeordneten Anlagearmen 82 eine im Wesentlichen Y-förmigen Tibia-Anlageblock-Montageeinheit aus, die in eine entsprechende distale Fußfessel-Aufnahme 5 auf Seiten der Teleskopvorrichtung 10 eingesetzt werden kann.

Hierzu bildet das T-Stück 92 den Dorn/Einsteckstab 93 aus, der in seinem Querschnitt vorzugsweise rechteckförmig ist und an zumindest zwei voneinander abgewandten Längsseiten eine Rast-/Grippstruktur 94 hat, durch welche die gesamte Klemmvorrichtung 2 bzw. die Montageeinheit an der Teleskopvorrichtung 10 bzw. deren Aufnahme 5 verschiebbar befestigt/befestigbar ist.

Die Fig. 12 zeigt die Klemmvorrichtung 2 in Draufsicht und im geschlossenen Zustand, wobei zwischen den Klammerelementen 4, 4a der ovalförmige Bereich 22 zur Aufnahme der Tibia (schematisch in Fig. 12 dargestellt) ausgebildet ist. Die Tibia 3 ist dabei von dem V-förmigen Anlageblock/-element 86 einschließlich der seitlichen Anlagearme 82 und den Klammerelementen 4, 4a aufgenommen. Zwischen dem V-förmigen Anlageblock 86 und den seitlichen Anlagearmen 82 ist im mittleren Anlageberich 78 ein Winkel C ausgebildet, der vorzugsweise 45° an jeder Seite beträgt (d.h. die beiden Anlagearme 82 schließen einen gemeinsamen Winkel von ca. 90° ein). So ist eine besonders ergonomische Anlage der Tibia in dem V-förmigen Anlageblock 86 ausgebildet.

Des Weiteren zeigt die Fig. 12 die Kraftbeaufschlagungsrichtungen auf die Tibia 3, welche mit der erfindungsgemäßen Fußfessel 2 erreichbar sind.

Demzufolge umschließen die beiden Klammerelemente 4, 4a die Tibia vollständig, indem sich ihre Zinken/Finger an der Tibia-Rückseite ineinander überkreuzen/verhaken/überlappen und so die Tibia gegen den vorderseitigen Anlageblock/Schlitten 86 drücken. Da die Klammerelemente 4, 4a vorgebogen und auch federelastisch sind, können diese gleichzeitig auch eine Einspannkraft von den Seiten auf die Tibia aufbringen, wodurch diese quasi rundum eingespannt wird. Dies wird durch die Kraftpfeile in der Fig. 12 anschaulich dargestellt.

Die Fig. 13a zeigt die Klemmvorrichtung 2 mit den Klammerelementen 4, 4a in der geöffneten Stellung, so das eine Tibia einführbar ist. Durch eine zueinander gerichtete Bewegung der Ratschenhebel 84 werden die Klammerelemente 4, 4a bzw. der Ratschenmechanismus freigegen, sodass die Klemmelemente 4, 4a infolge der inneren Federvorspannung in eine geöffnete Stellung gebracht werden können, wohingegen durch eine auseinander gerichtete Bewegung der beiden Ratschenhebel 84, der Ratschenmechanismus wieder in Funktion gesetzt wird, sodass die Klammerelemente 4, 4a individuell in eine geschlossene Stellung gebracht und dort verrastet werden können.

Wie vorstehend bereits ausgeführt wurde, ist/sind der/die Drehknöpfe 304 dazu vorgesehen, eine seitliche Verstellbarkeit des Schlittens/Tibea-Anlageblocks 86 und der daran gelagerten Klammerelemente 4, 4a zu bewirken, wobei die mittels der Drehknöpfe 304 betätigbare Spindel 90 zur Verschiebung des Schlittens 86 in das T-Stück 92 bzw. den Querbalken 95 integriert ist.

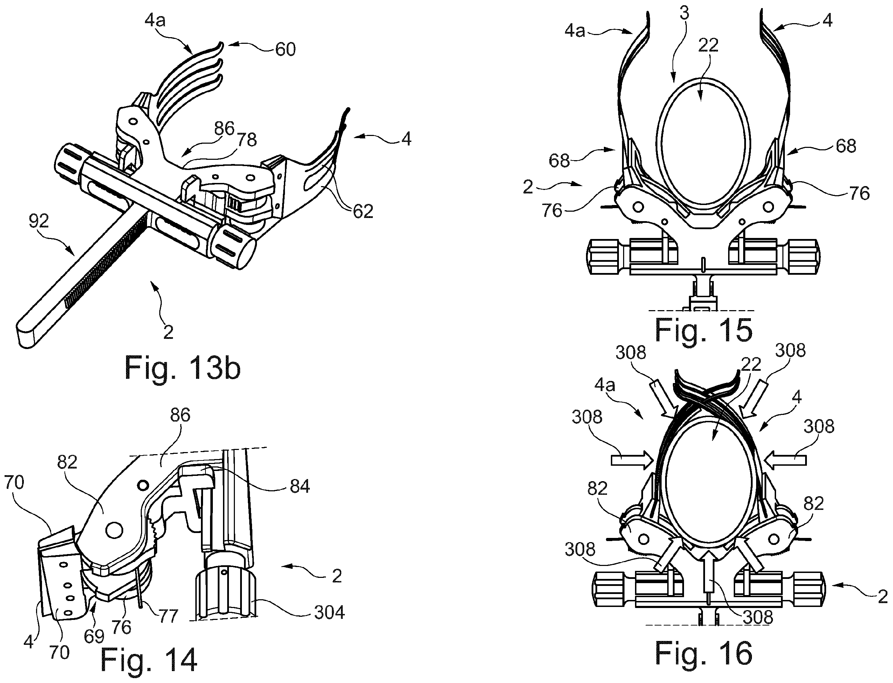

Die Fig. 13b zeigt die Klemmvorrichtung 2 mit den jeweiligen Klammerelementen 4 und 4a, die wiederum so ausgebildet sind, dass sie durch die jeweiligen Gabeln 60 ineinandergreifen, sodass die Tibia nahezu rundumfänglich geklemmt wird und ggf. in Umfangsrichtung eine nahezu gleichbleibende Einspann-Kraft bewirkt ist.

Die Fig. 14 zeigt die Klemmvorrichtung 2 und einen Scharnierbereich 86 eines Klammerelementes 4 und des Anlageblocks/Schlittens 86. Das Klammerelement 4 ist mit dem Ratschenmechanismus 76 gekoppelt, der über den Ratschenhebel 84 freigegeben werden kann, wobei in der Fig. 14 nur ein Ratschenhebel 84 dargestellt ist. Zu erkennen ist die Rastklinke (ohne Bezugszeichen) des Ratschenhebels 84, welche in eine Außenverzahnung im Scharnierbereich des Klemmelements 4 federvorgespannt eingreift, sowie der einstückig damit verbundene Ratschenhebel 84 zum Ausrücken der Rastklinke aus der Außenverzahnung. Des Weiteren ist der Drehknopf 304 dargestellt, durch welchen der Tibia-Anlageblock 86 in Seitenrichtung verstellbar/verschiebbar ist.

Die Fig. 15 zeigt eine Draufsicht auf die Klemmvorrichtung 2 im offenen Zustand. Die Klammerelemente 4, 4a sind dabei über deren jeweiligen (inneren/scharnier-nahen) Enden/Scharnierbereiche 68 an den Ratschenmechanismus 76 angekoppelt. Die Klammerelemente 4, 4a sind vorzugsweise aus dünnem Blech bzw. anderweitig federndem Material hergestellt. An ihren freien Enden/Spitzen 66 weisen die Klammerelemente 4, 4a jeweils (radial) nach außen (sind nach außen umgebogen), um ein verletzungsfreies Entfernen bzw. Abziehen der jeweiligen Klammerelemente 4, 4a von der Tibia zu ermöglichen. Die scharnier-nahen Enden 68 der Klammerelemente 4, 4a sind vorzugsweise mit einem Kunststoffteil (Verstärkungselement) 70 verstärkt (bzw. mit Kunststoff ummantelt), um insbesondere eine sanfte Momenteneinleitung in die weitere Struktur so zu bewirken, dass die Klammerelemente 4, 4a ausreichend dauerfest sind.

Fig. 16 zeigt ebenfalls die Klammerelemente 4, 4a im geschlossenen Zustand. Zwischen den Klammerelementen 4, 4a ist der ovalförmige Bereich 22 zur Aufnahme der Tibia bzw. des Fußgelenkes ausgebildet. Durch die Klammerelemente 4, 4a, das V-förmige Anlageelement /Anlageblock/Schlitten 86 und die jeweiligen seitlichen Anlagebereiche/Anlagearme 82 des Anlageblocks 86 wird die Kraftwirkung 308 ausgebildet, die in Umfangsrichtung um die Tibia wirkt. Die Klammerelemente 4, 4a sind im Konkreten so ausgelegt, dass die Kraftwirkung 308 an mehreren unterschiedlichen Stellen in Umfangsrichtung der Tibia auf die Tibia wirkt, sodass diese ringsum fest gehaltert ist.

Aus der Fig. 13b in Verbindung mit beispielsweise der Fig. 3 ist die räumliche Anordnung der Klemmarme 4, 4a bzgl. des Anlageblocks 86 bzw. des T-Stücks 92 zu erkennen.

Demzufolge sind die Klemm-/Klammerarme 4, 4a (nach Art einer Schaufel) bzgl. des T-Stücks 92 höhenversetzt angeordnet, d.h. nicht auf einer Ebene mit dem T-Stück 92 liegend. Dies hat unmittelbar zur Folge, dass im Fall einer Drehposition gemäß der Fig. 3 die Klammerarme 4, 4a unterhalb des T-Stücks liegen, wohingegen für den Fall, dass das T-Stück 92 um 180° gedreht wird, die Klammerarme 4, 4a oberhalb des T-Stücks 92 zu liegen kommen.

An dieser Stelle sei daran erinnert, dass der Einsetzdorn 93 gemäß der Fig. 13a ein Vierkantprofil ist und die Aufnahme 5 am distalen Ende der Teleskopvorrichtung 10 demzufolge einen rechteckigen Aufnahmeschacht bildet, in welchen die Einsetzdorn 93 eingeführt und darin verrastet wird. Damit ergibt sich, dass in Abhängigkeit der Drehausrichtung des T-Stücks 92 der Abstand zwischen den Klemmarmen 4, 4a (oberhalb oder unterhalb des T-Stücks 92) und dem proximal angeordneten Sägebock 8 vergrößert oder verkleinert ist, wodurch das Längenspektrum der Teleskopvorrichtung 10 insgesamt zusätzlich verkleinert oder vergrößert werden kann.

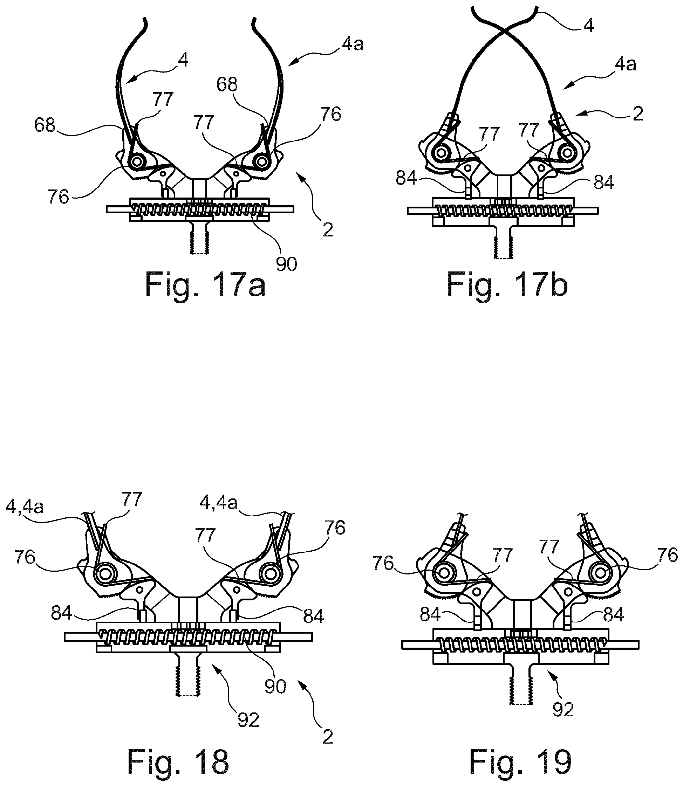

Die Fig. 17a, b bis Fig. 19 zeigen die Klemmvorrichtung 2 jeweils in einem Querschnitt. Die Fig. 17a, b zeigt die Klammerelemente 4, 4a, die jeweils über die scharnier-nahen Enden 68 der Klammerelemente 4, 4a an den Ratschenmechanismus 76 angekoppelt sind. Die Fig. 17a zeigt dabei die geöffnete Klammerstellung und die Fig. 17b zeigt die Klammerelemente 4, 4a in geschlossener Klammerstellung. Zu erkennen sind die Federn 77, welche die Klammerelemente 4, 4a in Öffnungsrichtung vorspannen sowie die beiden Ratschenmechanismen 76 mit daran sich anschließenden Ratschenhebeln 84 für ein manuelles, individuelles außer Funktion setzen der Ratschenwirkungen.

Die Fig. 18 und 19 zeigen in Vergrößerung die Klemmvorrichtung 2 mit den jeweiligen Klammerelementen 4, 4a, die an den jeweiligen Ratschenmechanismus 76 angekoppelt sind. Zusätzlich ist jeweils die entspannte Rückstellfeder 77 des jeweiligen Ratschenmechanismus 76 zu erkennen.

Die Klemmvorrichtung 2, die auch als Fußklammer bezeichnet wird, ist so ausgelegt, dass die Teleskopvorrichtung 10 sowohl an der proximalen Adapterschnittstelle 12 des Tibia-Sägeblockes 8 und in einer besonders einfachen Handhabungsaktion von der Tibia des Patienten gewebeschonend (d.h. atraumatisch) abgezogen werden kann, wofür die Federelastizität der Klemmarme 4, 4a ausgenutzt wird. D.h., will ein Chirurg die Teleskopvorrichtung 10 von der Tibia entfernen unter Belassung des Sägeblocks 8 an der Tibia, muss dieser nur die Drucktaste 16 betätigen, um den Sägeblock 8 von der Teleskopvorrichtung 10 abzukoppeln und gleichzeitig die Klemmvorrichtung 2 einfach abziehen (ohne Betätigen der Ratschenhebel 84).

Des Weiteren bewirkt die Federelastizität der Klemmarme 4, 4a eine über den Tibia-Umfang nahezu gleichbleibende Klemmkraft, sodass Hemmatome infolge punktueller Krafteinwirkung vermeidbar sind. Die Klemmvorrichtung 2, also die Fußklammer, zeichnet sich für diese Aufgabe dadurch besonders aus, dass
- die Klammerelemente 4, 4a, also die Halteelemente der Fußfessel 2, ausreichend elastisch sind, sodass man sie ohne einen Lösemechanismus zu bedienen, einfach von der distalen Tibia abziehen kann und dass diese gleichzeitig eine ausreichend hohe Rotations- und Translationsstabilität gegenüber ungewolltem Verstellen der ETA aufweist und dass
- der Ratschenmechanismus 76 auch manuell mittels der Ratschenhebel 84 geöffnet werden kann.

Der Ratschenmechanismus 76 wird über den jeweiligen Ratschenhebel 84 in/außer Funktion gesetzt. Des Weiteren ist es möglich, die Klammerelemente 4, 4a nicht von der Tibia einfach abzuziehen, sondern den Ratschenhebel 84 zu lösen. Durch die Rückstellfeder 77 werden dann die beiden Klammerelemente 4, 4a nach Betätigen der Ratschenhebel 84 automatisch geöffnet und geben somit die Tibia frei.

Wie vorstehend bereits ausgeführt wurde, ist die Adapter-Schnittstelle für den Tibia-Sägeblock 8, also für die Werkzeugführungsvorrichtung, derart gestaltet, dass der die Adapterschnittstelle bildende Sägeblockadapter 12 zum Lösen der Teleskopvorrichtung 10 von dem Sägeblock 8 mit einem einfachen Daumendruck auf die Drucktaste 16 betätigbar ist, wodurch ein Haltemechanismus 18 (wird nachfolgend detaillierter beschrieben) den Sägeblock 8 freigibt. Durch die vorstehend beschriebenen Varianten bezüglich des Sägeblocks 8 wie auch der Klemmvorrichtung 2 ist es nun möglich, nach Befestigen des Sägeblocks 8 an der Tibia und der Bedienung des Sägeblockadapters 12 (d.h. Betätigen des Haltemechanismus 18) die Teleskopvorrichtung 10 einschließlich Adapter 12 und Klemmvorrichtung 2 mit einer (einzigen) Hand und einem Daumendruck sowie unter Ausnutzung der Federelastizität der Klammerarme 4, 4a von der Tibia des Patienten abzuziehen, ohne dass eine weitere Bedienaktion notwendig wäre. Dies erleichtert in erheblichem Umfang die Handhabung der gesamten Ausrichtungsvorrichtung 1.

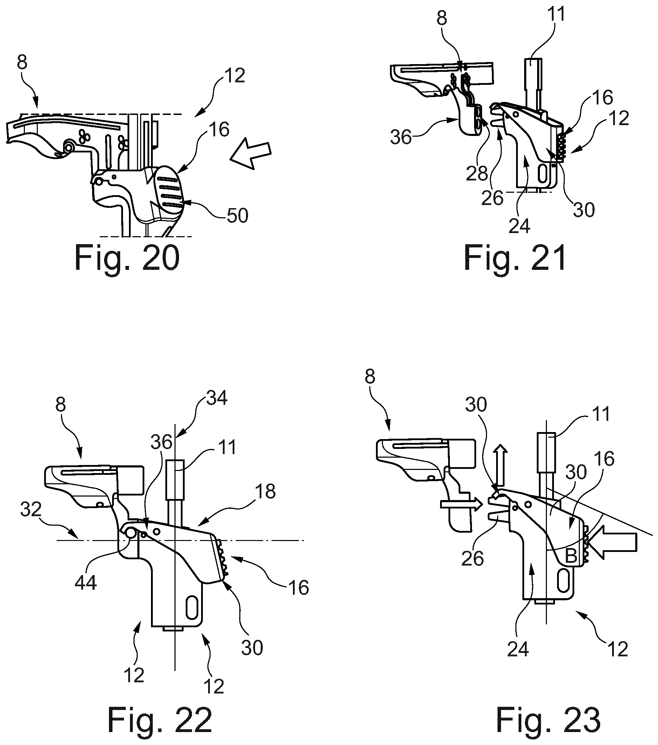

Die Fig. 20 bis Fig. 23 zeigen verschiedene perspektivische Ansichten der Werkzeugführungsvorrichtung/Sägeblock 8 in Kombination mit der Entkopplungsvorrichtung/Sägeblockadapter 12. In der Fig. 20 ist die Werkzeugführungsvorrichtung 8 an der Entkopplungsvorrichtung 12 (wahlweise) befestigt/eingekoppelt. Die Entkopplungsvorrichtung 12 weist dabei den Haltemechanismus 18 einschließlich das Druckelement 16 auf, welches vorzugsweise eine reliefartige Oberfläche 50 aufweist. Die Daumendruckkraft des jeweils behandelnden Arztes drückt dabei auf die reliefartige Oberfläche 50 des Druckelementes 16.

Die Fig. 21 zeigt das Zusammenwirken der Werkzeugführungsvorrichtung 8 mit der Entkopplungsvorrichtung 12. Die Werkzeugführungsvorrichtung 8 weist im Konkreten einen Adaptersockel 36 auf, welcher wiederum mehrere weibliche Aufnahmeelemente 28 aufweist. Die weiblichen Aufnahmeelemente 28 sind jeweils bevorzugt Ausnehmungen/Bohrungen in dem Adaptersockel 36, die dazu vorgesehen sind, die jeweiligen männlichen Aufnahmeelemente/Vorsprünge/Stifte 26 der Entkopplungsvorrichtung 12 formschlüssig aufzunehmen. Durch das Ineinanderfügen der jeweiligen männlichen Aufnahmeelemente 26 in die jeweiligen weiblichen Aufnahmeelemente 28 ist ein sicherer Halt der Werkzeugführungsvorrichtung 8 an der Entkopplungsvorrichtung 12 in den sämtlichen drei Raumrichtungen gewährleistet. An der Entkopplungsvorrichtung 12 ist zusätzlich der Haltemechanismus 18 in Form eines Sicherungsbügels bzw. Hakenelements 30 gelagert, welches dazu vorgesehen ist, den Adaptersockel 36 der Werkzeugführungsvorrichtung 8 zu umschließen bzw. in/an diesem an einer entsprechenden Hinterschneidung 44 zu verhaken.

Die Fig. 22 zeigt eine Seitenansicht auf die Werkzeugführungsvorrichtung 8 und den Sägeblockadapter 12 in gekoppeltem Zustand. Demzufolge besteht der Sicherungsbügel 30 vorzugsweise aus einem Blechbauteil, welches unter Ausbildung eines im Wesentlichen U-Hohlprofils abgekantet ist, wobei die beiden, jeweils frei endenden Stege/Seitenflanken des U-Profils zu Haken und der Verbindungssteg des U-Profils zum Druckelement 16 ausgeformt sind. Dieser so gestaltete Sicherungsbügel 30 ist an einem Grundkörper 24 des Sägeblockadapters 12 beidseitig von diesem anscharniert (d.h. der Grundkörper 24 ist in dem U-Hohlprofil aufgenommen). Das U-Hohlprofil bildet so eine Art Wippe mit dem Druckelement 16 auf der einen und den Haken auf der anderen Seite des Wippenscharniers. Im gekoppelten Zustand gemäß der Fig. 22 greifen die Haken des Sicherungsbügels 30 mit den Hinterschneidungen 44 in Form von bolzen- oder stiftartigen Vorsprüngen am Sägeblock 8, insbesondere an dessen Montage-/Adaptersockel 36 ein und halten diesen so an der Adapterschnittstelle des Sägeblockadapters 12 fest.

Darüber hinaus ist in der Fig. 22 ein seitlicher Betätigungsknopf am Grundkörper 24 des Sägeblockadapters 12 angedeutet, der mit einem Rastmechanismus zusammenwirkt (an der unteren Stirnseite des Sägeblockadapters 12 symbolisch dargestellt), mittels welchem der Sägeblockadapter 12 an der oberen Stirnseite (oberen Handgriffabschnitt) des Handgriffs 14 mit diesem gekoppelt werden kann, um so wahlweise eine Einheit mit dem Handgriff 14 zu bilden, wie dies beispielsweise in der Fig. 15 dargestellt ist.

Die Fig. 23 zeigt die Funktionsweise der Entkopplungsvorrichtung/Sägeblockadapter 12 (insbesondere des Haltemechanismus) und der Werkzeugführungsvorrichtung/Sägeblock 8, die am Grundkörper 24 der Entkopplungsvorrichtung 12 gekoppelt werden soll.

Es ist zu erkennen, dass durch Betätigung des Druckelements 16 per Daumendruck der Sicherungsbügel 30 verschwenkt und dabei dessen Haken angehoben werden können. **In** diesem Zustand kann der Sägeblock 8 auf die Adapterschnittstelle der Entkopplungsvorrichtung 12 aufgesetzt werden, wobei die männlichen Vorsprünge/Zapfen 26 in die weiblichen Ausnehmungen/Bohrungen 28 nach dem Stecker-Steckdosenprinzip eingeführt werden. Abschließend wird das Druckelement 16 freigegeben, worauf die Haken des Sicherungsbügels 30 (schwerkraftbedingt oder federvorgespannt) nach unten schwenken und dabei hinter die Vorsprünge 44 am Sägeblock 8 (siehe Fig. 22) greifen. Das Abtrennen der Entkopplungsvorrichtung 12 vom Sägeblock 8 erfolgt in entsprechend umgekehrter Weise.

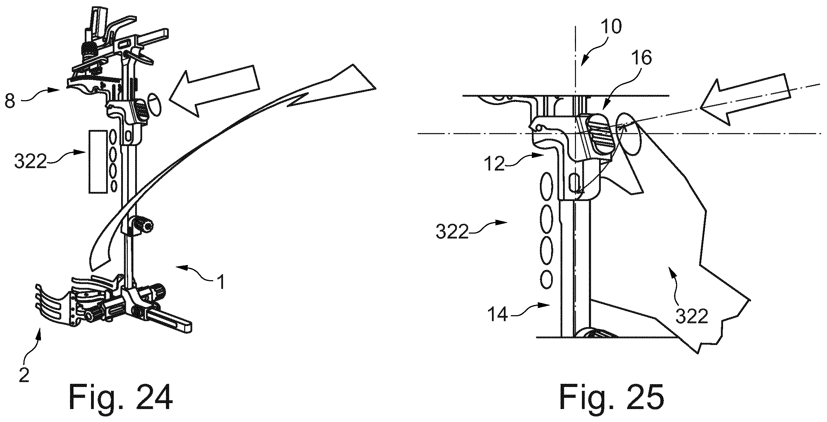

Die Fig. 24 zeigt die Abzieh-/Abnehmrichtung der Ausrichtungsvorrichtung 1 (der Teleskopvorrichtung 10 samt Fußklammer 2 und Adapter 12), wenn die Werkzeugführungsvorrichtung 8 an der Tibia befestigt ist/bleibt und somit von der Teleskopvorrichtung 10 getrennt werden soll. Der behandelnde Arzt drückt dabei mit der Kraft seines Daumens auf die reliefartige Oberfläche 50 des Druckelementes 16 (wodurch der Haltebügel 30 den Sägeblock 8 freigibt) und zieht die Teleskopvorrichtung 10 zusammen mit der Klemmvorrichtung 2 von der Tibia des Patienten einfach ab. Die Klemmvorrichtung 2 wird hierbei nicht gesondert geöffnet, sondern die Klammerelemente 4, 4a werden infolge ihrer Federelastizität beim Abziehen (selbsttätig) aufgespreizt. Die angedeutete Hand 322 des behandelnden Arztes umschließt dabei die Teleskopvorrichtung 10 bzw. den Handgriff 14.

Die Fig. 25 zeigt die Hand 322 des behandelnden Arztes, die die Daumendruckkraft auf das Druckelement 16 der Teleskopvorrichtung 10 bewirkt. Die Finger der Hand 322 des behandelnden Arztes umklammern dabei die Teleskopvorrichtung 10 an dem Handgriff 14.

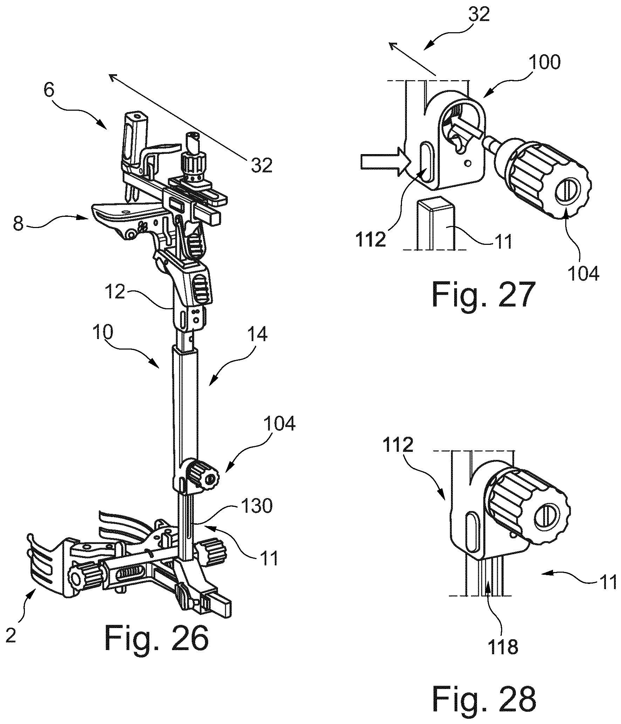

Die Fig. 26 und 27 zeigen die Ausrichtungsvorrichtung 1 gemäß der vorliegenden Erfindung mit der Teleskopvorrichtung 10 gemäß einer "proximalen Fixationsvariante", wobei im distalen Bereich der Teleskopvorrichtung 10 wiederum die Klemmvorrichtung 2 vorzugsweise gemäß vorstehender Beschreibung vorgesehen ist und im proximalen Bereich wiederum die Werkzeugführungsvorrichtung 8 vorzugsweise gemäß vorstehender Beschreibung über den Adapter 12 angeordnet ist, an welcher bevorzugt die Antastvorrichtung 6 befestigt/befestigbar ist. Die in Fig. 26 und 27 angedeutete Querrichtung 32 bedeutet dabei eine Annäherungsrichtung an die Tibia eines Patienten.

An der Teleskopvorrichtung 10 ist (bei sämtlichen Fixations-Varianten) ein Feststell- oder Sicherungselement 104 vorgesehen, welches bevorzugt in dieser Querrichtung 32 gesehen vor der Ausrichtungsvorrichtung 1 in die Teleskopvorrichtung 10 einsetzbar ist. Das Sicherungselement 104 ist u.a. dazu vorgesehen, in verschiedenen Stellungen die Teleskopvorrichtung 10 selbsthemmend in einer gewählten Längenposition zu sichern und/oder frei auseinanderziehen zu lassen. Das Sicherungselement 104 ist weiterhin dazu vorgesehen, in der Querrichtung 32 in ein Aufnahmeelement/Aufnahmeabschnitt 100 eingesetzt zu werden, welches(r) an dem Handgriff 14 ausgebildet ist. Des Weiteren ist ein Verriegelungselement 112 am Aufnahmeabschnitt 100 angeordnet, das dazu ausgebildet ist, in eine Freigabestellung manuell bewegt zu werden, in welcher das Sicherungselement 104 aus dem Aufnahmeabschnitt 100 entfernbar ist, wohingegen das Verriegelungselement 112 in einer (unbetätigten) Rastposition das Sicherungselement 104 im Aufnahmeabschnitt 100 hält.

Die Fig. 28 zeigt das Gleitstabelement 11 in einer vergrößerten Darstellung, wonach an einer dem Sicherungselement 104 zugewandten Seite eine längsnutförmige Ausnehmung (nachfolgend als Längsnut bezeichnet) 118 am Gleitstabelement 11 ausgebildet ist, die distal und proximal durch einen Endanschlag begrenzt ist, welche die minimalen und maximalen Ausziehpositionen (Teleskophub) definieren, wenn das Sicherungselement 104 mit der Längsnut 118 in Gleiteingriff ist.

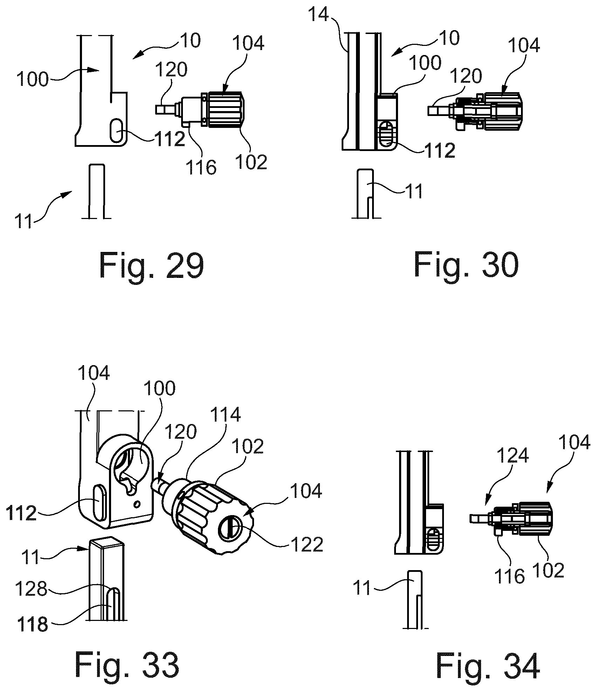

Die Fig. 29 bis Fig. 35 zeigen verschiedene Detailansichten des Sicherungselementes 104.

Die Fig. 29 zeigt das Sicherungselement 104 in Seitenansicht, welches dazu vorgesehen ist, in den entsprechenden Aufnahmeabschnitt 100 an der Teleskopvorrichtung 10 eingesetzt und mittels des Verriegelungselements 112 darin gehalten zu werden. Der entsprechende Aufnahmeabschnitt 100 ist an dem distalen Endbereich des Handgriffelements 14 vorgesehen. Das Handgriffelement 14 umschließt dabei das Gleitstabelement 11. **In** der Fig. 29 ferner gut erkennbar ist ein radial (nach unten) vom Sicherungselement 104 vorragender Zapfen 116, der als Eingriffselement/Hinterschnitt für das Verriegelungselement 112 dient. Andere Rasteingriffslösungen sind natürlich ebenfalls vorstellbar, etwa ein Bajonettverschluss oder eine Verschraubung, etc..

Die Fig. 30 zeigt den Aufnahmeabschnitt 100 samt Verriegelungselement 112 sowie das Sicherungselement 104 im Teilquerschnitt. Demzufolge besteht das Sicherungselement 104 im Allgemeinen aus einem federvorgespannten inneren Bolzen 120, der in einem Drehknopf 102 axialverschiebbar gelagert ist, um in die Längsnut 118 des Gleitstabelements 11 einzugreifen und einem vorzugsweise hülsenförmigen Gehäuse 114 für eine drehbare Aufnahme des Drehknopfs 102 zur Aufbringung einer Feststellungskraft auf das Gleitstabelement 11 unter Umgehung der bzw. parallel zur Federvorspannung.

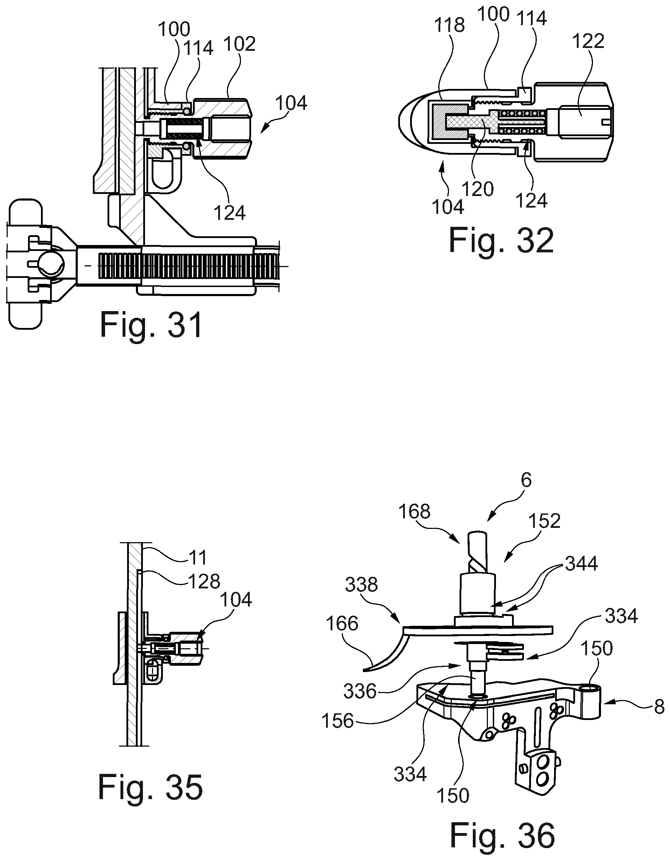

Die Fig. 31 und 32 zeigen das Verriegelungselement 104 im Detail, welches in den Aufnahmeabschnitt 100 des Handgriffs 14 eingesetzt ist. Demzufolge ist in der Längsnut 118 des Gleitstabs 11 ein Gleitblock (ohne Bezugszeichen) aufgenommen, in/an welchem der Bolzen 120 axial abgestützt ist. Der Bolzen 120 hat/bildet in seinem Mittenabschnitt einen Federteller, an dem sich wiederum eine Schraubenfeder 124 abstützt, um eine Vorspannkraft auf den Bolzen 120 in Richtung hin zum Gleitblock aufzubringen. Der Federteller dient gleichzeitig als Anschlagselement, das gegen einen Absatz im Drehknopf 102 anschlägt, wodurch ein Herausfallen des Bolzens 120 und der Feder 124 aus dem Drehknopf 102 verhindert wird. Der Bolzen 120 sowie die Vorspannfeder 124 sind in dem Drehknopf 102 in dessen Axialrichtung aufgenommen, der in das vorzugsweise hülsenförmige Gehäuse 114 axial eingesetzt/eingeschraubt ist, welches wiederum in den Aufnahmeabschnitt 100 eingesetzt und mittels des Verriegelungselements 112 darin gehalten/gesichert ist (siehe insbesondere Fig. 31). **In** den Drehknopf 102 ist eine Stellschraube 122 stirnseitig (an dessen freier Stirnseite) eingedreht, die als Widerlager für die Vorspannfeder 124 dient. Wird demzufolge die Stellschraube 122 innerhalb des Drehknopfes 102 gedreht, kann auf diese Weise die Federvorspannung auf den Bolzen 120 (innerhalb des Drehknopfs) verändert werden.

Darüber hinaus hat der Bolzen 120 einen Spulenabschnitt 121, der sich ausgehend vom Federteller in Richtung hin zur Stellschraube 122 erstreckt sowie von der Vorspannfeder 124 umgeben ist und somit die Vorspannfeder 124 führt.

Die Fig. 33 zeigt das Sicherungselement 104 mit seinem Einstellelement/Stellschraube 122, bspw. seiner Einstellschraube, die dazu vorgesehen ist, die auf den Bolzen (Klemmstift) 120 wirkenden Vorspannkräfte einzustellen. Des Weiteren ist das Verriegelungselement 112 zu erkennen, welches die Verriegelung des Sicherungselementes 104 in dem Aufnahmeabschnitt 100 des Handgriffs 14 bewirkt. **In** dem unteren Bereich der Fig. 33 ist das Gleitstabelement 11 dargestellt, welches den (oberen) Anschlagabschnitt 128 der nutförmige Ausnehmung 118 zeigt, der eine der beiden maximalen Ausziehpositionen 128, 130 des Gleitstabelements 11 bezüglich des Handgriffs 14 repräsentiert.

Die Funktionsweise des Sicherungselements 104 wird nachstehend anhand der Fig. 31 bis 35 kurz erläutert:

Zunächst wird das Sicherungselement 104 in den Aufnahmeabschnitt 100 (in eine dort ausgebildete Bohrung) vorzugsweise gleitend eingesetzt und dessen hülsenförmiges Gehäuse 114 mittels des Verriegelungselements 112 dreh- und axialfest gesichert. In diesem Zustand ragt der Bolzen/Klemmstift 120 innerhalb des Drehknopfs 104 in die Längsnut 118 des Gleitstabelements 11 und beaufschlagt dieses (indirekt über den in der Längsnut 118 aufgenommenen Gleitblock) mit einer Druckkraft in Abhängigkeit der Vorspannkraft der Feder 124. Auf diese Weise kann die Vorspannkraft der Feder 124 durch Drehen der Stellschraube 122 innerhalb des Drehknopfs 104 erhöht und/oder verringert werden, um so die Reibkraft zwischen dem Gleitstab 11 und dem Bolzen 120 des Sicherungselements 104 zu verändern. Damit lässt sich beispielsweise die Reibung auf quasi null reduzieren oder so erhöhen, dass zumindest ein sich Verstellen der aktuellen Hubstellung der Teleskopvorrichtung 10 infolge Schwerkraft vermieden wird.

Um die Hubposition festzustellen (einzufrieren), kann der Drehknopf 104 in das hülsenförmige Gehäuse 114 weiter eingedreht werden, wodurch der Drehknopf 104 ab einer bestimmten Eindreh-Position im Gehäuse 114 unmittelbar, d.h. im konkreten über den Spulenabschnitt 121 des Bolzens 120 (der sich nunmehr an der Stellschraube 122 im Drehknopf 102 axial abstützt) auf der Gleitblock drückt und diesen unter Umgehung der Federvorspannung direkt gegen den Gleitstab 11 drückt/verspannt.

Im Nachfolgenden wir die Höhenantastvorrichtung (auch als Höhenfeststell-/ oder Höhenverstelleinheit bezeichnet) 6 anhand der Fig. 36 bis 44 beschrieben.

Die Fig. 36 zeigt die Werkzeugführungsvorrichtung/Sägeblock 8 vorzugsweise gemäß vorstehender Beschreibung, die eine Aufnahmeausnehmung/Bohrung 150 umfasst/hat, in welche das/die Höhentastelement / Höhenverstelleinheit 6 wahlweise einsetzbar ist. Die Werkzeugführungsvorrichtung 8 hat dabei nur eine einzige oder mehrere Aufnahmebohrung(en)/Aufnahmeausnehmung(en) 150, wobei an dieser Stelle darauf hingewiesen wird, dass stattdessen auch eine Seiteneinkerbung mit Klemmschelle, eine Magnethalterung oder dergleichen vorgesehen sein kann. Die zumindest eine Aufnahmeausnehmung 150 bildet einen Teil einer Adapterschnittstelle 334 zwischen der Werkzeugführungsvorrichtung/Sägeblock 8 und der Höhenverstelleinheit 6.

Grundsätzlich besteht die Höhenverstelleinheit 6 im Wesentlichen aus
- einer Tastspitze 166, die an einem Tastarm 338 montiert oder ausgebildet ist (bilden gemeinsam eine Höhentastbaugruppe 152),
- einem Spindelmechanismus 168, an welcher der Tastarm 338 für zumindest eine Höhenverstellung gelagert ist und
- einem Einsteckdorn/-Schaft 156 vorzugsweise mit Rasteinrichtung/Rastmechanismus 154 als einen weiteren Teil der Adapterschnittstelle 334 für das in Eingriff kommen mit der Aufnahmeausnehmung 150 von einer Oberseite 151 des Sägeblocks 8 aus.

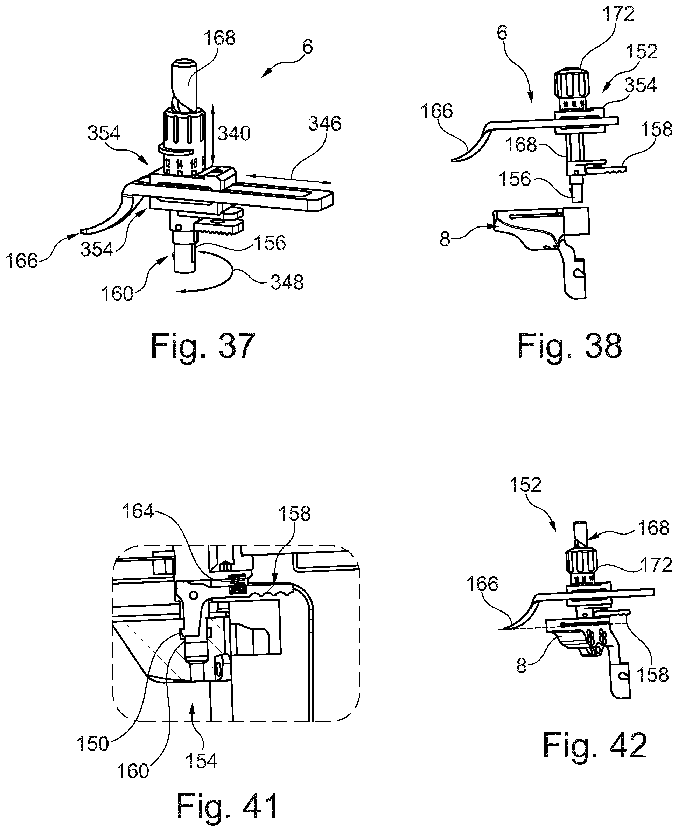

Die Fig. 37 zeigt eine perspektivische Ansicht der Höhenverstelleinheit 6 gemäß der vorliegenden Erfindung. Die Höhenverstelleinheit 6 hat demzufolge einen horizontalen Verschiebbarkeitsfreiheitsgrad 346, einen vertikalen Justierfreiheitsgrad 340 und ein Rotationsfreiheitsgrad 348. Die Höhenverstelleinheit 6 weist hierfür den Einsteckdorn/-Schaft 156 auf, der vorzugsweise als Hohlschaft ausgebildet ist, und somit die Rasteinrichtung 154 in sich aufnehmen kann, die mit der Aufnahmeausnehmung 150 auf Seiten des Sägeblocks 8 für ein axiales Festlegen der Höhenverstelleinheit 6 am Sägeblock 8 in hinterschneidenden Wirkeingriff bringbar ist. Hierfür ist eine federvorgespannte Rastnase 160 im Einsteckdorn 156 vorgesehen, die über den Umfang des Schafts 156 seitlich/radial vorragt und über eine Betätigungstaste 158 in den Einsteckdorn 156 zurückgezogen werden kann, um einen Rasteigriff mit dem Sägeblock 8 aufzuheben. Der Rotationsfreiheitsgrad 348 ist somit durch die Rotationsfreiheit des Einsteckdorns/-Schafts 156 in der Aufnahmebohrung 150 des Tibia-Sägeblocks 8 bewirkt.

Der Tastarm 338 ist mit einem Langloch (siehe Fig. 37) versehen, das vom Spindelmechanismus/Spindel 168 (gleitend/frei) durchdrungen ist, an welchem/welcher der Tastarm 338 über ein zwischengeschaltetes Reibschlusselement/Halteschlitten 354 gekoppelt ist, welches den Tastarm 338 reibbehaftet aber längsverschiebbar (ausziehbar) am Spindelmechanismus 168 hält. Der horizontale Verschiebbarkeitsfreiheitsgrad 346 ist somit durch die horizontale Verschiebbarkeit der Höhentastbaugruppe 152 bzw. des Tastarms 338 am Spindelmechanismus 168 (über das Reibschlusselement 354) bewirkt.

Die Fig. 38 zeigt das Höhentastelement 6 in Seitenansicht, welches dazu vorgesehen ist, in die Werkzeugführungsvorrichtung 8 eingesetzt zu werden. Das Höhentastelement 6 weist demzufolge an seiner unteren (dem Sägeblock 8 zugewandten) Seite den Einsteckdorn 156 und das Betätigungselement in Form eines Hebels 158 auf, welcher im unbetätigten Zustand an einem Hebelanschlag (ohne Bezugszeichen) anliegt, der am Einsteckdorn 156 angeordnet oder ausgebildet ist. Des Weiteren ist zur Antastung der knöchernen Landmarke der Tibia die Tastspitze 166 am äußeren (proximalen) Ende des Tastarms 338 vorgesehen. Schließlich ist der Spindelmechanismus mit einer Spindel/Wendel 168 dargestellt, die axial mit dem Einsteckdorn 156 gekoppelt ist und in ihrem Mittenabschnitt das Reibschlusselement 354 einschließlich des Tastarms 338 trägt.

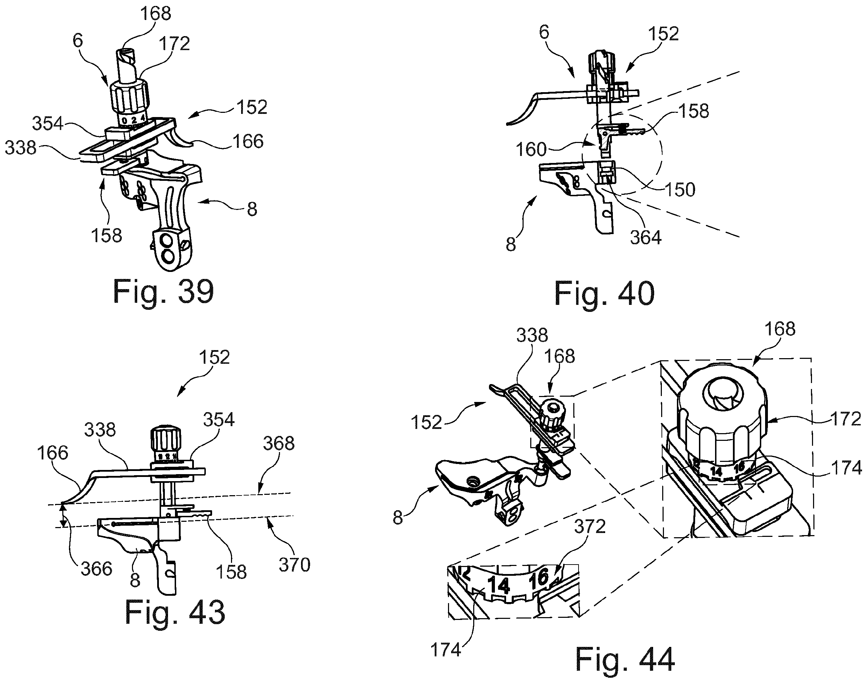

Die Fig. 39 und 40 zeigen in einer Perspektivendarstellung den Sägeblock/ Werkzeugführungsvorrichtung 8, in welcher die Höhenverstelleinheit 6 bereits eingesetzt ist. Der Spindelmechanismus der Höhenverstelleinheit 6 hat die vorstehend bereits angedeutete Spindel/Wendelelement 168 sowie ein Wendelrad 172, welches an dem Wendelelement 168 vorgesehen/gelagert ist. Das Wendelrad 172 ist dabei relativ drehbar mit dem Reibschlusselement 354 gekoppelt, sodass dieses (einschließlich des Tastarms 338) über das Wendelrad 172 an der Spindel 168 (axialbewegbar) gehalten wird.

Ein Ausschnitt aus der Fig. 40 ist in der Fig. 41 gezeigt. Die Fig. 41 zeigt die Rasteinrichtung 154 mit der Rastnase 160, welche in die Aufnahmeausnehmung 150 am Sägeblock 8 eingesetzt wird. Des Weiteren ist ein Federelement 164 zu erkennen, welches zwischen dem Hebelanschlag und dem Betätigungs-Hebel 158 zur Betätigung der Rastnase 160 angeordnet ist und den Hebel 158 in eine Position vorspannt, in welcher sich die Rastnase 160 in einer Rasteingriffsstellung (radial vorragend gemäß Fig. 41) befindet. Im Konkreten ist der Hebel 158 als ein zwei-schenkliger rechtwinklig abgebogender Umlenkhebel ausgebildet, der in seinem Mittenabschnitt innerhalb des Einsteckdorns 156 schwenkgelagert ist, wobei der eine Schenkel den Betätigungshebel/Betätigungsabschnitt bildet und der andere Schenkel mit der Rastnase 160 wirkverbunden ist, die vorzugsweise in Form eines Schiebklotzes ausgebildet ist und von dem einen Schenkel des Betätigungshebels 158 über dessen Federvorspannung radial nach außen gedrückt wird.

Die Fig. 42 und die Fig. 43 zeigen die Werkzeugführungsvorrichtung/Sägeblock 8 mit der eingesetzten Höhenverstelleinheit 6 in unterschiedlichen Einstellpositionen.

Zwischen der Einstellposition gemäß der Fig. 42 und der Einstellposition gemäß der Fig. 43 ist die eingestellte Schnitthöhe/Schnitthöhendifferenz (oder auch als Antastlevel bezeichnet) 366 zu erkennen. Das Antastlevel 368 definiert dabei die Höhe der Tastspitze 166 relativ zu dem horizontalen Säge-Aufnahmeschlitz im Sägeblock 8, welcher das Schnittlevel 370 definiert.

Die Fig. 44 zeigt wiederum die Werkzeugführungsvorrichtung 8 mit dem aufgesetzten Höhentastelement 152, welches das Wendelelement/Spindel 168 aufweist. Das Wendelelement 168 wiederum ist über das Wendelrad 172 zur Höheneinstellung der Höhentastbaugruppe 152 betätigbar. Im Konkreten ist die Wendel/Spindel 168 vorzugsweise im Einsteckdorn 156 relativdrehbar geführt, wobei die Höhentastbaugruppe 152 auf der Wendel 168 über das Reibschlusselement 354 schraubgelagert ist. Das Wendelrad 172 ist wiederum (als weiterer Bestandteil der Höhentastbaugruppe 152) am Reibschlusselement 354 relativ drehbar gelagert, sodass eine Drehung des Wendelrads 172 an der Wendel/Spindel 168 zu einer Verschiebung des Tastarms 338 längs der Spindel 168 führt. Zwischen dem Reibschlusselement 354 und dem Wendelrad 172 ist schließlich bevorzugt ein Rastmechanismus 372 angeordnet, der dazu vorgesehen ist, eine definierte Höhe (Axialposition an der Spindel) des Höhentastelementes 152 zu halten.

Die Funktionsweise der Höhenverstelleinheit/Schnitthöhentaster 6 gemäß der vorliegenden Erfindung lässt sich wie folgt zusammenfassen:
Der einstellbare Schnitthöhentaster 6 hat die grundsätzliche Funktion, dass er als einfache Montage- und als einfache Demontageeinheit an der Werkzeugführungsvorrichtung 8, also an dem Tibia-Sägeblock, vorgesehen ist. Am distalen Ende des Stylus, also der Höhenfeststelleinheit 6, ist der gefederte Rastmechanismus angebracht, der hier durchgängig mit dem Bezugszeichen 154 versehen ist. Der Rastmechanismus 154 arretiert den Stylus, nachdem der Stylus in die dafür vorgesehene Aufnahmeausnehmung (Durchgangsloch) 150 des Tibia-Sägeblocks 8 eingebracht/eingesteckt wurde, wobei vorzugsweise der Stylus dabei um die Steckachse rotierbar bleibt.

Die axiale Arretierung erfolgt dabei über die gefederte Rastnase 160. D.h., beim Einbringen/Einstecken des Einsteckdorns 156 in die Aufnahmeausnehmung 150 wird die gefederte Rastnase 160 aufgrund deren äußerer abgeschrägten (distalen) Anlage-/Abgleitfläche lateral in den Einsteckdorn 156 zurückgeschoben und bei vollständigem Einführen in den Tibia-Sägeblock 8 wird die Rastnase 160 vorzugsweise in eine Nut in dem Tibia-Sägeblock 8 eingerastet. Aus der Einrastposition wird die gefederte Rastnase 160 mittels des Betätigungshebels 158 gelöst, der die Rastnase 160 bei manuellem Verschwenken des Betätigungshebels 158 gegen die Feder 164 zurückzieht. In diesem Zustand kann der Stylus vom Sägeblock 8 einfach abgezogen werden. Mit der Tastspitze 166 wird ferner das Abtasten einer knöchernen Landmarke der Tibia bewirkt. Die vom Anwender gewählte Landmarke ist die Referenz, gegen welche die Schnitthöhe der Werkzeugführungsvorrichtung 8 eingestellt wird, indem das Wendelrad 172 entsprechend gedreht wird. Die Landmarke wird mit der Tastspitze 166 detektiert, die am Ende der des Tastarms 338 angebracht ist. Durch die Tastgenauigkeit der Tastspitze 166 können auch sehr kleine knöcherne Strukturen mittels Augenkontrolle optisch sehr gut und genau detektiert werden.

Die horizontale Verschiebbarkeit der Höhentastbaugruppe 152, insbesondere des Tastarms 338 dient zur Adaption an die verschiedenen Anatomien der Tibia und zum Erreichen der medialen und lateraltibialen Ausrichtung von derselben Adapterstelle aus. Hierzu ist die horizontale Verschiebbarkeit des Tastarms 338 am Reibschlusselement 354 vorgesehen, welche in Fig. 37 dargestellt ist. Mit Hilfe des rotierenden Stylus und des entlang seiner Hauptachse verschiebbaren Tastarms 338 kann jede knöcherne Landmarke an der proximalen Oberfläche der Tibia erreicht werden. Dabei ist die Dimension des Tastarms 338 so ausgelegt, dass die weltweit existierende Anatomie (bspw. von asiatischen oder kaukasischen Menschen) berücksichtigbar ist.

Damit die gewünschte ausgefahrene Länge des Tastarmes 338 beibehalten werden kann, ist der Tastarm 338 selbsthemmend mittels Reibschluss gegen ein Verschieben in Axialrichtung am Reibschlusselement 354 gesichert. Durch das Einrasten des Stylus in der Werkzeugführungsvorrichtung 8, also im Tibia-Sägeblock, und einem definierten Anschlag des Stylus am Sägeblock 8, wird der Abstand der Tastspitze 166 des Stylus bezogen auf die Unterkante des Sägeschlüssels in dem Sägeblock 8 sicher erreicht, wobei die eingestellte Schnitthöhe beispielsweise durch Zahlen 344 am Umfang des Wendelrads 172 angezeigt wird. Die Zahl, welche die eingestellte Höhe angibt, wird bevorzugt mit einem Zeigeelement am anterioren Ende des Reibschlusselements 354 (Halteeinheit) indiziert.

Das Einstellen der Tibia-Schnittdicke von 0 bis 16 mm (oder von 0 bis 14 mm) wird beispielsweise mit nur einer Umdrehung des Wendelrads 172 erreicht. Ein Anschlagelement/Anschlagabschnitt am oberen Ende der Wendel 168 verhindert, dass das Wendelrad 172 vollständig vom Stylus heruntergedreht werden kann.

Nach dem Einstellen der gewünschten Schnitthöhe relativ zu der Tastspitze 166 wird Höhentastbaugruppe 152 gegen die von dem Anwender ausgewählte Landmarke gefahren und die Ausrichtungsvorrichtung wird ausgerichtet. Das Anfahren der Landmarke erfolgt insbesondere durch das (manuelle) Verschieben des Handgriffes 14 mit den Aufbauelementen längs des Gleitstabs 11 bei bereits in Eingriff befindlicher distaler Klemmvorrichtung 2. Ist die Ausrichtungsvorrichtung 1 in Höhe, Varus, Valgus und Slope, wie vom Anwender gewünscht, ausgerichtet, wird die Werkzeugführungsvorrichtung 8, also der Sägeblock, endgültig mit Fixationspins, vorzugsweise Nägeln, durch die dafür vorgesehene Fixierlöcher 300 am Knochen fest verankert. Danach muss/kann der Stylus entfernt werden, um den tibialen Sägeschnitt durchführen zu können. Hierzu kann die Teleskopvorrichtung 1 an der Tibia verbleiben oder zusammen mit der Klemmvorrichtung 2 und dem Adapter 12 einfach abgenommen werden, während der Sägeblock 8 (ohne die bereits abgenommene Antastvorrichtung 6) an der Tibia verbleibt.

Im nachfolgenden wird eine proximale Fixationseinheit/Fixierungsvorrichtung 202 beschrieben, die wahlweise am proximalen Endabschnitt der Teleskopvorrichtung 10 vorzugsweise gemäß vorstehender Beschreibung montierbar ist, um aus einer Ausrichtungsvorrichtung der anterioren Fixationsversion eine Ausrichtungsvorrichtung der proximalen Fixationsversion zu machen.

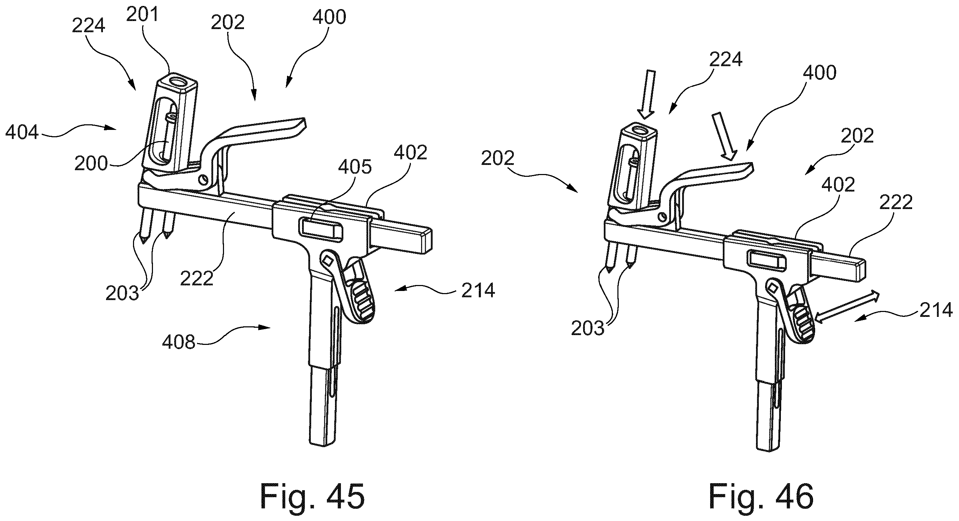

Die Fig. 45 zeigt hierfür eine optionale proximale Einschlagvorrichtung/ Fixationseinheit 202, die bevorzugt zumindest folgendes aufweist:
- einen Einschlagmechanismus 224, der dazu ausgebildet ist, vorzugsweise zwei im Einschlagmechanismus 224 gleitgeführte Pins einzuschlagen,
- einen Schlaghebel 400, der zum Lösen der beiden eingeschlagenen Pins vorgesehen und ausgebildet ist und
- einen Verbindungsmechanismus 406 zum (vorzugsweise klemmenden) Verbinden der proximalen Fixationseinheit 202 an der Ausrichtungsvorrichtung (Teleskopvorrichtung) gemäß der Version "anteriore Fixation" zur wahlweisen/temporären Erzeugung einer Ausrichtungsvorrichtung in der Version "proximale Fixation".

Im Einzelnen hat die Fixierungsvorrichtung 202 einen Querträger 222, an dessen einem freien Endabschnitt der Einschlagmechanismus 224 und der Schlaghebel 400 angeordnet sind. Die Einschlagmechanismus 224 hat eine Schlagpineinheit 404, welche als eine Art Amboss für die bevorzugt zwei Schlagpins dient. Hierfür ist ein zentraler Führungsstift 200 unter einem Winkel nahe 90° zur Querträgerlängsachse im Querträger 222 (fest) verankert, an welchem der Amboss in Form eines den Führungsstift 200 umgebenden Rahmens/Rahmengehäuses 201 gleitgelagert ist. An einer dem Querträger 222 zugewandten Unterseite des Ambosses/Rahmens 201 sind die vorzugsweise zwei Schlagpins 203 in Parallelausrichtung zu dem Führungsstift 200 fixiert, die bevorzugt in zwei Durchgangsbohrungen am Querträger 222 gelagert/geführt sind. Wird somit auf den Amboss manuell ein Hammerschlag ausgeübt, treibt dieser die daran gehaltenen/fixierten, sowie im Querträger 222 längsgeführten Pins 203 in einen Patientenknochen, wobei die Einschlagrichtung durch den am Querträger 222 fixierten, sowie den Amboss längsführenden Führungsstift 200 gewährleitet ist.

Der Schlaghebel 400 ist wippenartig am Querträger 222 anscharniert und hat einen Eingriffsabschnitt auf einer dem Einschlagmechanismus 224 zugewandten Seite, welcher mit dem Amboss/Rahmengehäuse 201 in Wirkeingriff steht und einen Schlagabschnitt auf einer gegenüberliegenden Seite, auf den mit einem Hammer oder dergleichen Schlagwerkzeug geschlagen werden kann. D.h. wird auf den Schlagabschnitt des wippenartigen Schlaghebels 400 geschlagen, übt dessen Eingriffsabschnitt eine Kraft auf die Unterseite des Rahmengehäuses 201 entgegen der Pin-Einschlagrichtung aus, wodurch die Pins 203 aus dem Patientenknochen gezogen werden.

Der Querträger 222 ist axial gleitfähig in einem Aufnahmegehäuse/Fixationselement 402 eingesetzt, in welchem eine Rutsch-/Gleitbremse (in Fig. 45 angedeutet) 405, beispielsweise in Form einer gebogenen Blattfeder untergebracht ist, die eine Axial-Gleitbewegung des Querträgers 222 im Aufnahmegehäuse 402 abbremst. Dabei ist der Querträger 222 bevorzugt aus einem Mehrkantprofil (Rechteckprofil) hergestellt, sodass eine Rotation des Querträgers 222 um dessen Längsachse im Gehäuse 402 verhinderbar ist.

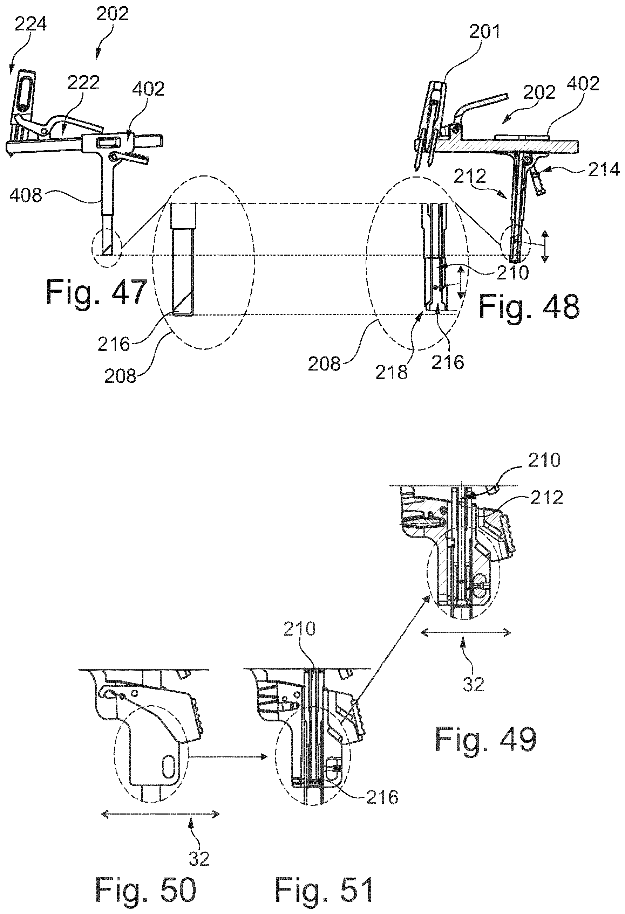

Am Aufnahmegehäuse 402 ist ein Stütz-/Tragpfeiler 408 fixiert, der in einem im Wesentlichen rechten Winkel bzw. leicht geneigt zum Querträger 222 ausgerichtet ist und in/an welchem der Verbindungsmechanismus 406 vorzugsweise in Form eines Rast-/Klemmmechanismus 208 (siehe insbesondere Fig. 47) für ein wahlweises Befestigen der Fixationseinheit 202 an der Teleskopvorrichtung 10 vorgesehen ist. Dieser Klemmmechanismus 208 besteht gemäß der Fig. 47 im Wesentlichen aus einer keilförmigen Klemmplatte 216, die an der freien distalen Stirnseite des Tragpfeilers 408 anliegt, wobei die Stirnseite des Tragpfeilers 408 bevorzugt bezüglich der Pfeilerlängsachse keilförmig angeschrägt/geneigt ist. Der Tragpfeiler 408 ist aus einem zumindest teilweise rohförmigen (Hohl-)Körper 210 gebildet, in welchem ein Zugelement (Zugstab) bzw. Ansteuerelement 210 längsverschiebbar gelagert ist. Die Klemmplatte 216 ist über das im Tragpfeiler 408 geführte Zugelement (Zugstab) bzw. Ansteuerelement 210 mit einem Betätigungshebel 214 wirkverbunden, der am Aufnahmegehäuse 402 für den Querträger 222 schwenkbar gelagert ist. Der Betätigungshebel 214 hat gemäß der Fig. 48 und 49 einen Betätigungsabschnitt mit einer vorzugsweise aufgerauten oder gerippten Drucktaste zur rutschsicheren Druckbeaufschlagung des Betätigungshebels 214 beispielsweise mittels eines Daumens des Anwenders. Wird folglich der Betätigungshebel 214 umgelegt und so eine Zugkraft auf den Zugstab 210 ausgeübt, wird dadurch die keilförmige Klemmplatte 216 längs der (keilförmig) angeschrägten Stirnseite 218 des Tragpfeiles 408 radial nach außen verschoben und so der Gesamtquerschnitt des Pfeilers 408 künstlich vergrößert.

Der Tragpfeiler 408 hat zudem an seinem gehäuseabgewandten (distalen) Endabschnitt einen Bereich mit kleinem Querschnitt(sfläche) und in seinem gehäusezugewandten (proximalen) Endabschnitt einen Bereich mit großem Querschnitt(sfläche), welche durch einen umlaufenden Absatz (siehe insbesondere Fig. 48) voneinander getrennt sind. Der Bereich mit kleinem Querschnitt ist so dimensioniert, dass er (mit geringem Spiel) in den hohlförmigen Teleskopstab/Gleitstabelement 11 der Teleskopvorrichtung 10 einsteckbar und mittels des Klemmmechanismus 208 darin feststellbar ist. Der Bereich mit großem Querschnitt entspricht im Wesentlichen dem Außenquerschnitt des Gleitstabelements 11, sodass bei vollständigem Einstecken des Tragpfeiles 408 in das Gleitstabelement 11 (bis zum umlaufenden Absatz als Anschlag) eine im Wesentlichen glatte Gleitstaboberfläche entsteht.

Die Fig. 46 zeigt die Fixationseinheit 202 mit dem Einschlagmechanismus 224 einschließlich deren Betätigungsmöglichkeiten. Die Fixation wird damit durch Einschlagen der Pins 203 vorzugsweise durch einen Hammerschlag bewirkt. Durch einen weiteren Hammerschlag auf den Schlaghebel 400 werden die Pins 203 gelöst. Durch Betätigen der Drucktaste 214 wird der Klemmmechanismus 208 aktiviert/deaktiviert.

Die Fig. 47 zeigt die Fixationseinheit 202, welche den Fixierungsmechanismus 224 und den Verbindungs-/Klemmmechanismus 208 aufweist. Wenn sich der Verbindungs-/Klemmmechanismus 208 bzw. dessen manueller Betätigungshebel 214 in nach oben (in Richtung proximal) gezogener Stellung befindet, ist die Klemmmwirkung zwischen dem Tragpfeiler 408 und dem Gleitstabelement 11 aufgehoben, in welches der Tragpfeiler 408 in der proximalen Fixationsvariante der Ausrichtungsvorrichtung 1 eingesteckt ist. Der Verbindungs-/Klemmmechanismus 208 wirkt dabei über das Ansteuerelement/Zugstab 210 auf die keilförmige Klemmplatte 216, welche durch die Abschrägung 218 der distalen Stirnseite des Tragpfeilers 408 radial nach außen oder innen bezüglich des Tragpfeilers 408 bewegbar ist. Sobald die Klemmplatte 216 über den Zugstab 210 nach oben (in Richtung proximal) gezogen wird, indem der Betätigungshebel 214 nach unter (in Richtung distal) gedrückt wird, wird die Klemmplatte 216 seitlich, insbesondere nach radial außen in eine Klemmposition mit dem Gleitstab 11 bewegt, in welchen der Tragpfeiler 408 bereits eingeführt ist. Wenn hingegen der Hebel 214 nach oben gedrückt wird, bewegt sich das Ansteuerelement 210, also der Stab nach unten (in Richtung distal) und gibt die Klemmplatte 216 frei. Diese wandert infolge ihrer Keilform radial nach innen, wodurch die Klemmwirkung aufgehoben wird.

Die Fig. 47 zeigt den geschlossenen Klemmmechanismus 208 gemäß der Fig. 48 in vergrößerter Darstellung, bei welchem der Betätigungshebel 216 nach unten bewegt ist, wie dies vorstehend bereits beschrieben wurde. Demzufolge ist die keilförmige Klemmplatte 216 an ihrer, dem Tragpfeiler 408 zugewandten Seite unter einem Winkel von ca. 45° bezüglich der Plattenmittelachse ebenfalls angeschrägt. Eine in etwa gleiche Schräge findet sich auch an der freien (distalen) Stirnseite des Tragpfeilers 408, sodass bei einem Wirk-/Gleiteingriff beider Schrägseiten die Klemmplatte 216 in etwa senkrecht zur Mittelachse des Stützpfeilers 408 ausgerichtet bleibt. Alternativ zu dieser Konstruktion ist es aber gemäß der Fig. 48 bis 51 auch möglich, am distalen Ende/Endabschnitt des hohlen Stützpfeilers 408 eine Art Spreizkegel vorzusehen, der den distalen Endabschnitt bei einer entsprechenden Betätigung des Betätigungshebels 214 in den hohlen/rohrförmigen Stützpfeiler 408 hineinzieht und diesen radial elastisch aufweitet. Zu diesem Zweck kann der Stützpfeiler 408 in seinem distalen Endabschnitt mit Spreizschlitzen (nicht weiter dargestellt) ausgeformt sein. Auch ist es denkbar, einen elastischen Verspannkörper (z.B. aus einem Kunststoffmaterial) am distalen Ende des Stützpfeiles 408 vorzusehen, der bei Betätigung des Hebels 214 axial zusammengedrückt wird und dabei Kunststoffmaterial radial nach außen verdrängt.

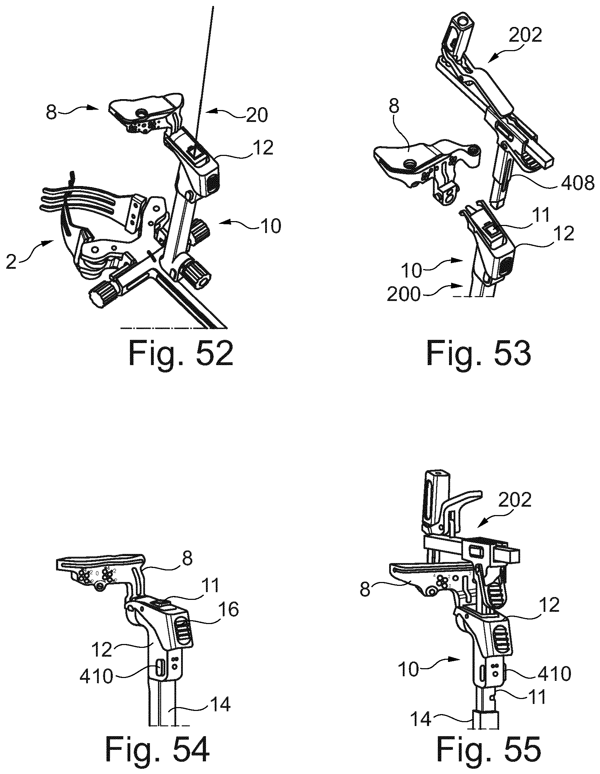

Die Fig. 52 bis 55 zeigen die Ausrichtungsvorrichtung in der "anterioren Fixationsvariante", insbesondere deren Teleskopvorrichtung 10 mit der Werkzeugführungsvorrichtung 8, welche an die Teleskopvorrichtung 10 bereits adaptiert ist. In Längsrichtung der Teleskopvorrichtung 10 erstreckt sich eine Längsachse 20 als Referenzachse, welche gemäß Fig. 52 gleichzeitig auch die Mittelachse des Gleitstabs 11 darstellt. Gut erkennbar ist der Sägeblockadapter 12, der axial (längs der Längsachse 20) verschiebbar über den Gleitstab 11 mittels des Adapters oberhalb/proximal vom Handgriff 14 geführt ist, wobei der Gleitstab 11 an der Oberseite (proximale Seite) des Sägeblockadapters 12 aus der darin ausgeformten, den Gleitstab 11 aufnehmenden Durchgangsöffnung vorragt, wie dies beispielsweise in der Fig. 54 dargestellt ist.

Die Fig. 53 zeigt die Teleskopvorrichtung 10 in Explosionsdarstellung, wonach der Gleitstab 11 als hohlförmiger Schaft ausgebildet ist, der in Richtung proximal eine offene Stirnseite hat. In der gezeigten Explosionsdarstellung sind die Werkzeugführungsvorrichtung / Sägebock 8 und die optionale Fixationseinheit 202 zur wahlweisen Ausbildung der Ausrichtungsvorrichtung 1 als "proximale Fixationsvariante" dargestellt. Gut dargestellt sind die äußeren Abmessungen des Gleitstabs 11 sowie des Stützpfeilers 408 der Fixationseinheit 202, dergestalt, dass der Stütz-/Tragpfeiler 408 in seinem distalen Endabschnitt mit kleinem Außendurchmesser nahezu spielfrei in den Gleitstab 11 bis maximal zu dem Absatz für eine Längeneinstellung/Anpassung des Gleitstabs 11 an die Patientenanatomie einführbar ist, welcher den Stützpfeiler-eigenen distalen Endabschnitt mit kleinem Außendurchmesser vom proximalen Abschnitt mit großem Außendurchmesser trennt, welcher im Wesentlichen dem Außendurchmesser des Gleitstabs 11 entspricht.

Die Fig. 54 zeigt die Werkzeugführungsvorrichtung 8 sowie den Adapter 12, der oberhalb des Handgriffs 14 auf den Gleitstab 11 gesetzt ist und dessen Betätigungselement, vorzugsweise der Drück-/Schiebeknopf 410, nicht betätigt/gedrückt ist, sodass der Tibia-Schnittblockadapter 12 am Handgriff 14 der Teleskopvorrichtung 10 zu einer Einheit arretiert ist. In diesem Zustand kann der Handgriff 14 relativ zum Gleitstab 11 für ein Ausrichten der Antastnadel 166 verschoben werden. Dies entspricht einer Ausrichtungsvorrichtung 1 der anterioren Fixationsvariante. Die Fig. 55 zeigt hingegen die Teleskopvorrichtung 10, bei welcher der Drückknopf 410 am Adapter 12 gedrückt worden ist und somit der Tibia-Schnittblockadapter 12 vom Handgriff 14 gelöst und damit relativ zu diesem frei bewegbar ist. Die Einschlagvorrichtung 202 ist in den Hohlkörper/Gleitstabelement 11 eingesetzt. Dies entspricht der Ausrichtungsvorrichtung 1 in der proximalen Fixationsvariante, wonach die Teleskopvorrichtung 10, d.h. der Gleitstab 11 durch den Stützpfeiler 408 in Richtung proximal verlängert ist und somit eine verlängerte Bewegungsführung für den Sageblockadapter 12 bildet.

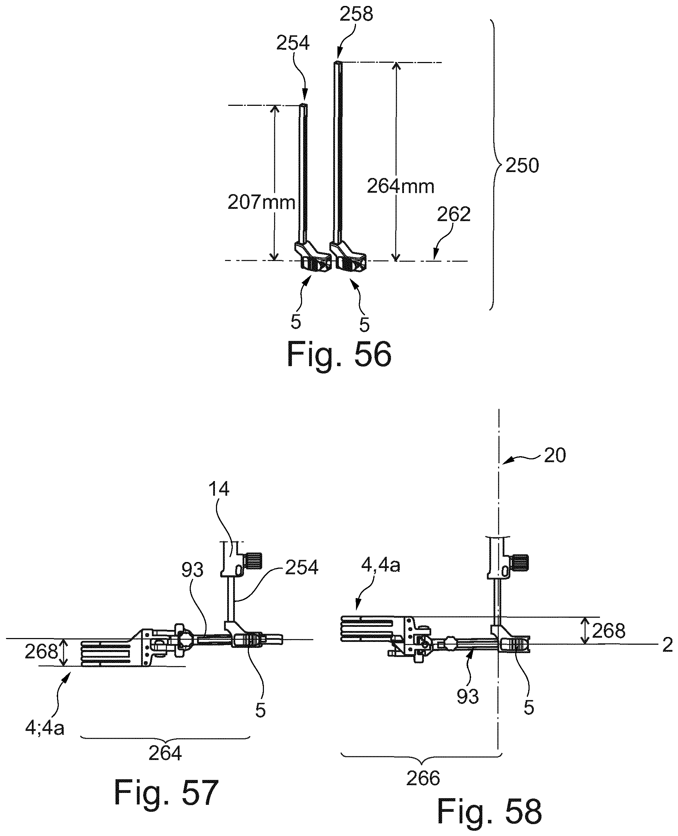

Die Fig. 56 zeigt einen Gleitstabelementensatz 250, beispielsweise aus mindestens zwei zueinander unterschiedlich langen Gleitstabelementen 254 und 258. Das erste Gleitstabelement 254 weist beispielsweise eine erste (kurze) Länge auf, die beispielsweise 207 mm beträgt, welche somit den kurzen Gleitstab kennzeichnet. Der zweite Gleitstab 258 weist eine zweite (lange) Länge auf, die unterschiedlich zur ersten Länge ist und beispielsweise 264 mm beträgt. Die Längsachse der Fußklammeraufnahmevorrichtung/Aufnahmeschacht 5 definiert dabei den jeweils unteren Punkt der Gleitstäbe 254, 258, von welchem aus die Gleitstablänge jeweils gemessen werden kann.

An dieser Stelle sei darauf hingewiesen, dass der erfindungsgemäße Satz 250 an Gleitstäben auch mehr als zwei Gleitstäbe unterschiedlicher Längen aufweisen kann. Auch ist es durchaus denkbar, alternativ oder zusätzlich hierzu mehrere Fixationseinheiten mit unterschiedlich langen Stützpfeilern 408 in einem Satz vorzusehen, um unterschiedlichen Patientenanatomien Rechnung zu tragen.

Die Fig. 57 zeigt den distalen Endbereich der Teleskopvorrichtung 10 mit der Fußklammeraufnahmevorrichtung/Aufnahmeschacht 5, welche an dem ersten (kurzen) Gleitstab 254 befestigt ist. Der Kragarm 93 ist dabei von der Fußklammeraufnahmevorrichtung 5 aufgenommen. Die Klammerelemente 4, 4a sind bezüglich des Kragarms 93 durch entsprechende Drehausrichtung des Kragarms 93 im Aufnahmeschacht 5 zum distalen Ende hin versetzt angeordnet. Es ist somit eine Längenveränderung um +15 mm im Vergleich zu einer zentralen Klammerelemente-Anordnung bewirkt, wie dies vorstehend bereits ausgeführt wurde.

Die Fig. 58 zeigt eine zweite Stellung der Fußklammervorrichtung. Die Klammerelemente 4, 4a sind in diesem Fall bezüglich des Kragarms 93 zum proximalen Ende hin versetzt angeordnet (also nach oben), wobei der Kragarm 93 wiederum von der Fußklammeraufnahmevorrichtung 5 aufgenommen ist. Die Mittelachse der Fußklammeraufnahmevorrichtungen 2 und die Oberkante der Fußklammerelemente 4, 4a zeigen den Höhenversatz 268. In der zweiten Stellung ist eine mittlere Längenveränderung von -15 mm im Vergleich zu einer zentralen Klammerelemente-Anordnung bereitgestellt.

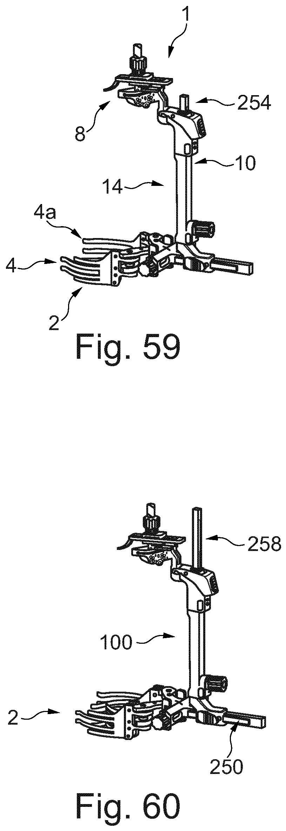

Die Fig. 59 zeigt die Ausrichtungsvorrichtung 1 mit der Werkzeugführungsvorrichtung 8, die auf dem kurzen Gleitstab 254 aufgesetzt ist. Die Fig. 60 zeigt die Ausrichtungsvorrichtung 1 mit dem langen Gleitstab 258, der gegenüber der kurzen Gleitstabversion deutlich weiter über den Handgriff 14 in Richtung proximal vorragt.

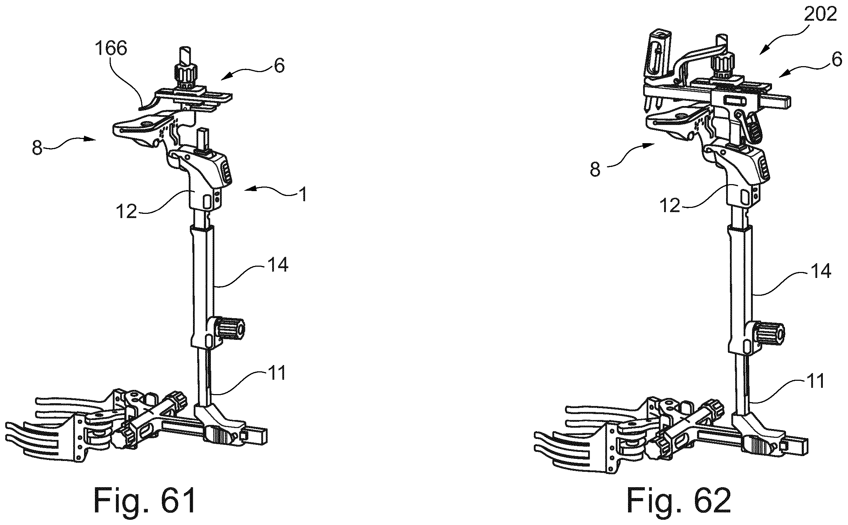

Die Fig. 61 zeigt die Ausrichtungsvorrichtung 1 mit der Werkzeugführungsvorrichtung 8 und der Antastvorrichtung 6 für die Version "Anteriore Fixierung", bei welcher der Sageblockadapter 12 für eine Feinjustage des Höhenabstands zwischen Abtastnadel 166 und Sägeblock 8 vom Handgriff 14 gelöst ist. Die Fig. 62 zeigt die Ausrichtungsvorrichtung 1, welche die Werkzeugführungsvorrichtung 8 umfasst und auf welche die Antastvorrichtung 6 mit der Einschlagvorrichtung 202 für die proximale Fixierung zusätzlich aufgesetzt ist. Auch in diesem Fall ist der Sägeblockadapter 12 vom Handgriff 14 gelöst. Durch die Ausbildung des erfindungsgemäßen Satzes an unterschiedlich langen Gleitstäben ist die Ausrichtungsvorrichtung 1 für die weltweit gegebenen unterschiedlichen Beinlängen, z. B. für Asiaten mit kurzen Beinlängen oder Kaukasier mit sehr langen Beinlängen, anwendbar. Dieses wird in vorteilhafter Weise durch ein einfaches Auswechseln der Gleitstäbe mit unterschiedlichen Stablängen erreicht.

Durch diese Längenausführungen sind die folgenden Beinlängen einstellbar:
- Mit dem kurzen Gleitstab 254 sind Beinlängen von ca. 200 mm bis ca. 380 mm einstellbar und
- mit dem langen Gleitstab 258 sind Beinlängen von ca. 260 mm bis ca. 438 mm einstellbar.

Durch das Überlappen der Nutzlängen der beiden Gleitstablängen 254 und 258 von ca. 120 mm kann sich der Anwender für einen der Gleitstäbe 254, 258 entscheiden und er kann in vorteilhafter Weise die Ausrichtungsvorrichtung 1 bei einem Großteil der Patienten anwenden.

Hinsichtlich der beiden Ausführungsformen zur Ausrichtung differenzieren sich die Längeneinstellungen bevorzugt wie folgt:
- Für die beispielsweise in der Fig. 61 gezeigte "Anteriore Fixation" weist der erste Gleitstab 254 eine Länge 256 von ca. 200 mm bis ca. 360 mm auf und das zweite Gleitstabelement 258 weist eine Länge von ca. 260 mm bis ca. 420 mm auf.
- Für die in der Fig. 62 gezeigte Ausrichtungsvorrichtung 1 der Ausführungsform "Proximale Fixation" weist der erste Gleitstab 254 der kurzen Gleitstablänge eine erste Länge, von 255 mm bis ca. 380 mm auf. Bezüglich der zweiten Länge des zweiten Gleitstabelementes 258 weist dieses für die Ausführungsform der in Fig. 62 gezeigten proximalen Fixation eine Länge von ca. 315 mm bis ca. 438 mm auf.

Des Weiteren wird durch die Wendbarkeit der Fußklammer 2 um 180° eine Längenveränderung um + bzw. -15 mm erreicht, wie dieses in den Fig. 57 und 58 gemäß vorstehender Beschreibung gezeigt ist. Diese Längenänderung erfolgt in vorteilhafter Weise ohne den Austausch des Gleitstabes 11. Die beschriebene Längenänderung um + bzw. -15 mm, welche durch die Wendbarkeit der Fußklammer erreicht wird, ist bereits in der vorstehend ausgeführten Beschreibung der Längenänderung der Ausrichtungsvorrichtung mit eingerechnet. Für sehr kurze Längen der Tibia besteht bei der "Anteriore Fixation" noch die Möglichkeit, die in der Fig. 61 gezeigt ist, die Einstellung auf ca. 180 mm zu reduzieren. Dieses hat jedoch zur Konsequenz, dass das Langloch bzw. der Einschlagschlitz 302 in der Werkzeugführungsvorrichtung 8, also dem Tibia-Sägeblock, für die Version primäre anteriore Fixation der Ausrichtungsvorrichtung (der ETA) nicht genutzt werden kann.

Im Folgenden sind bevorzugte Ausführungsformen der erfindungsgemäßen Ausrichtungsvorrichtung 1 zusammenfassend beschrieben:
Eine erste Ausführungsform der Ausrichtungsvorrichtung 1 für eine tibiale Resektionsführung hat:
- eine Klemmvorrichtung 2, die zumindest zwei gegeneinander wirkende Klammerelemente 4, 4a zum Klemmen des distalen Endes einer Tibia 3 eines Patienten aufweist;
- eine Werkzeugführungsvorrichtung oder Sägeblock 8 zum Führen eines Werkzeuges während der Resektion der Tibia 3,
- optional eine Antastvorrichtung 6 zum Antasten des proximalen Endes der Tibia 3, die an der Werkzeugführungsvorrichtung 8 montierbar ist und
- eine Teleskopvorrichtung 10, die proximal mit der Werkzeugführungsvorrichtung 8 und distal mit der Klemmvorrichtung 2 trennbar verbunden ist und die dazu ausgebildet ist, die Vorrichtungen 2, 6 gegenüber der Tibia 3 auszurichten, wobei
- die Teleskopvorrichtung 10 in ihrem proximalen Bereich 52 zusätzlich eine Entkopplungsvorrichtung bzw. Schnittblockadapter 12 aufweist, der dazu ausgebildet ist, bei manueller Aktivierung den Sägeblock 8 von der Teleskopvorrichtung 10 zu trennen; und/oder dass
- die Klammerelemente 4 der Klemmvorrichtung 2 biegeelastisch sind, sodass die Teleskopvorrichtung 10 vorzugsweise nach Aktivierung der Entkopplungsvorrichtung 12 unter Ausnutzung der Biegeelastizität der Klammerelemente von der Tibia abziehbar ist.

Ferner kann es vorgesehen sein, dass die Teleskopvorrichtung 10 in ihrem proximalen Bereich 52 einen Handgriff 14 aufweist, der so ausgestaltet ist, dass dieser von der einen Hand eines Bedieners umgreifbar ist und dass die Entkopplungsvorrichtung/Sägeblockadapter 12 zur Aktivierung/Sägeblockfreigabe oberhalb des Handgriffs 14 ein Betätigungs-/Druckelement 16 aufweist, welches vorzugsweise in einem Winkel A 18 zwischen 90° und 150°, weiter vorzugsweise in einem Winkel A zwischen 95° und 120° Grad und insbesondere in einem Winkel A 18 von 100° Grad zu der Längsachse 20 der Teleskopvorrichtung 10 angeordnet ist, so dass das Druckelement 16 von dem Daumen der einen Hand 56 betätigbar ist.

Ferner kann es vorgesehen sein, dass die Klemmvorrichtung 2 im distalen Bereich 54 der Teleskopvorrichtung angeordnet ist und dass die beiden federelastischen Klammerelemente 4, 4a jeweils eine bogenförmige Form aufweisen, die so zueinander ausgerichtet sind, dass diese in Richtung der Längsachse 20 der Teleskopvorrichtung 10 betrachtet im geschlossenen Zustand zwischen sich einen ovalförmigen Bereich 22 ausbilden, der zur klemmenden Aufnahme des distalen Bereiches der Tibia 3 vorgesehen ist.

Ferner kann es vorgesehen sein, dass die Entkopplungsvorrichtung 12 einen Grundkörper 24 aufweist, welcher zumindest ein männliches (oder weibliches) Aufnahmeelement 26 umfasst, welches dazu ausgebildet ist, mit zumindest einem weiblichen (oder männlichen) Aufnahmeelement 28 der Werkzeugführungsvorrichtung 8 in formschlüssigen Wirkeingriff zu kommen und dass das Betätigungs-/Druckelement 16 der Entkopplungsvorrichtung 12 ein wirkverbundenes Haken-/Bügelelement 30 aufweist, welches dazu ausgebildet ist, eine Hinterschneidung an der Werkzeugführungsvorrichtung 8, vorzugsweise zumindest ein von der Werkzeugführungsvorrichtung 8 in Querrichtung 32 zu der Längsachse 34 verlaufendes Pin-/Stiftelement 44 zu umfassen, so dass im geschlossenen Zustand ein Lösen der Aufnahmeelemente 26, 28 voneinander verhindert ist. In bevorzugter Weise sind die männlichen Aufnahmeelemente 28 konusförmig ausgestaltet.

Ferner kann es vorgesehen sein, dass der Grundkörper 24 der Entkopplungsvorrichtung 12 einen Lagerzapfen aufweist, der in Querrichtung betrachtet vorzugsweise zwischen dem mindestens einen männlichen Aufnahmeelement 26 und einer Aufnahmeausnehmung 40 für einen Gleitstab 11 der Teleskopvorrichtung 10 angeordnet ist und dass das Haken-/Bügelelement 30 an dem Lagerzapfen, vorzugsweise in einem Winkel bis zu 30°, verschwenkbar angeordnet/gelagert ist.

Ferner kann es vorgesehen sein, dass das Stiftelement 44 der Werkzeugführungsvorrichtung 8 in Querrichtung betrachtet tropfenförmig ausgebildet ist und dass bei Ineinanderfügen des männlichen Aufnahmeelementes 26 der Werkzeugführungsvorrichtung 8 in das weibliche Aufnahmeelement 28 des Grundkörpers 24 das Hakenelement 30 an der Tropfenform 44 entlanggleitet und dadurch anhoben wird, so dass das Hakenelement 30 mit dem tropfenförmigen Stiftelement 44 bei Ineinanderfügen der Aufnahmeelemente 26, 28 verrastet.

Ferner kann es vorgesehen sein, dass der Grundkörper 24 der Entkopplungsvorrichtung zusätzlich einen in dessen Querrichtung 32 verlaufenden Anschlagszapfen aufweist, der vorzugsweise zwischen dem Lagerzapfen und einer Anschlagsfläche für die Werkzeugführungsvorrichtung 8 angeordnet ist und dass in dem Hakenelement 30 zusätzlich eine Anschlagskerbe ausgebildet ist, die dazu vorgesehen ist, durch Zusammenwirken mit dem Anschlagszapfen bei einer nicht verrasteten Werkzeugführungsvorrichtung 8 den Schwenk-Endanschlag des Hakenelementes 30 zu bilden.

Eine unabhängig beanspruchbare Ausführungsform der Ausrichtungsvorrichtung 1 für eine tibiale Resektionsführung ist dadurch bereitgestellt, dass diese an ihrem distalen Bereich 54 eine Klemmvorrichtung 2 aufweist, die zumindest zwei gegeneinander wirkende Klammerelemente 4, 4a zum Klemmen des distalen Endes einer Tibia eines Patienten umfasst; wobei die Klammerelemente 4 der Klemmvorrichtung 2 jeweils bogenförmig ausgebildet sind und so zueinander ausgerichtet sind, dass diese (in einem geschlossenen Zustand) in Richtung der Längsachse 20 der Ausrichtvorrichtung 1 betrachtet zwischen sich einen ovalförmigen Bereich 22 ausbilden, der zur klemmenden Aufnahme des distalen Bereiches der Tibia ausgebildet ist, wobei die Klammerelemente 4, 4a jeweils federelastisch ausgebildet sind, so dass die Klemmvorrichtung 2 der Ausrichtvorrichtung (1) einhändig von der Tibia (5) abziehbar ist.

Ferner kann es vorgesehen sein, dass die Klammerelemente 4, 4a zusätzlich gabelförmig ausgebildet sind und dass die Zinken 62 der jeweiligen Gabel 60 so zueinander versetzt angeordnet sind, dass diese im geschlossenen Zustand überlappend ineinandergreifen, so dass die Tibia 3 festgehalten ist.

Ferner kann es vorgesehen sein, dass die jeweiligen Spitzen 66 der Zinken 62 entgegen der jeweiligen Bogenform der Klammerelemente 4,4a geformt sind, so dass der Abziehvorgang von der Tibia 3 atraumatisch erfolgt.

Ferner kann es vorgesehen sein, dass die (distalen/scharniernahen) Enden 68 der Klammerelemente/Klemmgabeln 4, 4a jeweils von einem Verstärkungselement 70 umfasst/umgriffen und gehaltert sind, welches das jeweilige Klammerelement 4, 4a mit jeweils einem an einen V-förmigen Anlageblock 86 angebundenes Rastelement 74 verbindet, wobei das Verstärkungselement 70 vorzugsweise aus Kunststoff gefertigt ist.

Ferner kann es vorgesehen sein, dass die Rastelemente 74 jeweils einen Ratschenmechanismus 76 aufweisen, so dass die Klammerelemente/Klammergabeln 4, 4a so vorspannbar sind, dass im geschlossenen Zustand 64 eine einstellbare Vorspannkraft auf die Tibia wirkt.

Ferner kann es vorgesehen sein, dass der V-förmige Anlagebock 86 in Längsrichtung 20 der Ausrichtvorrichtung 1 betrachtet einen mittigen Anlagebereich 78 aufweist, von dem sich beidseitig jeweils seitliche Anlagearme 82 V-förmig erstrecken, mit einem Winkel C vorzugsweise in einem Bereich zwischen 30° und 60°, weiter vorzugsweise in einem Bereich zwischen 40° und 50° und insbesondere einen Winkel von 45°.

Ferner kann es vorgesehen sein, dass der V-förmige Anlageblock 86 über einen Spindelmechanismus 90 seitenverstellbar an ein T-Stück 92 angekoppelt ist (wodurch sich im Zusammenspiel mit den Lagerarmen 82 des Anlageblocks 86 insgesamt eine Y-Form ergibt), welches vorzugsweise über eine Raststruktur 94 verstellbar an die Teleskopvorrichtung 10 der Ausrichtvorrichtung 1 angebunden ist.

Eine ggf. unabhängig beanspruchbare Ausführungsform der Ausrichtungsvorrichtung 1 für eine tibiale Resektionsführung hat
- eine (distale) Klemmvorrichtung 2 zum Klemmen des distalen Endes einer Tibia eines Patienten;
- eine (proximale) Werkzeugführungsvorrichtung 8 zum Führen eines Werkzeuges während der Resektion der Tibia,
- eine Teleskopvorrichtung 10, die proximal mit der Werkzeugführungsvorrichtung 8 und distal mit der Klemmvorrichtung 2 verbunden/verbindbar ist und die dazu ausgebildet ist, die proximale und distale Vorrichtungen 2, 6 gegenüber der Tibia auszurichten, wobei die Teleskopvorrichtung 10 ein Handgriffelement 14 aufweist, welches dazu ausgebildet ist, ein verschiebbar darin gelagertes Gleitstabelement 11 ausziehbar aufzunehmen, wobei
die Teleskopvorrichtung 10 ein Feststell-/Sicherungselement 104 aufweist, welches zwischen dem Handgriffelement 14 und dem Gleitstabelement 11 angeordnet ist und welches in eine erste Stellung einstellbar ist, in der eine erste Druckkraft zwischen den Elementen 14, 11 bewirkt ist, die eine gebremste Relativverschiebung beider Elemente 14, 11 erlaubt und welches zusätzlich in eine zweite Stellung einstellbar ist, in der eine zweite Druckkraft zwischen den Elementen 14, 11 bewirkt ist, welche beide Elemente 14, 11 zueinander feststellt.

Ferner kann es vorgesehen sein, dass das Sicherungselement 104 zusätzlich so einstellbar ist, dass in einer dritten Stellung eine dritte Druckkraft zwischen den Elementen 14, 11 wirkt, die eine im Wesentlichen ungebremste Relativverschiebung beider Elemente 14, 11 erlaubt.

Ferner kann es vorgesehen sein, dass zwischen dem Sicherungselement 104 und der Teleskopvorrichtung 10 ein Verriegelungselement 112 angeordnet ist, welches dazu ausgebildet ist, in einer Verriegelungsstellung das Sicherungselement 104 an der Teleskopvorrichtung 10 zu halten und in einer Freigabestellung eine Freigabe des Sicherungselementes 104 zu bewirken, so dass dieses von der Teleskopvorrichtung 10 abnehmbar ist.

Ferner kann es vorgesehen sein, dass das Gleitstabelement 11 in seiner Längserstreckung eine nutförmige Ausnehmung 118 aufweist, die dazu vorgesehen ist, einen Klemmstift 120 des Sicherungselementes 104 aufzunehmen und diesen zu führen. Die nutförmige Ausnehmung 118 bewirkt in vorteilhafter Weise eine definierte Führung des Klemmstiftes 120.

Ferner kann es vorgesehen sein, dass das Sicherungselement 104 ein Einstellelement 122 aufweist, welches so auf ein Federelement 124 des Sicherungselementes 104 wirkt, dass die vom Federelement 124 erzeugten Druck-/Klemmkräfte in den jeweiligen Stellungen 106,108, 110 einstellbar sind.

Ferner kann es vorgesehen sein, dass in Querrichtung 32 der Ausrichtvorrichtung 1 betrachtet an der abgewandten Seite der Tibia ein Aufnahmeelement/Aufnahmeabschnitt 100 am Handgriff 14 angeordnet/ausgebildet ist, welches dazu ausgebildet ist, das Verriegelungselement 112 vorzugsweise verschiebbar aufzunehmen.

Ferner kann es vorgesehen sein, dass das Gleitstabelement 11 in seiner nutförmigen Ausnehmung 118 an seinen beiden axialen Endabschnitten Anschläge 128, 130 für das Sicherungselement 104 aufweist, um ein Herausgleiten des Gleitstabelement 11 aus dem Handgriffelement 14 zu verhindern.

Eine ggf. unabhängig beanspruchbare Ausführungsform der Ausrichtungsvorrichtung 1 für eine tibiale Resektionsführung, umfasst
- eine Klemmvorrichtung 2 zum Klemmen des distalen Endes einer Tibia 3 eines Patienten;
- eine Werkzeugführungsvorrichtung 8 zum Führen eines Werkzeuges während der Resektion der Tibia,
- eine Teleskopvorrichtung 10, die proximal mit der Werkzeugführungsvorrichtung 8 und distal mit der Klemmvorrichtung 2 verbunden ist und die dazu ausgebildet ist, die Vorrichtungen 2, 8 gegenüber der Tibia 3 auszurichten,
wobei
die Werkzeugführungsvorrichtung 8 eine Aufnahmeausnehmung 150 aufweist, die dazu ausgebildet ist, eine Antastvorrichtung 6 zur Ertastung der Resektionshöhe wiederlösbar aufzunehmen.

Ferner kann es vorgesehen sein, dass die Aufnahmeausnehmung 150 eine Bohrung ist, die sich ausgehend von der proximalen Oberseite 151 der Werkzeugführungsvorrichtung 8 entlang der Längsachse 20 der Ausrichtungsvorrichtung 1 erstreckt und dass die Antastvorrichtung 6 ein einen Rastmechanismus 154 aufweisendes Einsteckelement/Einsteckdorn 156 aufweist, welches in die Aufnahmeausnehmung 150 (axial) verrastbar (und drehbar) einsteckbar ist.

Ferner kann es vorgesehen sein, dass der Rastmechanismus 154 ein Hebelelement/Betätigungshebel 158 hat, welches vorzugsweise in/an dem Einsteckdorn 156 angeordnet ist und über welches eine Rastnase 160 betätigbar ist, die im eingesteckten Zustand des Einsteckdorns 156 hinter die Aufnahmeausnehmung 150 greift, um die Antastvorrichtung 6 darin axial zu halten.

Ferner kann es vorgesehen sein, dass die Antastvorrichtung 6 eine Höhentastbaugruppe 152 aufweiset, die eine am proximalen Ende eines Tastarms 338 angeordnete Tastspitze 166 hat, der in Längsrichtung 34 und in Querrichtung 32 der Ausrichtungsvorrichtung 1 einstellbar an der Werkzeugführungsvorrichtung 8 festsetzbar ist.

Ferner kann es vorgesehen sein, dass zwischen dem Einsteckdorn 156 und der Tastspitze 166 zur Einstellung in Längsrichtung 20 ein Wendelelement 168 vorgesehen ist, welches vorzugsweise den Tastarm 338 gleitend/frei durchdringt und dazu ausgebildet ist, den Höhenabstand der Tastspitze 166 zu einer Schnittebene 170 der Werkzeugführungsvorrichtung 8 ablesbar auszugeben.

Ferner kann es vorgesehen sein, dass das Wendelelement 168 ein relativ drehbar daran gehaltenes woei mit dem Wendelelement 168 in Schraubeingriff stehendes Wendelrad 172 aufweist, welches vorzugsweise an seiner Umfangsseite mit Zahlenwerten 174 versehen ist und über welches der Höhenabstand einstellbar ist.

Eine ggf. unabhängig beanspruchbare Ausführungsform der Ausrichtungsvorrichtung 1 für eine tibiale Resektionsführung, hat:
- eine (distale) Klemmvorrichtung 2 zum Klemmen des distalen Endes einer Tibia 3 eines Patienten;
- eine (proximale) Werkzeugführungsvorrichtung 8 zum Führen eines Werkzeuges während der Resektion der Tibia,
- eine Teleskopvorrichtung 10 mit einem Handgriff 14 und einem im Handgriff 14 gleitgeführten Gleitstab 11, wobei die Teleskopvorrichung 10 proximal mit der Werkzeugführungsvorrichtung 8 und distal mit der Klemmvorrichtung 2 verbunden und dazu ausgebildet ist, die Vorrichtungen 2, 8 gegenüber der Tibia 3 (längs-)auszurichten, wobei
die Teleskopvorrichtung 10 in ihrem proximalen Endbereich 52 zur Aufnahme einer Einschlagvorrichtung 202 eine Einschlagvorrichtungsaufnahme 204 aufweist, die durch den zumindest abschnittsweise als Hohlkörper ausgebildeten Gleitstab 11 der Teleskopvorrichtung 10 gebildet ist. Vorzugsweise durchdringt der Gleitstab 11 den Handgriff 14 in Längsrichtung der Teleskopvorrichtung 10 (in Konstruktionslage) vollständig und bildet so proximal zum Handgriff 14 die Einschlagvorrichtungsaufnahme 204.

Ferner kann es vorgesehen sein, dass die Einschlagvorrichtung 202 an ihrem distalen Ende so in den Hohlkörperabschnitt des Gleitstabs 11 einführbar ist, dass über einen Klemmmechanismus 208 seitens der Einschlagvorrichtung 202 eine Verklemmung der Einschlagvorrichtung 202 in dem Hohlkörperabschnitt bewirkbar ist.

Ferner kann es vorgesehen sein, dass der Klemmmechanismus 208 ein länglich ausgebildetes Ansteuerelement 210 vorzugsweise in Form eines Zug-/Druckstabs aufweist, welches innerhalb eines Hohlkörpers/Stützrohrs 408 der Einschlagvorrichtung 202 verschiebbar geführt ist und welches an seinem proximalen Bereich über ein Hebelelement 214 so in Längsrichtung 20 bewegbar ist, dass eine das Hebelelement 214 beaufschlagende (manuelle) Betätigungskraft auf ein mit dem Hohlkörperabschnitt des Gleitstabs 11 verklemmbares Verspann-/Kantelement 216 bewirkbar ist.

Ferner kann es vorgesehen sein, dass der Hohlkörper 408 der Einschlagvorrichtung 202 in seinem distalen Ende/Stirnseite eine Abschrägung 218 aufweist und dass das Kantelement 216 an seiner dem Ansteuerelement 210 zugewandten Ende/Stirnseite ebenfalls eine entsprechende Abschrägung 220 aufweist, so dass das Kantelement 216 bei einer Zugkraftbeaufschlagung durch das Ansteuerelement 210 an dem Hohlkörper 408 der Einschlagvorrichtung 202 in Quer-/Radialrichtung 32 abgleitet, so dass das Kantelement 216 der Einschlagvorrichtung 202 mit dem Gleitstab 11 der Teleskopvorrichtung 10 verklemmbar ist.

Ferner kann es vorgesehen sein, dass die Einschlagvorrichtung 202 so mit dem Gleitstab 11 der Teleskopvorrichtung 10 im eingesetzten Zustand umfangsseitig bündig abschließt, dass die Werkzeugführungsvorrichtung 8 in Längsrichtung 20 entlang des Gleitstabs 11 der Teleskopvorrichtung 10 und des Hohlkörpers 408 der Einschlagvorrichtung 202 verschiebbar ist.

Ferner kann es vorgesehen sein, dass die Einschlagvorrichtung 202 an ihrem proximalen Endabschnitt einen Querbalken 222 aufweist, der im Wesentlichen rechtwinklig zu dem Hohlkörper 408 längsverschiebbar an dem Hohlkörper 408 der Einschlagvorrichtung 202 gelagert ist und der an seinem freien (proximalen) Endabschnitt eine Fixierungsvorrichtung 224 zur proximalen Fixierung der Ausrichtungsvorrichtung 1 an der Tibia 3 aufweist. In vorteilhafter Weise ist die Einschlagvorrichtung 202 durch diese Ausführungsform in sämtlichen drei Raumrichtungen (d.h. in Höhenrichtung längs des Hohlkörpers 408, in Querrichtung längs des Querbalkens 222 sowie ggf. rotatorisch um den Hohlkörper 408 herum) frei beweglich gelagert und somit an die jeweiligen Randbedingungen anpassbar.

Eine bevorzugte, ggf. unabhängig beanspruchbare Ausführungsform der Ausrichtungsvorrichtung 1 hat:
- eine (distale) Klemmvorrichtung 2, die zumindest zwei gegeneinander schwenkbare Klammerelemente/Klemmarme 4, 4a zum Klemmen des distalen Endes einer Tibia 3 eines Patienten aufweist, die an einem Kragarm 93 gelagert sind, der dazu ausgebildet ist, in eine (distale) Fußklammeraufnahmevorrichtung 5 an einer Teleskopvorrichtung 10 der Ausrichtungsvorrichtung 1 eingeführt zu werden,
- eine Werkzeugführungsvorrichtung 8 zum Führen eines Werkzeuges während der Resektion der Tibia,
- die Teleskopvorrichtung 10, die mit der Werkzeugführungsvorrichtung 8 und mit der Klemmvorrichtung 2 verbunden ist und die dazu ausgebildet ist, die Vorrichtungen 2, 6 gegenüber der Tibia 3 auszurichten, wobei die Teleskopvorrichtung 10 ein Handgriffelement 14 aufweist, welches in sich ein Gleitstabelement 11 längsverschiebbar lagert, wobei
die Ausrichtungsvorrichtung 1 einen Satz 250 an Gleitstabelementen umfasst, aufweisend zumindest ein erstes Gleitstabelement 254 mit einer ersten kurzen Gleitstablänge und zumindest ein zweites Gleitstabelement 258 mit einer zweiten langen Gleitstablänge, wobei die jeweiligen Gleitstabelemente 254, 258 des erfindungsgemäßen Gleitstabsatzes 250 dazu vorgesehen sind, bedarfsweise in das Handgriffelement 14 eingesetzt zu werden, wobei die jeweiligen Gleitstäbe 254, 258 des Gleitstabsatzes 250 ansonsten baugleich zueinander ausgebildet sind und insbesondere an ihrem distalen Endabschnitten mit einer Fußklammeraufnahmevorrichtung 5 verbunden/ausgebildet sind und dass das Verhältnis der ersten Länge des ersten Gleitstabelementes 254 zur zweiten Länge des zweiten Gleitstabelementes 258 vorzugsweise zwischen 1 und 1,5, weiter vorzugsweise zwischen 1,1 und 1,3 und insbesondere 1,27 ist, so dass durch den ersten und den zweiten Gleitstab 254, 258 des Gleitstabsatzes 250 jeweils unterschiedliche Längen der Tibia resektionierbar sind, wobei die Länge jeweils vom proximalen Ende des Gleitstabes 254, 258 bis zur Mittelachse 262 der Fußklammeraufnahmevorrichtung 5 gemessen wird.

Eine ggf. unabhängig beanspruchbare. Ausführungsform der erfindungsgemäßen Ausrichtungsvorrichtung 1 sieht vor, dass die Klammerelemente/Klammerarme 4, 4a der Fußklammeraufnahmevorrichtung 5 bezüglich Längsrichtung des Kragarms 93 achsversetzt angeordnet sind, wobei der Kragarm 93 durch jeweils eine Drehung um 180° Grad in einer ersten Stellung und in einer zweiten Stellung in die Fußklammeraufnahmevorrichtung 5 einführbar ist, so dass in der ersten Stellung die Klammerelemente 4, 4a zum distalen Ende der Ausrichtungsvorrichtung 1 und in der zweiten Stellung zum proximalen Ende der Ausrichtungsvorrichtung 1 positioniert sind, wodurch ein Höhen-/Längsversatz der Klammerelemente 4, 4a in Längsrichtung 20 der Teleskopvorrichtung 10 bewirkt ist, wenn der Kragarm 93 von der ersten in die zweite Stellung um 180° Grad gedreht in die Fußklammeraufnahmevorrichtung 5 eingeführt wird.

Ferner kann es vorgesehen sein, dass der Höhenversatz der Fußklammeraufnahmevorrichtung 5 von der Mittelachse 262 der Fußklammeraufnahmevorrichtung 5 zu dem jeweiligen Anlenkpunkt 270 der Klammerelemente 4, 4a gemessen wird und vorzugweise zwischen 10 mm und 20 mm und insbesondere 15 mm beträgt.

### Bezugszeichen

- 1: Ausrichtungsvorrichtung
- 2: Klemmvorrichtung
- 3: Tibia
- 4, 4a: Klammerelemente
- 5: Klemmvorrichtungsaufnahme
- 6: Antastvorrichtung
- 8: Werkzeugführungsvorrichtung
- 10: Teleskopvorrichtung
- 11: Gleitstab (-element)
- 12: Sägeblockadapter/Entkopplungsvorrichtung
- 14: Handgriff
- 16: Druckelement
- 18: Haltemechanismus
- 20: Längsachse
- 22: ovalförmiger Bereich
- 24: Grundkörper
- 26: männliches Aufnahmeelement
- 28: weibliches Aufnahmeelement
- 30: Hakenelement
- 32: Querrichtung
- 34: Längsachse
- 36: Adaptersockel
- 44: Hinterschneidungen/Vorsprünge
- 50: reliefartige Oberfläche
- 52: proximaler Bereich
- 54: distaler Bereich

- 60: Gabel
- 62: Zinken
- 66: Spitzen
- 68: Enden der Klammerelemente
- 70: Verstärkungselement
- 74: Rastelement
- 76: Ratschenmechanismus
- 77: Vorspannfeder
- 78: mittiger Anlagebereich
- 82: Lagerarme/seitlicher Anlagebereich
- 84: Ratschenhebel
- 86: Schlitten/Tibia-Anlageblock
- 90: Spindelmechanismus
- 92: T-Stück
- 93: Einsteckstab
- 94: Raststruktur
- 95: Querträger

- 100: Aufnahmeabschnitt
- 102: Drehknopf
- 104: Sicherungselement
- 112: Verriegelungselement
- 114: Gehäuse
- 116: Zapfen
- 118: nutförmige Ausnehmung
- 120: Klemmstift/Bolzen
- 122: Einstellelement
- 124: Federelement
- 128: oberes Anschlagelement
- 130: unteres Anschlagelement

- 150: Aufnahmeausnehmung
- 151: Oberseite
- 152: Höhentastbaugruppe
- 154: Rastmechanismus
- 156: Einsteckelement
- 158: Hebelelement
- 160: Rastnase
- 164: Federelement
- 166: Tastspitze
- 168: Wendelelement
- 170: Schnittebene der Werkzeugführungsvorrichtung
- 172: Wendelrad
- 174: Zahlenwerte

- 200: Führungsstift
- 201: Rahmen
- 202: Einschlagvorrichtung
- 203: Schlagpin
- 204: Einschlagvorrichtungsaufnahme
- 208: Klemmmechanismus
- 210: Ansteuerelement
- 212: Hohlkörper
- 214: Hebelelement
- 216: Kantelement/Klemmplatte
- 218: Abschrägung
- 222: Querbalken
- 224: Einschlagmechanismus

- 250: Gleitstabelementen-Satz
- 254: erstes Gleitstabelement
- 258: zweites Gleitstabelement
- 262: Mittelpunkt der Fußklammeraufnahmevorrichtung
- 268: Höhenversatz
- 270: Randpunkt

- 300: Einschlaglöcher
- 302: Werkzeugführungsschlitze
- 303: Einschlagschlitz
- 304: Verstellschrauben/Drehknöpfe
- 306: Fixierungskraft
- 308: Kraftwirkung
- 322: Hand
- 334: Adapterschnittstelle
- 336: Höhenanschlag
- 338: Tastarm
- 340: vertikale Verschiebbarkeit
- 344: Anzeigeelement
- 346: Horizontale Verschiebbarkeit
- 348: Rotationsfreiheit
- 354: Reibschlusselement
- 364: Arretierungsnut
- 366: Schnitthöhe
- 368: Antastlevel
- 370: Schnittlevel
- 372: Rastmechanismus

- 400: Schlaghebel
- 402: Aufnahmegehäuse
- 404: Schlagpineinheit
- 405: Gleitbremse
- 406: Verbindungsmechanismus
- 408: Tragpfeiler/Hohlkörper
- 410: Schiebe-/Druckknopf

## Patentansprüche

1. Ausrichtungsvorrichtung (1), insbesondere für eine tibiale Resektionsführung, mit einer Teleskopvorrichtung (10), die an ihrem proximalen Endbereich mit einem Sägeblock (8) bestückbar oder bestückt ist und an ihrem distalen Endbereich eine Fußfesselvorrichtung, insbesondere eine Klemmvorrichtung (2) aufweist, die zumindest zwei gegeneinander wirkende sowie schwenkbare Klammerelemente (4, 4a) zum Klemmen des distalen Endes beispielsweise einer Tibia eines Patienten hat, wobei die Klammerelemente (4, 4a) der Klemmvorrichtung (2) jeweils bogenförmig ausgebildet sind, **dadurch gekennzeichnet, dass**
die Klammerelemente (4, 4a) jeweils zumindest abschnittsweise federelastisch sind, derart, dass die Klemmvorrichtung (2) ausschließlich unter Ausnutzung der Federelastizität der Klammerelemente (4, 4a) von der Tibia abziehbar ist.

2. Ausrichtungsvorrichtung (1) nach Anspruch 1, **dadurch gekennzeichnet, dass** die Klemmvorrichtung (2) einen Tibia-Anlageblock (86) unter anderem bestehend aus zwei im Wesentlichen V-förmig auseinanderstrebenden, starren Anlagearmen (82), an deren jeweils freien Endbereichen die federelastisch biegbaren/nachgebenden Klammerelemente (4, 4a) schwenkbar angelenkt sind, umfasst.

3. Ausrichtungsvorrichtung (1) nach Anspruch 1 oder 2, **dadurch gekennzeichnet, dass** die Klemmvorrichtung (2) ferner umfasst: einen Ratschen-Mechanismus (76), über welchen die Klammerelemente (4, 4a) jeweils am Tibia-Anlageblock (86) gelagert sind und durch den die Klammerelemente (4, 4a) unabhängig voneinander und vorzugsweise mit unterschiedlich hohen Vorspannkräften vorspannbar sind.

4. Ausrichtvorrichtung (1) nach einem der Ansprüche 1bis 3, **dadurch gekennzeichnet, dass** die Klammerelemente (4, 4a) zusätzlich gabelförmig ausgebildet sind und dass die Zinken (62) der jeweiligen Gabel (60) so zueinander versetzt angeordnet sind, dass diese im geschlossenen Zustand (64) überlappend ineinandergreifen.

## Claims

1. An alignment device (1), in particular for a tibial resection guide, comprising a telescopic device (10) which can be equipped or is equipped with a saw block (8) at its proximal end portion and has an ankle shackle device, in particular a clamping device (2), at its distal end portion, which has at least two clamping elements (4, 4a) which can act against one another and can be pivoted, for clamping the distal end of, for example, a tibia of a patient, the clamping elements (4, 4a) of the clamping device (2) each being of arcuate design, **characterized in that**
each of the clamping elements (4, 4a) is resilient at least in sections, such that the clamping device (2) can be removed from the tibia exclusively by utilizing the spring elasticity of the clamping elements (4, 4a).

2. The alignment device (1) according to claim 1, **characterized in that** the clamping device (2) comprises a tibia contact block (86) which, inter alia, consists of two substantially V-shaped and diverging, rigid contact arms (82), to the free end regions of which the resiliently bendable/yielding clamping elements (4, 4a) are pivotally articulated.

3. The alignment device (1) according to claim 1 or 2, **characterized in that** the clamping device (2) further comprises: a ratchet mechanism (76) via which the clamping elements (4, 4a) are supported in each case on the tibia contact block (86) and by means of which the clamping elements (4, 4a) can be pretensioned independently of each other and preferably with pretensioning forces of different magnitude.

4. The alignment device (1) according to any of claims 1 to 3, **characterized in that** the clamping elements (4, 4a) are additionally designed so as to be forkshaped and that the prongs (62) of the respective fork (60) are arranged in offset manner relative to one another such that in the closed state (64) they engage into one another in an overlapping manner.

## Revendications

1. Dispositif d'alignement (1), en particulier pour un guidage de résection tibial, avec un dispositif télescopique (10) qui est ou peut être équipé, au niveau de sa zone d'extrémité proximale, d'un bloc de sciage (8) et présente, au niveau de sa zone d'extrémité distale, un dispositif de bracelet de cheville, en particulier un dispositif de serrage (2) qui présente au moins deux éléments pince (4, 4a) pivotants ainsi qu'agissant l'un contre l'autre pour le serrage de l'extrémité distale par exemple d'un tibia d'un patient, dans lequel les éléments pince (4, 4a) du dispositif de serrage (2) sont respectivement formés en arc, **caractérisé en ce que**
les éléments pince (4, 4a) sont élastiques respectivement au moins par sections de sorte que le dispositif de serrage (2) puisse être retiré du tibia exclusivement en utilisant l'élasticité des éléments pince (4, 4a).

2. Dispositif d'alignement (1) selon la revendication 1, **caractérisé en ce que** le dispositif de serrage (2) comprend un bloc d'appui de tibia (86) se composant entre autres de deux bras d'appui (82) rigides, divergeant sensiblement en V, au niveau des zones d'extrémité libres respectivement desquels les éléments pince (4, 4a) flexibles/extensibles élastiquement sont articulés de manière pivotante.

3. Dispositif d'alignement (1) selon la revendication 1 ou 2, **caractérisé en ce que** le dispositif de serrage (2) comprend de plus : un mécanisme à cliquet (76) par le biais duquel les éléments pince (4, 4a) sont logés respectivement au niveau du bloc d'appui de tibia (86) et par lequel les éléments pince (4, 4a) sont indépendants l'un de l'autre et précontraignables de préférence avec des forces de précontrainte d'intensités différentes.

4. Dispositif d'alignement (1) selon l'une quelconque des revendications 1 à 3, **caractérisé en ce que** les éléments pince (4, 4a) sont formés en outre en forme de fourche et **en ce que** les dents (62) de la fourche (60) respective sont agencées en déport les unes des autres de sorte que celles-ci soient en prise les unes dans les autres dans l'état fermé (64) par chevauchement.
